(19) Europäisches Patentamt / European Patent Office / Office européen des brevets

(11) **EP 4 674 868 A1**

(12) **EUROPEAN PATENT APPLICATION**
published in accordance with Art. 153(4) EPC

(43) Date of publication:
**07.01.2026 Bulletin 2026/02**

(21) Application number: **24763247.4**

(22) Date of filing: **01.03.2024**

(51) International Patent Classification (IPC):
**C07K 16/28** (2006.01)       **A61P 35/00** (2006.01)
**C07K 16/46** (2006.01)

(52) Cooperative Patent Classification (CPC):
**A61P 35/00; C07K 16/28; C07K 16/46**

(86) International application number:
**PCT/CN2024/079551**

(87) International publication number:
**WO 2024/179567 (06.09.2024 Gazette 2024/36)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **01.03.2023 CN 202310186509**

(71) Applicants:
• **Jiangsu Hengrui Pharmaceuticals Co., Ltd.
Lianyungang, Jiangsu 222047 (CN)**
• **Shanghai Shengdi Pharmaceutical Co., Ltd
Shanghai 201210 (CN)**

(72) Inventors:
• **LI, Dan
Shanghai 201210 (CN)**
• **CHEN, Simeng
Shanghai 201210 (CN)**
• **HU, Rongting
Shanghai 201210 (CN)**
• **LIN, Yuan
Shanghai 201210 (CN)**
• **LIAO, Cheng
Shanghai 201210 (CN)**

(74) Representative: **Regimbeau
20, rue de Chazelles
75847 Paris Cedex 17 (FR)**

Remarks:
The complete document including Reference Table(s) and the Sequence Listing(s) can be downloaded from the EPO website

(54) **FAP/4-1BB/CD40 BINDING MOLECULE AND MEDICINAL USE THEREOF**

(57) The present disclosure relates to an FAP/4-1BB/CD40 binding molecule and a medicinal use thereof. Specifically, the present disclosure relates to an FAP/4-1BB/CD40 binding molecule, a 4-1BB binding molecule, a polynucleotide encoding a binding molecule, a vector, a pharmaceutical composition, a method for using same for treatment of cancer, and a related pharmaceutical use.

EP 4 674 868 A1

**Description**

The present disclosure claims priority to Chinese Patent Application No. 202310186509.9, filed on March 1, 2023, which is incorporated herein by reference in its entirety.

**TECHNICAL FIELD**

The present disclosure relates to the field of biopharmaceutics and particularly to a FAP/4-1BB/CD40-binding molecule, a 4-1BB-binding molecule, a polynucleotide encoding a binding molecule, a vector, and a pharmaceutical composition, as well as a method for treating cancer using same and related pharmaceutical use thereof.

**BACKGROUND**

CD40 (TNFRSF5) is a transmembrane phosphorylated glycoprotein and a member of the tumor necrosis factor receptor superfamily (TNFRS). It is mainly expressed on the surface of DCs, macrophages, and B cells, and it is also expressed on non-immune cells such as platelets and endothelial cells. When its ligand CD40L is restricted to a trimeric arrangement, CD40 signaling through receptors is in an optimal state, thereby enhancing the aggregation of CD40 itself and the subsequent recruitment and interaction with TNF receptor-associated factors. CD40L binds to the CD40 on DCs, inducing DC maturation, which is manifested in upregulated expression of the B7 family of costimulatory factors (CD80 and CD86) and increased secretion of proinflammatory cytokines such as interleukin 12 (IL-12). The interaction between CD40 and CD40L provides a costimulatory signal for T cell activation and promotes DCs' presentation of antigens to T cells.

4-1BB (CD137, TNFRSF9) is a transmembrane protein and also belongs to the tumor necrosis factor receptor superfamily. It is mainly expressed on activated CD4+ and CD8+ T cells, activated B cells, and natural killer (NK) cells. 4-1BB is expressed in a monomeric or dimeric form on the cell surface and, upon binding to its ligand (4-1BBL), signals through trimerization. 4-1BB is not constitutively expressed on T cells-its expression is induced upon activation of T cell receptors (TCRs). The stimulation by its natural ligand 4-1BBL or an antibody agonist can further upregulate 4-1BB expression. The 4-1BB intracellular domain is believed to act by recruiting TRAF1 and TRAF2 or may act through an adaptor protein of a TRAF3-mediated K63 polyubiquitination reaction. 4-1BBL trimerization leads to 4-1BB receptor aggregation and TRAF-mediated activation of the NF-κB and MAPK intracellular signaling pathways, resulting in proliferation, maturation, and extended survival of T cells, cytokine production, etc.

Fibroblast activation protein (FAP) is a tumor-associated antigen. FAP is lowly expressed in normal tissues of healthy adults and selectively expressed in 93% of tumor tissues, with 30% being high expression, such as colon cancer, pancreatic cancer, breast cancer, gastric cancer, prostate cancer, bladder cancer, and oral squamous cell carcinoma. At present, no multispecific antibodies against CD40, 4-1BB, and FAP are commercially available.

Therefore, there is an urgent need in the art to develop a multispecific antibody that can mediate tumor-specific CD40 and 4-1BB activation through FAP and has good therapeutic effects on tumors and low toxicity. The present disclosure provides a novel anti-FAP/4-1BB/CD40 trispecific antibody that can mediate tumor-specific CD40 and 4-1BB activation through FAP, has maturation-promoting and activating effects on APCs (e.g., dendritic cells (DCs)) and T cells, can eliminate hepatotoxicity, peripheral hematotoxicity, and other peripheral toxicities, and has an excellent administration window and druggability, thus providing a solution for clinical applications.

**SUMMARY**

The present disclosure provides a FAP/4-1BB/CD40-binding molecule, a 4-1BB-binding molecule, and a coding nucleic acid, a vector, a host cell, and a pharmaceutical composition thereof, as well as a method for treating or alleviating a cell proliferative disease (e.g., a tumor or cancer) using same and related pharmaceutical use thereof.

<u>FAP-Binding Molecule</u>

The present disclosure provides a FAP-binding molecule comprising a first antigen-binding domain that specifically binds to FAP. In some embodiments, the first antigen-binding domain comprises a heavy chain variable region (VH) and a light chain variable region (VL), wherein: the VH comprises a HCDR1, a HCDR2, and a HCDR3 from the amino acid sequence set forth in SEQ ID NO: 1, and/or the VL comprises a LCDR1, a LCDR2, and a LCDR3 from the amino acid sequence set forth in SEQ ID NO: 2.

The CDRs are defined according to the Kabat, IMGT, Chothia, AbM, or Contact numbering scheme. In some specific embodiments, the CDRs are defined according to the Kabat numbering scheme.

In some embodiments, the FAP-binding molecule comprises a VH and/or a VL as shown below: the VH comprises a HCDR1, a HCDR2, and a HCDR3 set forth in SEQ ID NOs: 3, 4, and 5, respectively, and the VL comprises a LCDR1, a LCDR2, and a LCDR3 set forth in SEQ ID NOs: 6, 7, and 8, respectively.

In some embodiments, the FAP-binding molecule comprises a VH and a VL, wherein: any one of the HCDRs that the VH comprises comprises 0, 1, 2, 3, 4, or 5 amino acid mutations compared to any one of the aforementioned HCDRs, and/or any one of the LCDRs that the VL comprises comprises 0, 1, 2, 3, 4, or 5 amino acid mutations compared to any one of the aforementioned LCDRs.

In some specific embodiments, the above amino acid mutations are conservative replacements, substitutions, or modifications, and/or deletions or additions that do not affect function.

In some embodiments, the present disclosure provides a FAP-binding molecule comprising any one or a combination of any two or more (e.g., 2, 3, 4, 5, or 6) of the aforementioned HCDR1, HCDR2, HCDR3, LCDR1, LCDR2, and LCDR3.

In some embodiments, the FAP-binding molecule comprises a VH and a VL, wherein:

the VH comprises the amino acid sequence set forth in SEQ ID NO: 1 or an amino acid sequence having at least 80% sequence identity thereto; and/or,

the VL comprises the amino acid sequence set forth in SEQ ID NO: 2 or an amino acid sequence having at least 80% sequence identity thereto.

In some embodiments, the present disclosure provides a FAP-binding molecule comprising any one or a combination of both of the aforementioned VH and VL.

In some embodiments, the FAP-binding molecule further comprises a light chain constant region and/or a heavy chain constant region.

In some embodiments, the light chain constant region is derived from a κ light chain, a λ light chain, or a variant of any one of the foregoing. For example, the light chain constant region is derived from a human κ light chain, a human λ, light chain, or a variant of any one of the foregoing. In some specific embodiments, the light chain constant region is derived from a human κ light chain or a variant thereof.

In some embodiments, the heavy chain constant region is derived from IgG1, IgG2, IgG3, IgG4, or a variant of any one of the foregoing. For example, the heavy chain constant region is derived from human IgG1, human IgG2, human IgG3, human IgG4, or a variant of any one of the foregoing.

In some embodiments, the FAP-binding molecule comprises a Fab heavy chain and a Fab light chain, wherein the Fab heavy chain comprises a heavy chain variable region (VH) and a heavy chain constant region (CH1), and the Fab light chain comprises a light chain variable region (VL) and a light chain constant region (CL).

In some specific embodiments, the FAP-binding molecule comprises a Fab heavy chain and a Fab light chain, wherein:

the Fab heavy chain comprises the amino acid sequence set forth in SEQ ID NO: 66 or an amino acid sequence having at least 80% sequence identity thereto, and/or the Fab light chain comprises the amino acid sequence set forth in SEQ ID NO: 67 or an amino acid sequence having at least 80% sequence identity thereto.

In the context of the present disclosure, "at least 80% (sequence) identity" encompasses at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99%, or 100% (sequence) identity; "at least 90% (sequence) identity" encompasses at least 90%, at least 91%, at least 92%, at least 93%, at least 94%, at least 95%, at least 96%, at least 97%, at least 98%, at least 99%, or 100% (sequence) identity.

In some embodiments, the FAP-binding molecule further comprises a human immunoglobulin Fc region. In some specific embodiments, the Fc region is a human IgG1, IgG2, or IgG4 Fc region.

In some embodiments, the aforementioned FAP-binding molecule further comprises a human immunoglobulin Fc region; for example, the Fc region is a human IgG1, IgG2, or IgG4 Fc region. In some embodiments, the Fc region may comprise mutations, and exemplary mutations include L234A/L235A, L234A/L235A/P329G, L234E, L234F, L234E/L235F, or L234E/L235F/P329G on IgG1, V234A/G237A/P238S/H268A/V309L/A330S/P331S on IgG2, F234A/L235A on IgG4, S228P/F234A/L235A on IgG4, N297A on IgG2 or IgG4, V234A/G237A on IgG2, K214T/E233P/L234V/L235A/G236 deletion/A327G/P331A/D365E/L358M on IgG1, H268Q/V309L/A330S/P331S on IgG2, S267E/L328F on IgG1, L234F/L235E/D265A on IgG1, L234A/L235A/G237A/P238S/H268A/A330S/P331S on IgG1, and S228P/F234A/L235A/G237A/P238S on IgG4. A hybrid IgG2/4 Fc domain may also be used, such as an Fc with residues 117-260 from IgG2 and residues 261-447 from IgG4. In some embodiments, a mutation is introduced into the Fc region to reduce or eliminate an effector function of the Fc region (e.g., reduce or eliminate the ADCC effect).

In some specific embodiments, the aforementioned FAP-binding molecule comprises a human IgG1 Fc region, wherein the human IgG1 Fc region comprises at least one of the following mutations: 234A, 235A, 220A, 297A or 297Q, 267E, and 328F.

Illustratively, the human IgG1 Fc region comprises: mutations 234A/235A, 234A/235A/297A, 220A/234A/235A, 220A/267E/328F, 220A/234A/235A/297A, 267E/328F, or 234A/235A/297A. In some specific embodiments, the human IgG1 Fc region comprises: mutations L234A/L235A, L234A/L235A/N297A, C220A/L234A/L235A, C220A/267E/328F, C220A/L234A/L235A/N297A, S267E/L328F, or L234A/L235A/N297A.

In some specific embodiments, the aforementioned FAP-binding molecule comprises a human IgG4 Fc region, wherein the human IgG4 Fc region comprises at least one of the following mutations: 228P, 234A, 235A, and 447A. Illustratively, the human IgG4 Fc region comprises mutation S228P.

In the context of the mutations that the Fc region comprises in the present disclosure, "/" represents "and"; for example, "L234A/L235A" represents "L234A and L235A"; that is, the Fc comprises mutations L234A and L235A; the amino acid positions of the mutations are numbered according to the EU numbering scheme.

In some embodiments, the FAP-binding molecule comprises a heavy chain (HC) and a light chain (LC), wherein:

the heavy chain comprises the amino acid sequence set forth in SEQ ID NO: 9 or an amino acid sequence having at least 80% sequence identity thereto; and/or,
the light chain comprises the amino acid sequence set forth in SEQ ID NO: 10 or an amino acid sequence having at least 80% sequence identity thereto.

In some embodiments, the present disclosure provides a FAP-binding molecule comprising any one or a combination of both of the aforementioned heavy chain (HC) and light chain (LC).

In some embodiments, the aforementioned FAP-binding molecule binds to human FAP or an epitope thereof with a $K_D$ value of $\leq 10^{-7}$; the aforementioned FAP-binding molecule binds to FAP (e.g., human FAP) or a fragment thereof with a $K_D$ of $10^{-7}$ M, $10^{-8}$ M, $10^{-9}$ M, $10^{-10}$ M, $10^{-11}$ M, or less.

In some embodiments, the FAP-binding molecule is an anti-FAP antibody or an antigen-binding fragment thereof; illustratively, the anti-FAP antibody is a murine antibody, a chimeric antibody, a humanized antibody, or a human antibody.

In some embodiments, the FAP-binding molecule is a human antibody or an antigen-binding fragment thereof, or a humanized antibody or an antigen-binding fragment thereof.

In some specific embodiments, the anti-FAP antibody or the antigen-binding fragment thereof includes, but is not limited to, Fab, Fv, sFv, Fab', F(ab')$_2$, linear antibodies, single-chain antibodies, scFv, sdAb, sdFv, nanobodies, peptibodies, domain antibodies, and multispecific antibodies (bispecific antibodies, diabodies, triabodies, tetrabodies, tandem di-scFv, and tandem tri-scFv); for example, the anti-FAP antigen-binding fragment is an scFv, Fv, Fab, or Fab' fragment.

In some embodiments, provided is an anti-FAP antibody or an antigen-binding fragment thereof that binds to or competes for binding to the same FAP (e.g., human FAP) epitope with the aforementioned anti-FAP antibody or the antigen-binding fragment thereof.

In some embodiments, provided is an anti-FAP antibody or an antigen-binding fragment thereof that blocks the binding of the aforementioned anti-FAP antibody or the antigen-binding fragment thereof to FAP (e.g., human FAP).

In some embodiments, provided is an anti-FAP antibody or an antigen-binding fragment thereof whose binding to FAP (e.g., human FAP) is blocked by the aforementioned anti-FAP antibody or the antigen-binding fragment thereof.

CD40-Binding Molecule

The present disclosure provides a CD40-binding molecule comprising a second antigen-binding domain that specifically binds to CD40. In some embodiments, the second antigen-binding domain comprises at least one immunoglobulin single variable domain that specifically binds to CD40.

In some embodiments, the immunoglobulin single variable domain that specifically binds to CD40 comprises a CDR1, a CDR2, and a CDR3 as shown in any one of the following:

1) a CDR1, a CDR2, and a CDR3 from the amino acid sequence set forth in SEQ ID NO: 11, 15, or 32;
2) the CDR1 comprises the amino acid sequence set forth in SEQ ID NO: 12, and/or the CDR2 comprises the amino acid sequence set forth in SEQ ID NO: 13, and/or the CDR3 comprises the amino acid sequence set forth in SEQ ID NO: 14; and
3) a CDR1, a CDR2, and a CDR3 set forth in SEQ ID NOs: 12, 13, and 14, respectively. The above CDR1, CDR2, and CDR3 are defined according to the Kabat, IMGT, Chothia, AbM, or Contact numbering scheme. In some specific embodiments, the CDR1, CDR2, and CDR3 are defined according to the Kabat numbering scheme.

In some embodiments, the present disclosure provides an immunoglobulin single variable domain that specifically binds to CD40, wherein the CDR1 that the immunoglobulin single variable domain comprises comprises 0, 1, 2, 3, 4, or 5 amino acid mutations compared to any one of the aforementioned CDR1s, and/or the CDR2 that the immunoglobulin single variable domain comprises comprises 0, 1, 2, 3, 4, or 5 amino acid mutations compared to any one of the aforementioned CDR2s, and/or the CDR3 that the immunoglobulin single variable domain comprises comprises 0, 1, 2, 3, 4, or 5 amino acid mutations compared to any one of the aforementioned CDR3s.

In some specific embodiments, the amino acid mutations in the CDR1, CDR2, and/or CDR3 are conservative substitutions.

In some embodiments, the present disclosure provides an immunoglobulin single variable domain comprising any one or a combination of any two or more (e.g., 2 or 3) of the aforementioned CDR1, CDR2, and CDR3.

In some embodiments, the immunoglobulin single variable domain is engineered by humanization, avidity maturation, T-cell epitope removal, reduction of antibody deamidation, reduction of antibody aggregation, reduction of antibody isomerization, reduction of binding to pre-existing anti-drug antibodies (pre-ADAs), and/or reduction of binding to anti-drug antibodies (ADAs).

In some specific embodiments, the immunoglobulin single variable domain is engineered by humanization. Illustratively, a framework region template of a human germline gene used for the humanization engineering is derived from IGHV3-48*03.

In some specific embodiments, the immunoglobulin single variable domain is engineered by reduction and/or removal of binding to pre-existing anti-drug antibodies (pre-ADAs) and/or reduction of binding to anti-drug antibodies (ADAs). Illustratively, a C-terminal sequence of the immunoglobulin single variable domain is mutated to "TVSAA" (SEQ ID NO: 69) to remove and/or reduce the binding of the 4-1BB-binding molecule to pre-ADAs. In some specific embodiments, a C-terminal sequence of the immunoglobulin single variable domain is mutated from "TVSS" (SEQ ID NO: 68) to "TVS<u>AA</u>" (SEQ ID NO: 69).

In some embodiments, the immunoglobulin single variable domain in the second antigen-binding domain comprises the amino acid sequence set forth in any one of SEQ ID NO: 11, 15, or 32 or an amino acid sequence having at least 80% sequence identity thereto.

In some embodiments, the aforementioned CD40-binding molecule comprises or is an antibody or an antigen-binding fragment thereof that specifically binds to CD40 or a fragment thereof. In some specific embodiments, the antibody or the antigen-binding fragment thereof is, for example, a camelid antibody, a chimeric antibody, a humanized antibody, or a fully human antibody, or an antigen-binding fragment thereof. In some specific embodiments, the antibody or the antigen-binding fragment thereof is, for example, a recombinant antibody or a fragment thereof. In some specific embodiments, the antibody or the antigen-binding fragment thereof is a linear antibody, a single-chain antibody, a nanobody, a peptibody, a domain antibody, or a multispecific antibody (a bispecific antibody, a diabody, a triabody, a tetrabody, a tandem di-scFv, or a tandem tri-scFv).

In some embodiments, the immunoglobulin single variable domain in the aforementioned CD40-binding molecule is a VHH.

In some embodiments, the present disclosure provides a CD40-binding molecule comprising one or more (e.g., 2, 3, 4, 5, 6, 7, or 8) aforementioned immunoglobulin single variable domains, wherein the immunoglobulin single variable domains may be identical or different, and any two of the immunoglobulin single variable domains may be linked, either directly or by a linker.

In some embodiments, provided is an anti-CD40 antibody or an antigen-binding fragment thereof that binds to or competes for binding to the same epitope with the aforementioned anti-CD40 antibody or the antigen-binding fragment thereof.

In some embodiments, provided is an anti-CD40 antibody or an antigen-binding fragment thereof that blocks the binding of the aforementioned anti-CD40 antibody or the antigen-binding fragment thereof to CD40 (e.g., human CD40).

In some embodiments, provided is an anti-CD40 antibody or an antigen-binding fragment thereof whose binding to CD40 (e.g., human CD40) is blocked by the aforementioned anti-CD40 antibody or the antigen-binding fragment thereof.

In some embodiments, the aforementioned CD40-binding molecule further comprises a human immunoglobulin Fc region; for example, the Fc region is a human IgG1, IgG2, or IgG4 Fc region. In some embodiments, the Fc region may comprise a mutation.

An exemplary human IgG1 Fc region comprises at least one of the following mutations: 234A, 235A, 220A, 297A or 297Q, 267E, and 328F. In some specific embodiments, the human IgG1 Fc region comprises: mutations 234A/235A, 234A/235A/N297A, C220A/234A/235A, C220A/267E/L328F, C220A/L234A/L235A/N297A, S267E/L328F, or L234A/L235A/N297A.

Illustratively, the human IgG4 Fc region comprises at least one of the following mutations: 228P, 234A, 235A, and 447A. In some specific embodiments, the human IgG4 Fc region comprises mutation S228P.

In some embodiments, in the aforementioned CD40-binding molecule, the immunoglobulin single variable domain and the Fc region are linked, either directly or by a linker. The linker may be a non-functional amino acid sequence of 1-20 or more amino acids without a secondary or higher structure. For example, the linker is a flexible linker, such as $G_4S$, GS, GAP, $(G_4S)_2$, $(G_4S)_3$, $(G_4S)_4$, $(G_4S)_5$, or ASGS.

4-1BB-Binding Molecule

The present disclosure provides a 4-1BB-binding molecule comprising a third antigen-binding domain that specifically binds to 4-1BB. In some embodiments, the third antigen-binding domain comprises at least one immunoglobulin single variable domain that specifically binds to 4-1BB.

In some embodiments, the immunoglobulin single variable domain in the third antigen-binding domain comprises a CDR1, a CDR2, and a CDR3 from the amino acid sequence set forth in any one of SEQ ID NOs: 28, 23-26, 18, and 33.

The CDR1, CDR2, and CDR3 are defined according to the Kabat, IMGT, Chothia, AbM, or Contact numbering scheme.

In some embodiments, the immunoglobulin single variable domain in the third antigen-binding domain comprises a CDR1, a CDR2, and a CDR3 from the amino acid sequences set forth in SEQ ID NOs: 19, 30, and 31. The amino acid sequence set forth in SEQ ID NO: 30 is DINSGGX$_1$STFYX$_2$DSVKG, wherein X$_1$ is E or Q, and X$_2$ is E or A. The amino acid sequence set forth in SEQ ID NO: 31 is HPLTX$_3$TIATMNDYDY, wherein X$_3$ is F or Y.

In some embodiments, the immunoglobulin single variable domain in the third antigen-binding domain comprises:

a CDR1 set forth in SEQ ID NO: 19,
a CDR2 set forth in SEQ ID NO: 20 or 29, and
a CDR3 set forth in SEQ ID NO: 21 or 27.

In some embodiments, the immunoglobulin single variable domain in the third antigen-binding domain comprises a CDR1, a CDR2, and a CDR3 as shown in any one of the following:

a-1) a CDR1, a CDR2, and a CDR3 set forth in SEQ ID NOs: 19, 29, and 27, respectively;
a-2) a CDR1, a CDR2, and a CDR3 set forth in SEQ ID NOs: 19, 20, and 27, respectively; and
a-3) a CDR1, a CDR2, and a CDR3 set forth in SEQ ID NOs: 19, 20, and 21, respectively.

In some embodiments, the CDR1 that the immunoglobulin single variable domain provided by the present disclosure comprises comprises 0, 1, 2, 3, 4, or 5 amino acid mutations compared to any one of the aforementioned CDR1s; and/or,

the CDR2 that the immunoglobulin single variable domain comprises comprises 0, 1, 2, 3, 4, or 5 amino acid mutations compared to any one of the aforementioned CDR2s; and/or,
the CDR3 that the immunoglobulin single variable domain comprises comprises 0, 1, 2, 3, 4, or 5 amino acid mutations compared to any one of the aforementioned CDR3s.

In some specific embodiments, the amino acid mutations in the CDR1, CDR2, and/or CDR3 are conservative substitutions.

In some embodiments, the present disclosure provides an immunoglobulin single variable domain comprising any one or a combination of any two or more (e.g., 2 or 3) of the aforementioned CDR1, CDR2, and CDR3.

In some embodiments, the immunoglobulin single variable domain is engineered by humanization, avidity maturation, T-cell epitope removal, reduction of antibody deamidation, reduction of antibody aggregation, reduction of antibody isomerization, reduction of binding to pre-existing anti-drug antibodies (pre-ADAs), and/or reduction of binding to anti-drug antibodies (ADAs).

In some specific embodiments, the immunoglobulin single variable domain is engineered by humanization. Illustratively, a framework region template of a human germline gene used for the humanization engineering is derived from IGHV3-64*04, IGHV3-23*03, and/or IGHV3-74*01. For example, FR1 is derived from IGHV3-64*04, FR2 is derived from IGHV3-23*03, and FR3 is derived from IGHV3-74*01.

In some specific embodiments, the immunoglobulin single variable domain is engineered by removal and/or reduction of T-cell epitopes (TCEs). By reducing and/or removing the TCE sites in one or more of the CDR1, CDR3, and CDR3, the immunogenicity of the 4-1BB-binding molecule can be reduced. Illustratively, the immunoglobulin single variable domain engineered by T-cell epitope removal comprises the following amino acid mutation: 99Y, and the mutation is numbered according to the Kabat numbering scheme.

In some specific embodiments, the immunoglobulin single variable domain is engineered by reduction and/or removal of binding to pre-existing anti-drug antibodies (pre-ADAs) and/or reduction of binding to anti-drug antibodies (ADAs). Illustratively, a C-terminal sequence of the immunoglobulin single variable domain is mutated to "TVSAA" (SEQ ID NO: 69) to remove and/or reduce the binding of the 4-1BB-binding molecule to pre-ADAs. In some specific embodiments, a C-terminal sequence of the immunoglobulin single variable domain is mutated from "TVSS" (SEQ ID NO: 68) to "TVS<u>AA</u>"

(SEQ ID NO: 69).

In some specific embodiments, the immunoglobulin single variable domain is engineered by removal and/or reduction of antibody aggregation. Illustratively, a C-terminal sequence of the immunoglobulin single variable domain is mutated to "TQVTVSS" (SEQ ID NO: 71) to reduce the risk of antibody aggregation. In some specific embodiments, a C-terminal sequence of the immunoglobulin single variable domain is mutated from "TLVTVSS" (SEQ ID NO: 70) to "TQVTVSS" (SEQ ID NO: 71).

In some embodiments, the immunoglobulin single variable domain in the third antigen-binding domain comprises the amino acid sequence set forth in any one of SEQ ID NOs: 18, 23-26, 28, and 33 or an amino acid sequence having at least 80% sequence identity thereto.

In some embodiments, the aforementioned 4-1BB-binding molecule comprises or is an antibody or an antigen-binding fragment thereof that specifically binds to 4-1BB or a fragment thereof. In some specific embodiments, the antibody or the antigen-binding fragment thereof is, for example, a camelid antibody, a chimeric antibody, a humanized antibody, or a fully human antibody, or an antigen-binding fragment thereof. In some specific embodiments, the antibody or the antigen-binding fragment thereof is, for example, a recombinant antibody or a fragment thereof. In some specific embodiments, the antibody or the antigen-binding fragment thereof is a linear antibody, a single-chain antibody, a nanobody, a peptibody, a domain antibody, or a multispecific antibody (a bispecific antibody, a diabody, a triabody, a tetrabody, a tandem di-scFv, or a tandem tri-scFv).

In some embodiments, the immunoglobulin single variable domain in the aforementioned 4-1BB-binding molecule is a VHH.

In some embodiments, the present disclosure provides a 4-1BB-binding molecule comprising one or more (e.g., 2, 3, 4, 5, 6, 7, or 8) aforementioned immunoglobulin single variable domains, wherein the immunoglobulin single variable domains may be identical or different, and any two of the immunoglobulin single variable domains may be linked, either directly or by a linker.

In some embodiments, the aforementioned 4-1BB-binding molecule of the present disclosure encompasses a variant, wherein the variant comprises one or more (e.g., 1, 2, 3, 4, 5, 6, 7, 8, 9, or 10) amino acid mutations compared to any one of SEQ ID NOs: 18, 23-26, 28, and 33; the amino acid mutations may be conservative replacements, substitutions, or modifications, and/or deletions or additions that do not affect function; the amino acid mutations may occur in the CDRs and/or FRs.

In some embodiments, provided is an anti-4-1BB antibody or an antigen-binding fragment thereof that binds to or competes for binding to the same epitope with the immunoglobulin single variable domain in the aforementioned 4-1BB-binding molecule of the present disclosure.

In some embodiments, provided is an anti-4-1BB antibody or an antigen-binding fragment thereof that blocks the binding of the immunoglobulin single variable domain in the aforementioned 4-1BB-binding molecule of the present disclosure to 4-1BB (e.g., human 4-1BB). In some specific embodiments, the anti-4-1BB antibody or the antigen-binding fragment thereof can also activate T cells and/or promote T cell proliferation.

In some embodiments, provided is an anti-4-1BB antibody or an antigen-binding fragment thereof whose binding to 4-1BB (e.g., human 4-1BB) is blocked by the immunoglobulin single variable domain in the aforementioned 4-1BB-binding molecule of the present disclosure.

In some embodiments, the aforementioned 4-1BB-binding molecule further comprises a human immunoglobulin Fc region; for example, the Fc region is a human IgG1, IgG2, or IgG4 Fc region. In some embodiments, the Fc region may comprise a mutation.

An exemplary human IgG1 Fc region comprises at least one of the following mutations: 234A, 235A, 220A, 297A or 297Q, 267E, and 328F. In some specific embodiments, the human IgG1 Fc region comprises: mutations 234A/235A, 234A/235A/N297A, C220A/234A/235A, C220A/267E/L328F, C220A/L234A/L235A/N297A, S267E/L328F, or L234A/L235A/N297A.

Illustratively, the human IgG4 Fc region comprises at least one of the following mutations: 228P, 234A, 235A, and 447A. In some specific embodiments, the human IgG4 Fc region comprises mutation S228P.

In some embodiments, in the aforementioned 4-1BB-binding molecule, the immunoglobulin single variable domain and the Fc region are linked, either directly or by a linker. The linker may be a non-functional amino acid sequence of 1-20 or more amino acids without a secondary or higher structure. For example, the linker is a flexible linker, such as $G_4S$, GS, GAP, $(G_4S)_2$, $(G_4S)_3$, $(G_4S)_4$, $(G_4S)_5$, or ASGS.

In some embodiments, the 4-1BB-binding molecule further comprises ii) a first antigen-binding domain that specifically binds to FAP, wherein the first antigen-binding domain is the first antigen-binding domain in any FAP-binding molecule provided by the present disclosure.

In some embodiments, the 4-1BB-binding molecule further comprises iii) a second antigen-binding domain that specifically binds to CD40. The second antigen-binding domain is the second antigen-binding domain in any CD40-binding molecule provided by the present disclosure.

In some embodiments, the 4-1BB-binding molecule comprises a third antigen-binding domain that specifically binds to 4-1BB, a second antigen-binding domain that specifically binds to CD40, and an Fc region.

In some specific embodiments, the third antigen-binding domain that specifically binds to 4-1BB is located at the N-terminus of the Fc region, and the second antigen-binding domain that specifically binds to CD40 is located at the C-terminus of the Fc region; or the third antigen-binding domain that specifically binds to 4-1BB is located at the C-terminus of the Fc region, and the second antigen-binding domain that specifically binds to CD40 is located at the N-terminus of the Fc region.

In some specific embodiments, the 4-1BB-binding molecule comprises a polypeptide represented by the following amino acid sequence: the amino acid sequence set forth in SEQ ID NO: 44 or an amino acid sequence having at least 90% sequence identity thereto.

FAP/4-1BB/CD40-Binding Molecule

The present disclosure provides a FAP/4-1BB/CD40-binding molecule comprising a first antigen-binding domain that specifically binds to FAP, a second antigen-binding domain that specifically binds to CD40, and a third antigen-binding domain that specifically binds to 4-1BB and capable of specifically binding to FAP, 4-1BB, and CD40 simultaneously or separately.

In some embodiments, the third antigen-binding domain is the third antigen-binding domain that specifically binds to 4-1BB in any of the aforementioned 4-1BB-binding molecules provided by the present disclosure.

Illustratively, in some specific embodiments, the third antigen-binding domain comprises at least one immunoglobulin single variable domain that specifically binds to 4-1BB, and the immunoglobulin single variable domain comprises a CDR1, a CDR2, and a CDR3 from the amino acid sequence set forth in any one of SEQ ID NOs: 28, 23-26, 18, and 33.

The CDR1, CDR2, and CDR3 are defined according to the Kabat, IMGT, Chothia, AbM, or Contact numbering scheme. In some specific embodiments, the CDR1, CDR2, and CDR3 are defined according to the Kabat numbering scheme.

Illustratively, in some specific embodiments, the immunoglobulin single variable domain in the third antigen-binding domain comprises a CDR1, a CDR2, and a CDR3 set forth in SEQ ID NOs: 19, 30, and 31, respectively.

Illustratively, in some specific embodiments, the immunoglobulin single variable domain in the third antigen-binding domain comprises:

a CDR1 set forth in SEQ ID NO: 19,
a CDR2 set forth in SEQ ID NO: 20 or 29, and
a CDR3 set forth in SEQ ID NO: 21 or 27.

Illustratively, in some specific embodiments, the immunoglobulin single variable domain in the third antigen-binding domain comprises a CDR1, a CDR2, and a CDR3 as shown in any one of the following:

a-1) a CDR1, a CDR2, and a CDR3 set forth in SEQ ID NOs: 19, 29, and 27, respectively;
a-2) a CDR1, a CDR2, and a CDR3 set forth in SEQ ID NOs: 19, 20, and 27, respectively; and
a-3) a CDR1, a CDR2, and a CDR3 set forth in SEQ ID NOs: 19, 20, and 21, respectively.

Illustratively, in some specific embodiments, the immunoglobulin single variable domain in the third antigen-binding domain is engineered by humanization, avidity maturation, T-cell epitope removal, reduction of antibody deamidation, reduction of antibody aggregation, reduction of antibody isomerization, reduction of binding to pre-existing anti-drug antibodies (pre-ADAs), and/or reduction of binding to anti-drug antibodies (ADAs).

Illustratively, the immunoglobulin single variable domain in the third antigen-binding domain is engineered by humanization, and a framework region template of a human germline gene used for the humanization engineering is derived from IGHV3-64*04, IGHV3-23*03, and/or IGHV3-74*01. For example, FR1 is derived from IGHV3-64*04, FR2 is derived from IGHV3-23*03, and FR3 is derived from IGHV3-74*01.

Illustratively, the immunoglobulin single variable domain in the third antigen-binding domain is engineered by removal and/or reduction of T-cell epitopes (TCEs). For example, the immunoglobulin single variable domain engineered by T-cell epitope removal comprises the following amino acid mutation: 99Y, and the mutation is numbered according to the Kabat numbering scheme.

Illustratively, the immunoglobulin single variable domain in the third antigen-binding domain is engineered by reduction and/or removal of binding to pre-existing anti-drug antibodies (pre-ADAs) and/or reduction of binding to anti-drug antibodies (ADAs). For example, a C-terminal sequence of the immunoglobulin single variable domain is mutated from "TVSS" (SEQ ID NO: 68) to "TVSAA" (SEQ ID NO: 69).

Illustratively, in some specific embodiments, the immunoglobulin single variable domain in the third antigen-binding domain is engineered by removal and/or reduction of antibody aggregation. For example, a C-terminal sequence of the immunoglobulin single variable domain is mutated from "TLVTVSS" (SEQ ID NO: 70) to "TQVTVSS" (SEQ ID NO: 71).

Illustratively, in some specific embodiments, the immunoglobulin single variable domain in the third antigen-binding domain comprises the amino acid sequence set forth in any one of SEQ ID NOs: 18, 23-26, 28, and 33 or an amino acid sequence having at least 80% sequence identity thereto.

Illustratively, in some specific embodiments, the third antigen-binding domain comprises one or more (e.g., 2, 3, 4, 5, 6, 7, 8, 9, or 10) aforementioned immunoglobulin single variable domains, wherein any two of the immunoglobulin single variable domains may be identical or different, and any two of the immunoglobulin single variable domains may be linked, either directly or by a linker.

In some embodiments, the second antigen-binding domain is the second antigen-binding domain that specifically binds to CD40 in any of the aforementioned CD40-binding molecules provided by the present disclosure.

Illustratively, in some specific embodiments, the second antigen-binding domain comprises at least one immunoglobulin single variable domain that specifically binds to CD40, and the immunoglobulin single variable domain comprises a CDR1, a CDR2, and a CDR3 as shown in any one of the following:

1) a CDR1, a CDR2, and a CDR3 from the amino acid sequence set forth in SEQ ID NO: 11, 15, or 32;
2) the CDR1 comprises the amino acid sequence set forth in SEQ ID NO: 12, and/or the CDR2 comprises the amino acid sequence set forth in SEQ ID NO: 13, and/or the CDR3 comprises the amino acid sequence set forth in SEQ ID NO: 14; and
3) a CDR1, a CDR2, and a CDR3 set forth in SEQ ID NOs: 12, 13, and 14, respectively. The above CDR1, CDR2, and CDR3 are defined according to the Kabat, IMGT, Chothia, AbM, or Contact numbering scheme. In some specific embodiments, the CDR1, CDR2, and CDR3 are defined according to the Kabat numbering scheme.

Illustratively, in some specific embodiments, the immunoglobulin single variable domain in the second antigen-binding domain is engineered by humanization, avidity maturation, T-cell epitope removal, reduction of antibody deamidation, reduction of antibody aggregation, reduction of antibody isomerization, reduction of binding to pre-existing anti-drug antibodies (pre-ADAs), and/or reduction of binding to anti-drug antibodies (ADAs).

Illustratively, in some specific embodiments, the immunoglobulin single variable domain in the second antigen-binding domain is engineered by humanization, and a framework region template of a human germline gene used for the humanization engineering is derived from IGHV3-48*03.

Illustratively, in some specific embodiments, the immunoglobulin single variable domain in the second antigen-binding domain is engineered by reduction and/or removal of binding to pre-existing anti-drug antibodies (pre-ADAs) and/or reduction of binding to anti-drug antibodies (ADAs). For example, a C-terminal sequence of the immunoglobulin single variable domain is mutated from "TVSS" (SEQ ID NO: 68) to "TVSAA" (SEQ ID NO: 69).

Illustratively, in some specific embodiments, the immunoglobulin single variable domain in the second antigen-binding domain comprises the amino acid sequence set forth in any one of SEQ ID NO: 11, 15, or 32 or an amino acid sequence having at least 80% sequence identity thereto.

Illustratively, in some specific embodiments, the second antigen-binding domain comprises one or more (e.g., 2, 3, 4, 5, 6, 7, 8, 9, or 10) aforementioned immunoglobulin single variable domains, wherein any two of the immunoglobulin single variable domains may be identical or different, and any two of the immunoglobulin single variable domains may be linked, either directly or by a linker.

In some embodiments, the first antigen-binding domain is the first antigen-binding domain that specifically binds to FAP in any of the aforementioned FAP-binding molecules provided by the present disclosure.

Illustratively, in some specific embodiments, the first antigen-binding domain comprises a heavy chain variable region (VH) and a light chain variable region (VL), wherein: the VH comprises a HCDR1, a HCDR2, and a HCDR3 from the amino acid sequence set forth in SEQ ID NO: 1, and/or the VL comprises a LCDR1, a LCDR2, and a LCDR3 from the amino acid sequence set forth in SEQ ID NO: 2.

The CDRs are defined according to the Kabat, IMGT, Chothia, AbM, or Contact numbering scheme. In some specific embodiments, the CDRs are defined according to the Kabat numbering scheme.

Illustratively, in some specific embodiments, the first antigen-binding domain comprises a VH and/or a VL as shown below: the VH comprises a HCDR1, a HCDR2, and a HCDR3 set forth in SEQ ID NOs: 3, 4, and 5, respectively, and the VL comprises a LCDR1, a LCDR2, and a LCDR3 set forth in SEQ ID NOs: 6, 7, and 8, respectively.

Illustratively, in some specific embodiments, the VH comprises the amino acid sequence set forth in SEQ ID NO: 1 or an amino acid sequence having at least 80% sequence identity thereto, and/or the VL comprises the amino acid sequence set forth in SEQ ID NO: 2 or an amino acid sequence having at least 80% sequence identity thereto.

Illustratively, in some specific embodiments, the first antigen-binding domain further comprises a light chain constant region and/or a heavy chain constant region. Illustratively, the light chain constant region is derived from a human κ light chain, a human λ light chain, or a variant of any one of the foregoing. In some specific embodiments, the light chain constant region is derived from a human κ light chain or a variant thereof. Illustratively, the heavy chain constant region is derived from human IgG1, human IgG2, human IgG3, human IgG4, or a variant of any one of the foregoing. Illustratively, in some specific embodiments, the first antigen-binding domain comprises a Fab heavy chain and a Fab light chain, wherein the Fab heavy chain comprises a heavy chain variable region (VH) and a heavy chain constant region (CH1), and the Fab light chain comprises a light chain variable region (VL) and a light chain constant region (CL).

Illustratively, in some specific embodiments, the Fab heavy chain comprises the amino acid sequence set forth in SEQ ID NO: 66 or an amino acid sequence having at least 80% sequence identity thereto, and/or the Fab light chain comprises the amino acid sequence set forth in SEQ ID NO: 67 or an amino acid sequence having at least 80% sequence identity thereto.

In some embodiments, the FAP/4-1BB/CD40-binding molecule further comprises an Fc region. In some specific embodiments, the Fc region is a human IgG1, IgG2, or IgG4 Fc region.

In some embodiments, the Fc region comprises amino acid mutations, and exemplary mutations include L234A/L235A, L234A/L235A/P329G, L234E, L234F, L234E/L235F, or L234E/L235F/P329G on IgG1, V234A/G237A/P238S/H268A/V309L/A330S/P331S on IgG2, F234A/L235A on IgG4, S228P/F234A/L235A on IgG4, N297A on IgG2 or IgG4, V234A/G237A on IgG2, K214T/E233P/L234V/L235A/G236 deletion/A327G/P331A/-D365E/L358M on IgG1, H268Q/V309L/A330S/P331S on IgG2, S267E/L328F on IgG1, L234F/L235E/D265A on IgG1, L234A/L235A/G237A/P238S/H268A/A330S/P331S on IgG1, and S228P/F234A/L235A/G237A/P238S on IgG4. A hybrid IgG2/4 Fc domain may also be used, such as an Fc with residues 117-260 from IgG2 and residues 261-447 from IgG4. In some specific embodiments, the FAP/4-1BB/CD40-binding molecule comprises a human IgG1 Fc region, wherein the human IgG1 Fc region comprises at least one of the following mutations: 234A, 235A, 220A, 297A or 297Q, 267E, and 328F.

Illustratively, the human IgG1 Fc region comprises: mutations 234A/235, 234A/235A/297A, 220A/234A/235A, 220A/267E/328F, 220A/234A/235A/297A, 267E/328F, or 234A/235A/297A. In some specific embodiments, the human IgG1 Fc region comprises: mutations L234A/L235A, L234A/L235A/N297A, C220A/L234A/L235A, C220A/267E/328F, C220A/L234A/L235A/N297A, S267E/L328F, or L234A/L235A/N297A.

In some specific embodiments, the FAP/4-1BB/CD40-binding molecule comprises a human IgG4 Fc region, wherein the human IgG4 Fc region comprises at least one of the following mutations: 228P, 234A, 235A, and 447A. Illustratively, the human IgG4 Fc region comprises mutation S228P.

In some embodiments, a mutation is introduced into the Fc region to reduce or eliminate an effector function of the Fc region (e.g., reduce or eliminate the ADCC effect).

In some embodiments, a mutation that pairs two subunits (Fc1 and Fc2) of the Fc region to form a dimer may also be introduced into the Fc region. For example, within the CH3/CH3 interface, one or more amino acid residues in the CH3 domain of Fc1 are mutated with one or more amino acid residues having a larger side-chain volume, thereby forming protuberances (or knobs) in the surface of the CH3 domain of Fc1; one or more, preferably two or three, of the amino acid residues in the CH3 domain of Fc2 that interact with the CH3 domain of Fc1 are mutated with amino acid residues having a smaller side-chain volume, thereby forming cavities (or holes) in the surface of the CH3 domain of Fc2 that interacts with the CH3 domain of Fc1. In some embodiments, an import residue having a larger side-chain volume is phenylalanine (F), tyrosine (Y), arginine (R), or tryptophan (W). In some embodiments, an import residue having a smaller side-chain volume is serine (S), alanine (A), valine (V), or threonine (T). Illustratively, the knob mutation includes substitution T366W, and the hole mutation is at least one, at least two, or three mutations selected from the group consisting of T366S, L368A, and Y407V.

In some embodiments, the Fc region enables the binding molecule to form a dimeric molecule while extending the *in vivo* half-life of the binding molecule.

In some embodiments, in the FAP/4-1BB/CD40-binding molecule, the first antigen-binding domain comprises a Fab heavy chain and a Fab light chain, wherein:

the Fab heavy chain is located at the N-terminus of the Fc region, the third antigen-binding domain is located at the C-terminus of the Fc region, and the second antigen-binding domain is located at the C-terminus of the third antigen-binding domain;
the Fab heavy chain is located at the N-terminus of the Fc region, the third antigen-binding domain is located at the C-terminus of the Fc region, and the second antigen-binding domain is located at the C-terminus of the Fab light chain;
the Fab heavy chain is located at the N-terminus of the Fc region, the second antigen-binding domain is located at the C-terminus of the Fc region, and the third antigen-binding domain is located at the C-terminus of the second antigen-binding domain;
or,
the Fab heavy chain is located at the N-terminus of the Fc region, the second antigen-binding domain is located at the C-terminus of the Fc region, and the third antigen-binding domain is located at the C-terminus of the Fab light chain.

In some embodiments, the third antigen-binding domain that specifically binds to 4-1BB and the second antigen-binding domain that specifically binds to CD40 may be linked, either directly or by a linker.

In some embodiments, the third antigen-binding domain that specifically binds to 4-1BB and the Fc region may be linked, either directly or by a linker.

In some embodiments, the second antigen-binding domain that specifically binds to CD40 and the Fc region may be linked, either directly or by a linker.

In some embodiments, the third antigen-binding domain that specifically binds to 4-1BB and the Fab light chain of the first antigen-binding domain that specifically binds to FAP may be linked, either directly or by a linker.

In some embodiments, the second antigen-binding domain that specifically binds to CD40 and the Fab light chain of the first antigen-binding domain that specifically binds to FAP may be linked, either directly or by a linker.

In some embodiments, the third antigen-binding domain that specifically binds to 4-1BB comprises 2, 3, 4, 5, 6, 7, 8, etc., immunoglobulin single variable domains. Any two of the immunoglobulin single variable domains may be linked, either directly or by a linker.

In some embodiments, the second antigen-binding domain that specifically binds to CD40 comprises 2, 3, 4, 5, 6, 7, 8, etc., immunoglobulin single variable domains. Any two of the immunoglobulin single variable domains may be linked, either directly or by a linker.

Illustratively, the linker includes, but is not limited to, amino acid sequences represented by $(G_mS_n)_h$ or $(GGNGT)_h$ (SEQ ID NO: 72) or $(YGNGT)_h$ (SEQ ID NO: 73) or $(EPKSS)_h$ (SEQ ID NO: 74), wherein m and n are each independently selected from the group consisting of integers of 1-8 (e.g., 1, 2, 3, 4, 5, 6, 7, or 8), and h is independently selected from the group consisting of integers of 1-20 (e.g., 1, 2, 3, 4, 5, 6, 7, 8, 9, 10, 11, 12, 13, 14, 15, 16, 17, 18, 19, or 20).

In some embodiments, the linker is a $(G_xS)_y$ linker, wherein x is selected from the group consisting of integers of 1-5 (e.g., 1, 2, 3, 4, or 5), and y is selected from the group consisting of integers of 1-6 (e.g., 1, 2, 3, 4, 5, or 6). In some specific embodiments, the linker is selected from the group consisting of $G_4S$ (SEQ ID NO: 75), GS, GAP, $(G_4S)_2$ (SEQ ID NO: 76), $(G_4S)_3$ (SEQ ID NO: 77), $(G_4S)_4$ (SEQ ID NO: 78), $(G_4S)_5$ (SEQ ID NO: 79), and ASGS (SEQ ID NO: 80); for example, the linker is $(G_4S)_2$.

In some embodiments, when the number of linkers contained in the FAP/4-1BB/CD40-binding molecule is 2 or more, any two of the linkers may be identical or different.

In some specific embodiments, the FAP/4-1BB/CD40-binding molecule comprises an anti-FAP antibody, and the anti-FAP antibody comprises two heavy chains (HCs) and two light chains (LCs). The VH of one of the HCs and the VL of one of the LCs form an antigen-binding site, and the VH of the other HC and the VL of the other LC form an antigen-binding site.

In some specific embodiments, the FAP/4-1BB/CD40-binding molecule comprises at least one (e.g., 1, 2, 3, 4, 5, 6, 7, or 8) VHH that specifically binds to CD40, and comprises at least one (e.g., 1, 2, 3, 4, 5, 6, 7, or 8) VHH that specifically binds to 4-1BB.

In some specific embodiments, any one VHH that specifically binds to CD40 may be linked to the C-terminus of the heavy chain of the anti-FAP antibody and/or to the C-terminus of the light chain of the anti-FAP antibody. The C-terminus of the VHH that specifically binds to CD40 may be further linked to one or more VHHs that specifically bind to CD40 and/or to one or more VHHs that specifically bind to 4-1BB.

In some specific embodiments, any one VHH that specifically binds to 4-1BB may be linked to the C-terminus of the heavy chain of the anti-FAP antibody and/or to the C-terminus of the light chain of the anti-FAP antibody. The C-terminus of the VHH that specifically binds to 4-1BB may be further linked to one or more VHHs that specifically bind to 4-1BB and/or to one or more VHHs that specifically bind to CD40.

In some embodiments, in the FAP/4-1BB/CD40-binding molecule, the valence ratio of the first antigen-binding domain to the second antigen-binding domain to the third antigen-binding domain is (1-2):(1-5):(1-5). In some embodiments, the valence ratio of the first antigen-binding domain to the second antigen-binding domain to the third antigen-binding domain is 1:(1-4):(1-4). In some specific embodiments, the valence ratio is 1:2:2, 1:2:4, 1:1:1, or 1:1:2.

In the present disclosure, "valence" is described as the number of antigen-binding moieties present in an antigen-binding molecule (e.g., an antigen-binding domain). Thus, a single binding molecule can bind to more than one binding site on a target molecule.

In some embodiments, the FAP/4-1BB/CD40-binding molecule comprises a first polypeptide chain and a second polypeptide chain, wherein in the direction from an N-terminus to a C-terminus, the FAP/4-1BB/CD40-binding molecule comprises any one or a combination of the following groups:

(I) the first polypeptide chain: [a Fab heavy chain of a first antigen-binding domain that specifically binds to FAP]-[an Fc region]-[a linker]a-[a third antigen-binding domain that specifically binds to 4-1BB]-[a linker]b-[a second antigen-binding domain that specifically binds to CD40]; and,
the second polypeptide chain: [a Fab light chain of a first antigen-binding domain that specifically binds to FAP];
(II) the first polypeptide chain: [a Fab heavy chain of a first antigen-binding domain that specifically binds to FAP]-[an Fc region]-[a linker]a-[a third antigen-binding domain that specifically binds to 4-1BB]; and,

the second polypeptide chain: [a Fab light chain of a first antigen-binding domain that specifically binds to FAP]-[a linker]b-[a second antigen-binding domain that specifically binds to CD40];

(III) the first polypeptide chain: [a Fab heavy chain of a first antigen-binding domain that specifically binds to FAP]-[an Fc region]-[a linker]a-[a third antigen-binding domain that specifically binds to 4-1BB]-[a linker]b-[a third antigen-binding domain that specifically binds to 4-1BB]; and,

the second polypeptide chain: [a Fab light chain of a first antigen-binding domain that specifically binds to FAP]-[a linker]c-[a second antigen-binding domain that specifically binds to CD40];

(IV) the first polypeptide chain: [a Fab heavy chain of a first antigen-binding domain that specifically binds to FAP]-[an Fc region]-[a linker]a-[a second antigen-binding domain that specifically binds to CD40]-[a linker]b-[a third antigen-binding domain that specifically binds to 4-1BB]; and,

the second polypeptide chain: [a Fab light chain of a first antigen-binding domain that specifically binds to FAP]; and

(V) the first polypeptide chain: [a Fab heavy chain of a first antigen-binding domain that specifically binds to FAP]-[an Fc region]-[a linker]a-[a second antigen-binding domain that specifically binds to CD40]; and,

the second polypeptide chain: [a Fab light chain of a first antigen-binding domain that specifically binds to FAP]-[a linker]b-[a third antigen-binding domain that specifically binds to 4-1BB];

wherein - represents a peptide bond, and any two linkers may be identical or different; a, b, and c are each independently selected from the group consisting of 0 and 1.

Illustratively, the linker includes, but is not limited to, amino acid sequences represented by $(G_mS_n)_h$ or $(GGNGT)_h$ (SEQ ID NO: 72) or $(YGNGT)_h$ (SEQ ID NO: 73) or $(EPKSS)_h$ (SEQ ID NO: 74), wherein m and n are each independently selected from the group consisting of integers of 1-8 (e.g., 1, 2, 3, 4, 5, 6, 7, or 8), and h is independently selected from the group consisting of integers of 1-20 (e.g., 1, 2, 3, 4, 5, 6, 7, 8, 9, 10, 11, 12, 13, 14, 15, 16, 17, 18, 19, or 20).

In some embodiments, the linker is a $(G_xS)_y$ linker, wherein x is selected from the group consisting of integers of 1-5 (e.g., 1, 2, 3, 4, or 5), and y is selected from the group consisting of integers of 1-6 (e.g., 1, 2, 3, 4, 5, or 6). In some specific embodiments, the linker is selected from the group consisting of $G_4S$, GS, GAP, $(G_4S)_2$, $(G_4S)_3$, $(G_4S)_4$, $(G_4S)_5$, and ASGS; for example, the linker is $(G_4S)_2$.

In some embodiments, the aforementioned FAP/4-1BB/CD40-binding molecule comprises two or more first antigen-binding domains that specifically bind to FAP, wherein any two of the first antigen-binding domains that specifically bind to FAP may be identical or different and are selected from the group consisting of any first antigen-binding domain provided by the present disclosure.

In some embodiments, the aforementioned FAP/4-1BB/CD40-binding molecule comprises two or more second antigen-binding domains that specifically bind to CD40, wherein any two of the second antigen-binding domains that specifically bind to CD40 may be identical or different and are selected from the group consisting of any second antigen-binding domain provided by the present disclosure.

In some embodiments, the aforementioned FAP/4-1BB/CD40-binding molecule comprises two or more third antigen-binding domains that specifically bind to 4-1BB, wherein any two of the third antigen-binding domains that specifically bind to 4-1BB may be identical or different and are selected from the group consisting of any third antigen-binding domain provided by the present disclosure.

In some embodiments, the FAP/4-1BB/CD40-binding molecule comprises a first polypeptide chain and a second polypeptide chain as shown in any one of the following:

1) the first polypeptide chain comprises the amino acid sequence set forth in SEQ ID NO: 49 or an amino acid sequence having 80% sequence identity thereto, and the second polypeptide chain comprises the amino acid sequence set forth in SEQ ID NO: 50 or an amino acid sequence having 80% sequence identity thereto;

2) the first polypeptide chain comprises the amino acid sequence set forth in SEQ ID NO: 34 or an amino acid sequence having 80% sequence identity thereto, and the second polypeptide chain comprises the amino acid sequence set forth in SEQ ID NO: 35 or an amino acid sequence having 80% sequence identity thereto;

3) the first polypeptide chain comprises the amino acid sequence set forth in SEQ ID NO: 36 or an amino acid sequence having 80% sequence identity thereto, and the second polypeptide chain comprises the amino acid sequence set forth in SEQ ID NO: 37 or an amino acid sequence having 80% sequence identity thereto;

4) the first polypeptide chain comprises the amino acid sequence set forth in SEQ ID NO: 38 or an amino acid sequence having 80% sequence identity thereto, and the second polypeptide chain comprises the amino acid sequence set forth in SEQ ID NO: 39 or an amino acid sequence having 80% sequence identity thereto;

5) the first polypeptide chain comprises the amino acid sequence set forth in SEQ ID NO: 40 or an amino acid sequence having 80% sequence identity thereto, and the second polypeptide chain comprises the amino acid sequence set forth in SEQ ID NO: 41 or an amino acid sequence having 80% sequence identity thereto;

6) the first polypeptide chain comprises the amino acid sequence set forth in SEQ ID NO: 42 or an amino acid sequence having 80% sequence identity thereto, and the second polypeptide chain comprises the amino acid sequence set forth in SEQ ID NO: 43 or an amino acid sequence having 80% sequence identity thereto;

7) the first polypeptide chain comprises the amino acid sequence set forth in SEQ ID NO: 45 or an amino acid sequence having 80% sequence identity thereto, and the second polypeptide chain comprises the amino acid sequence set forth in SEQ ID NO: 46 or an amino acid sequence having 80% sequence identity thereto; and

8) the first polypeptide chain comprises the amino acid sequence set forth in SEQ ID NO: 47 or an amino acid sequence having 80% sequence identity thereto, and the second polypeptide chain comprises the amino acid sequence set forth in SEQ ID NO: 48 or an amino acid sequence having 80% sequence identity thereto.

In some embodiments, the FAP/4-1BB/CD40-binding molecule comprises two first polypeptide chains and two second polypeptide chains. In some specific embodiments, the aforementioned FAP/4-1BB/CD40-binding molecule comprises two identical first polypeptide chains and two identical second polypeptide chains.

In some embodiments, the aforementioned FAP/4-1BB/CD40-binding molecule has at least one of the following properties:

a) specifically binding to human FAP or an epitope thereof;
b) specifically binding to human 4-1BB or an epitope thereof;
c) specifically binding to human CD40 or an epitope thereof;
d) when not crosslinked with FAP and CD40, having weak or no activation of the 4-1BB signaling pathway;
e) after being crosslinked with FAP and/or CD40, having relatively strong or strong activation of the 4-1BB signaling pathway; for example, $EC_{50} \leq 1$ nM; illustratively, $EC_{50} \leq 0.9$ nM, $EC_{50} \leq 0.8$ nM, $EC_{50} \leq 0.7$ nM, $EC_{50} \leq 0.6$ nM, $EC_{50} \leq 0.5$ nM, $EC_{50} \leq 0.4$ nM, $EC_{50} \leq 0.3$ nM, $EC_{50} \leq 0.2$ nM, $EC_{50} \leq 0.1$ nM, $EC_{50} \leq 0.09$ nM, $EC_{50} \leq 0.08$ nM, $EC_{50} \leq 0.07$ nM, $EC_{50} \leq 0.06$ nM, $EC_{50} \leq 0.05$ nM, $EC_{50} \leq 0.04$ nM, $EC_{50} \leq 0.03$ nM, $EC_{50} \leq 0.02$ nM or lower;
f) when not crosslinked with FAP and 4-1BB, having weak or no activation of the CD40 signaling pathway;
g) after being crosslinked with FAP and/or 4-1BB, having relatively strong or strong activation of the CD40 signaling pathway; for example, $EC_{50} \leq 1$ nM; illustratively, $EC_{50} \leq 0.9$ nM, $EC_{50} \leq 0.8$ nM, $EC_{50} \leq 0.7$ nM, $EC_{50} \leq 0.6$ nM, $EC_{50} \leq 0.5$ nM, $EC_{50} \leq 0.4$ nM, $EC_{50} \leq 0.3$ nM, $EC_{50} \leq 0.2$ nM, $EC_{50} \leq 0.1$ nM, $EC_{50} \leq 0.09$ nM, $EC_{50} \leq 0.08$ nM, $EC_{50} \leq 0.07$ nM, $EC_{50} \leq 0.06$ nM, $EC_{50} \leq 0.05$ nM, $EC_{50} \leq 0.04$ nM, $EC_{50} \leq 0.03$ nM, $EC_{50} \leq 0.02$ nM or lower;
h) activating T cells and/or promoting T cell proliferation;
l) promoting APC (e.g., dendritic cell (DC)) activation and/or promoting APC (e.g., dendritic cell (DC)) proliferation;
j) having low immunogenicity, and/or having a low level of binding to pre-existing anti-drug antibodies (pre-ADAs); and
k) inhibiting tumor growth.

In some embodiments, the aforementioned FAP/4-1BB/CD40-binding molecule of the present disclosure is capable of inhibiting tumor growth by at least about 10%, e.g., at least about 20%, about 30%, about 40%, about 50%, about 60%, about 70%, about 80%, or about 90%.

In some embodiments, the aforementioned FAP/4-1BB/CD40-binding molecule is an anti-FAP/4-1BB/CD40 multispecific antibody or an antigen-binding fragment thereof, wherein the antigen-binding fragment includes, but is not limited to: Fab, Fv, sFv, Fab', F(ab')$_2$, linear antibodies, single-chain antibodies, scFv, sdAb, sdFv, nanobodies, peptibodies, domain antibodies, and multispecific antibodies (bispecific antibodies, diabodies, triabodies, tetrabodies, tandem di-scFv, and tandem tri-scFv).

In some embodiments, provided is an anti-FAP/4-1BB/CD40 multispecific antibody or an antigen-binding fragment thereof that binds to or competes for binding to FAP, 4-1BB, and/or CD40, or binds to or competes for binding to the same epitope of FAP, 4-1BB, and/or CD40, with the aforementioned FAP/4-1BB/CD40-binding molecule of the present disclosure.

In some embodiments, provided is an anti-FAP/4-1BB/CD40 multispecific antibody or an antigen-binding fragment thereof that blocks the binding of the aforementioned FAP/4-1BB/CD40-binding molecule of the present disclosure to FAP, 4-1BB, and/or CD40.

In some embodiments, provided is a protein or molecule comprising any of the aforementioned FAP/4-1BB/CD40-binding molecules of the present disclosure. For example, the protein or molecule is a conjugate, and the conjugate may, for example, comprise any detectable label.

The anti-FAP/4-1BB/CD40 multispecific antibody provided by the present disclosure can mediate tumor-specific CD40 and 4-1BB activation through FAP, has maturation-promoting and activating effects on APCs (e.g., dendritic cells (DCs)) and T cells, has low or no hepatotoxicity, peripheral hematotoxicity, or other peripheral toxicities, and has an excellent administration window and druggability, thus providing a solution to clinical treatment of tumors.

Polynucleotide and Vector

The present disclosure provides a polynucleotide encoding any one of the following molecules provided by the present disclosure: a 4-1BB-binding molecule, a FAP/4-1BB/CD40-binding molecule, a FAP-binding molecule, and a CD40-binding molecule.

In some embodiments, the polynucleotide may be RNA, DNA, or cDNA. According to some embodiments of the present disclosure, the polynucleotide of the present disclosure is a substantially isolated nucleic acid.

The nucleic acid of the present disclosure may also be in the form of a vector and may be present in the vector and/or may be part of the vector. The vector is, for example, a plasmid, cosmid, YAC, or viral vector. The vector may especially be an expression vector, i.e., a vector that can provide *in vitro* and/or *in vivo* (i.e., in suitable host cells, host organisms, and/or expression systems) expression of the FAP/4-1BB/CD40-binding molecule, the 4-1BB-binding molecule, the FAP-binding molecule, or the CD40-binding molecule. The expression vector generally comprises at least one of the nucleic acids of the present disclosure, which is operably linked to one or more suitable expression regulatory elements (e.g., promoters, enhancers, and terminators). The selection of the elements and sequences thereof for expression in a particular host is common knowledge of those skilled in the art. Regulatory elements and other elements useful or necessary for the expression of the FAP/4-1BB/CD40-binding molecule, the 4-1BB-binding molecule, the FAP-binding molecule, and the CD40-binding molecule of the present disclosure are, for example, promoters, enhancers, terminators, integrators, selection labels, leader sequences, and reporter genes.

The nucleic acid of the present disclosure may be prepared or obtained by known means (e.g., by automatic DNA synthesis and/or recombinant DNA techniques) based on information about the amino acid sequences of the polypeptides of the present disclosure, and/or may be isolated from a suitable natural source.

Host Cell

The present disclosure provides a recombinant host cell that expresses or is capable of expressing one or more of the binding molecules of the present disclosure and/or comprises the nucleic acid or vector of the present disclosure. The binding molecules include at least one of the following: a FAP/4-1BB/CD40-binding molecule, a 4-1BB-binding molecule, a FAP-binding molecule, and a CD40-binding molecule.

In some embodiments, the host cell is a bacterial cell, a fungal cell, or a mammalian cell.

Illustratively, the bacterial cell includes, for example, cells of gram-negative bacterial strains (e.g., *Escherichia coli* strains, *Proteus* strains, and *Pseudomonas* strains) and gram-positive bacterial strains (e.g., *Bacillus* strains, *Streptomyces* strains, *Staphylococcus* strains, and *Lactococcus* strains).

Illustratively, the fungal cell includes, for example, cells of the species *Trichoderma, Neurospora,* and *Aspergillus,* or cells of the species *Saccharomyces* (e.g., *Saccharomyces cerevisiae*), *Schizosaccharomyces* (e.g., *Schizosaccharomyces pombe*), *Pichia* (e.g., *Pichia pastoris* and *Pichia methanolica*), and *Hansenula.*

Illustratively, the mammalian cell includes, for example, HEK293 cells, CHO cells, BHK cells, HeLa cells, COS cells, and the like.

Amphibian cells, insect cells, plant cells, and any other cells used in the art for expressing heterologous proteins may also be used in the present disclosure.

The cell of the present disclosure is unable to develop into a complete plant or animal individual.

Production or Preparation Method

The present disclosure provides a method for preparing any protein-binding molecule in the present disclosure. The protein-binding molecule includes at least one of the following: a FAP/4-1BB/CD40-binding molecule, a 4-1BB-binding molecule, a FAP-binding molecule, and a CD40-binding molecule. In some embodiments, the method comprises the following steps:

- culturing the host cell of the present disclosure under conditions suitable for the expression of the antibody; and
- isolating from the culture the protein of interest expressed by the host cell; and
- optionally, further purifying and/or modifying the protein of interest of the present disclosure.

The FAP/4-1BB/CD40-binding molecule, the 4-1BB-binding molecule, the FAP-binding molecule, the CD40-binding molecule, etc. of the present disclosure may be produced intracellularly (e.g., in the cytoplasm, in the periplasm, or in the inclusion bodies) in the cell described above, and is then isolated from the host cell and optionally further purified; or may be produced extracellularly (e.g., in the culture medium in which the host cell is cultured), and is then isolated from the culture medium and optionally further purified.

Methods and reagents for recombinant production of polypeptides, e.g., specific suitable expression vectors, transformation or transfection methods, selection labels, methods for inducing protein expression, culture conditions, and the like, are known in the art. Similarly, isolation and purification techniques suitable for use in the manufacture of proteins of interest, such as the binding molecules or antibodies of the present disclosure, are well known to those skilled in the art. Methods for producing and purifying antibodies are well known in the prior art and can be found in, for example, "Antibodies: A Laboratory Manual", Cold Spring Harbor Press (chapters 5-8 and 15). The engineered antibodies of the present disclosure may also be prepared and purified by conventional methods. For example, cDNA sequences encoding heavy and light chains can be cloned and recombined into an expression vector. Recombinant immunoglobulin expression vectors can be used to stably transfect cells. Mammalian expression systems may result in glycosylation of antibodies, particularly at the highly conserved N-terminus of the Fc region. Stable clones are obtained by expression of antibodies specifically binding to human antigens. Positive clones are expanded in a serum-free culture medium in a bioreactor to produce antibodies. The culture solution with the secreted antibodies can be purified and collected by conventional techniques. The antibodies can be filtered and concentrated by conventional methods. Soluble mixtures and multimers can also be removed by conventional methods, such as molecular sieves and ion exchange. The resulting products need to be immediately frozen, such as at -70 °C, or lyophilized.

However, the FAP/4-1BB/CD40-binding molecule, the 4-1BB-binding molecule, the FAP-binding molecule, the CD40-binding molecule, etc. of the present disclosure may also be obtained by other methods for producing proteins known in the art, such as chemical synthesis, including solid-phase or liquid-phase synthesis.

Composition

The present disclosure provides a composition comprising any one or any combination of the following: any FAP/4-1BB/CD40-binding molecule, 4-1BB-binding molecule, polynucleotide encoding a FAP/4-1BB/CD40-binding molecule or a 4-1BB-binding molecule, and vector provided by the present disclosure.

In some embodiments, the pharmaceutical composition comprises an amount of the FAP/4-1BB/CD40-binding molecule, the 4-1BB-binding molecule, the coding nucleic acid, or the vector described above that is effective in treating, alleviating, or preventing cancer.

In some embodiments, the pharmaceutical composition further comprises at least one pharmaceutically acceptable excipient, diluent, or carrier.

In some specific embodiments, a unit dose of the pharmaceutical composition may comprise 0.01 to 99 wt% of the FAP/4-1BB/CD40-binding molecule, or a unit dose of the pharmaceutical composition comprises 0.1-2000 mg of the FAP/4-1BB/CD40-binding molecule. In some specific embodiments, a unit dose of the pharmaceutical composition comprises 1-1000 mg of the FAP/4-1BB/CD40-binding molecule.

In some specific embodiments, a unit dose of the pharmaceutical composition may comprise 0.01 to 99 wt% of the 4-1BB-binding molecule, or a unit dose of the pharmaceutical composition comprises 0.1-2000 mg of the 4-1BB-binding molecule. In some specific embodiments, a unit dose of the pharmaceutical composition comprises 1-1000 mg of the 4-1BB-binding molecule.

In some specific embodiments, a unit dose of the pharmaceutical composition may comprise 0.01 to 99 wt% of the FAP/4-1BB/CD40-binding molecule and the 4-1BB-binding molecule, or a unit dose of the pharmaceutical composition comprises 0.1-2000 mg of the FAP/4-1BB/CD40-binding molecule and the 4-1BB-binding molecule. In some specific embodiments, a unit dose of the pharmaceutical composition comprises 1-1000 mg of the FAP/4-1BB/CD40-binding molecule and the 4-1BB-binding molecule.

In some embodiments, provided is an article of manufacture or product comprising the aforementioned FAP/4-1BB/CD40-binding molecule, 4-1BB-binding molecule, polynucleotide, and/or vector. Optionally, the article of manufacture comprises a container and a label. The container is, for example, a bottle, a syringe, and a test tube. The container accommodates a composition effective in treating a disorder. The label on or associated with the container indicates that the composition is used for treating the disorder of choice. The composition comprises the aforementioned FAP/4-1BB/CD40-binding molecule, 4-1BB-binding molecule, polynucleotide, and/or vector.

Kit and Detection

The present disclosure provides a kit comprising the aforementioned FAP/4-1BB/CD40-binding molecule, 4-1BB-binding molecule, polynucleotide, vector, or composition. The present disclosure further provides a method, system, or device for detecting FAP, 4-1BB, or CD40 *in vivo* or *in vitro*, comprising treating a sample with the aforementioned binding molecule, polynucleotide, vector, or composition of the present disclosure.

In some embodiments, the method, system, or device for *in vitro* detection may comprise, for example,

(1) contacting the sample with the FAP/4-1BB/CD40-binding molecule, the 4-1BB-binding molecule, the polynucleotide, the vector, or the composition;

(2) detecting a complex formed between the aforementioned binding molecule, polynucleotide, or vector and the sample; and/or

(3) contacting a reference sample (e.g., a control sample) with the binding molecule or the polynucleotide; and

(4) determining the extent of formation of the complex by comparison with the reference sample. A change (e.g., a statistically significant change) in the formation of the complex in the sample or subject as compared to a control sample or subject indicates the presence of FAP, 4-1BB, or CD40 in the sample.

In some other embodiments, the method, system, or device for *in vivo* detection may comprise:

(1) administering to a subject the aforementioned binding molecule, polynucleotide, or vector; and
(2) detecting the formation of a complex between the aforementioned binding molecule, polynucleotide, or vector and the subject.

The detection may comprise determining the location or time at which the complex is formed. The aforementioned binding molecule or nucleic acid may be labeled with a detectable substance, and the detection of a substance capable of binding to the protein or nucleic acid (e.g., FAP, 4-1BB, or CD40) is achieved by detecting the label. Suitable detectable substances include various enzymes, prosthetic groups, fluorescent substances, luminescent substances, and radioactive substances. The formation of a complex from the binding molecule or nucleic acid and FAP, 4-1BB, or CD40 can be detected by measuring or visualizing a substance that binds or does not bind to FAP, 4-1BB, or CD40. Conventional detection methods may be used, such as enzyme-linked immunosorbent assay (ELISA), radioimmunoassay (RIA), or tissue immunohistochemistry. For detection purposes, the binding molecule or nucleic acid of the present disclosure may be labeled with a fluorophore chromophore. In some embodiments, further provided are a diagnostic reagent comprising the nucleic acid or binding molecule described above, and related diagnostic use.

In some embodiments, further provided is a kit comprising the aforementioned binding molecule or polynucleotide, and the kit may further comprise instructions for diagnostic use. The kit may further comprise at least one additional reagent, such as a label or an additional diagnostic agent. For *in vivo* use, the binding molecule may be formulated into a pharmaceutical composition.

Method for Treating Disease and Pharmaceutical Use

Provided are use and a method of the FAP/4-1BB/CD40-binding molecule, the 4-1BB-binding molecule, the coding polynucleotide, the vector, or the pharmaceutical composition provided by the present disclosure in the prevention, treatment, or alleviation of a disease or disorder.

In some embodiments, provided is at least one of the following uses of the FAP/4-1BB/CD40-binding molecule, the 4-1BB-binding molecule, or the coding polynucleotide, the vector, or the pharmaceutical composition provided by the present disclosure:

1) use in the manufacture of a medicament for preventing, treating, or alleviating a disease or disorder;
2) use for preventing, treating, or alleviating a disease or disorder;
3) use for activating T cells;
4) use in the manufacture of a medicament for activating T cells;
5) use for promoting DC activation; and
6) use in the manufacture of a medicament for promoting DC activation.

In some embodiments, the present disclosure provides a method for preventing, treating, or alleviating a disease or disorder, comprising administering to a patient or subject an amount of the FAP/4-1BB/CD40-binding molecule, the 4-1BB-binding molecule, or the coding polynucleotide, the vector, or the pharmaceutical composition of the present disclosure that is effective in preventing, treating, or alleviating the disease or disorder.

In some embodiments, the disease or disorder is a tumor or cancer.

In some specific embodiments, the tumor or cancer is selected from the group consisting of lung cancer, prostate cancer, breast cancer, head and neck cancer, esophageal cancer, gastric cancer, colorectal cancer, bladder cancer, cervical cancer, uterine cancer, ovarian cancer, liver cancer, melanoma, renal cancer, squamous cell carcinoma, and hematological cancer.

In some embodiments, the FAP/4-1BB/CD40-binding molecule, the 4-1BB-binding molecule, or the coding polynucleotide, the vector, or the pharmaceutical composition of the present disclosure can be administered by any suitable method known in the art, and the administration may be systemic or local.

In some embodiments, the administration regimen may be adjusted to provide the optimum desired response (e.g., a therapeutic or prophylactic response). For example, a single dose may be administered, several doses may be administered over a period of time, or the dose may be proportionally reduced or increased depending on the exigencies of the therapeutic situation. Definitions of Terms

In order to facilitate the understanding of the present disclosure, certain technical and scientific terms are specifically defined below. Unless otherwise specifically defined in the present disclosure, all other technical and scientific terms used in the present disclosure have the meanings generally understood by those of ordinary skill in the art to which the present disclosure pertains.

The three-letter and single-letter codes for amino acids used in the present disclosure are as described in J. biol. chem., 243, p3558 (1968).

"CD40" and "CD40 antigen" refer to an approximately 48 kD glycoprotein expressed on the surface of normal and neoplastic B cells, which acts as a receptor for signals involved in cellular proliferation and differentiation (Ledbetter et al., 1987, J. Immunol. 138:788-785). A cell that endogenously expresses CD40 is any cell characterized by the surface expression of CD40, including but not limited to normal and neoplastic B cells, interdigitating cells, basal epithelial cells, carcinoma cells, macrophages, endothelial cells, follicular dendritic cells, tonsil cells, and bone marrow-derived plasma cells. In the present disclosure, "CD40" refers to any natural CD40 derived from any vertebrate, including mammals such as primates (e.g., humans) and rodents (e.g., mice and rats), unless otherwise indicated. A cDNA molecule encoding CD40 has been isolated from a library prepared from the Burkitt lymphoma cell line Raji (Stamenkovic et al., 1989, EMBO J. 8:1403). In the present disclosure, "CD40" encompasses full-length unprocessed CD40 as well as any form of CD40 that results from processing in a cell, and further encompasses naturally occurring variants of CD40, such as splice variants or allelic variants. In addition, artificially expressed forms, functional variants, etc. are also included.

"4-1BB" or "4-1BB antigen", also known as CD137 or tumor necrosis factor receptor superfamily 9, is a member of the TNF receptor superfamily (TNFRSF) and a costimulatory molecule expressed on the surface of CD8$^+$ and CD4$^+$ T cells, regulatory T cells (Tregs), NK cells and NKT cells, B cells, neutrophils, etc. 4-1BB is a costimulatory molecule expressed upon activation of immune cells. Human 4-1BB protein is under the NCBI accession number NP_001552.2. In the present disclosure, "4-1BB" may optionally include any protein of this kind, or fragments or variants thereof, including (but not limited to) the known or wild-type 4-1BB described in the present disclosure, as well as any naturally occurring splice variants, amino acid variants, or isoforms. In addition, artificially expressed forms, functional variants, etc. are also included.

"Fibroblast activation protein (FAP)" and "FAP antigen", also known as prolyl endopeptidase FAP or seprase (EC 3.4.21), refer to any natural FAP derived from any vertebrate, including mammals, such as primates (e.g., humans), non-human primates (e.g., cynomolgus monkeys) and rodents (e.g., mice and rats), unless otherwise indicated. In the present disclosure, "FAP" encompasses full-length unprocessed FAP as well as any form of FAP that results from processing in a cell, and further encompasses naturally occurring variants of FAP, such as splice variants or allelic variants. In addition, artificially expressed forms, functional variants, etc. are also included.

The amino acid sequence of human FAP is shown under UniProt (www.uniprot.org) accession No. Q12884, or NCBI (www.ncbi.nlm.nih.gov/) RefSeq NP_004451.2, or GeneBank accession No. AAC51668. The extracellular domain (ECD) of human FAP extends from amino acid position 26 to 760.

The term "functional variant" includes, but is not limited to, homologs, fragments, truncations, mutants, modifications, etc. of wild-type proteins. A functional variant of a protein has increased, decreased, or maintained protein activity compared to the wild-type protein.

"Binding molecule" encompasses any protein or polypeptide capable of specifically binding to an antigen (e.g., CD40, 4-1BB, or FAP) or any molecule comprising the protein or polypeptide, including but not limited to antibodies as defined in the present disclosure.

In the present disclosure, "polypeptide", "peptide", or "protein" is used interchangeably and refers to a polymer of amino acid residues, or a collection of polymers of multiple amino acid residues. These terms are used to describe amino acid polymers in which one or more amino acid residues are artificial chemical mimics of corresponding naturally occurring amino acids, as well as naturally occurring amino acid polymers and non-naturally occurring amino acid polymers. A polypeptide sequence is generally described as having an amino terminus (N-terminus) at the left end and having a carboxyl terminus (C-terminus) at the right end.

"Antibody" encompasses a variety of antibody structures, including but not limited to monoclonal antibodies, polyclonal antibodies, monospecific antibodies, multispecific antibodies (e.g., bispecific antibodies and trispecific antibodies), full-length antibodies, and antibody fragments (or antigen-binding fragments, or antigen-binding domains), as long as they exhibit the desired antigen-binding activity.

An antibody may refer to an immunoglobulin, which is of a four-peptide-chain structure formed by two heavy chains and two light chains linked by interchain disulfide bonds. The heavy chain constant regions of immunoglobulins differ in their amino acid composition and arrangement, and thus in their antigenicity. Accordingly, immunoglobulins can be divided into five classes, or isotypes of immunoglobulins, namely IgM, IgD, IgG, IgA, and IgE, with their corresponding heavy chains being μ chain, δ chain, γ chain, α chain, and ε chain, respectively. Ig of the same class can be divided into different subclasses according to differences in the amino acid composition of the hinge regions and the number and positions of disulfide bonds of the heavy chains; for example, IgG can be divided into IgG1, IgG2, IgG3, and IgG4. Light chains are divided into κ or λ chains according to differences in the constant regions. Each of the five classes of Ig may have a κ chain or λ chain. In the antibody heavy and light chains, the sequences of about 110 amino acids near the N-terminus vary considerably and thus are referred to as variable regions (V regions); the remaining amino acid sequences near the C-terminus are relatively stable and thus are referred to as constant regions (C regions). The variable regions comprise 3 hypervariable regions (HVRs) and 4 framework regions (FRs) with relatively conservative sequences. The 3 hypervariable regions determine the specificity of the antibody and thus are also known as complementarity-determining regions (CDRs). Each of the light chain variable regions (VLs) and the heavy chain variable regions (VHs) consists of 3 CDRs and 4 FRs arranged from the amino-terminus to the carboxyl-terminus as follows: FR1, CDR1, FR2, CDR2, FR3, CDR3, and FR4. The 3 CDRs of the light chain refer to LCDR1, LCDR2, and LCDR3; and the 3 CDRs of the heavy chain refer to HCDR1, HCDR2, and HCDR3.

The antibodies of the present disclosure may be polyclonal, monoclonal, xenogenic, allogeneic, syngeneic, or modified forms thereof, with the monoclonal antibodies being especially applicable in various examples. Generally, the antibodies of the present disclosure are recombinant antibodies. As used herein, "recombinant" generally refers to such products as a cell, a nucleic acid, a protein, or a vector, and indicates that the cell, the nucleic acid, the protein, or the vector has been modified by introduction of a heterologous nucleic acid or protein or alteration of a natural nucleic acid or protein, or that the cell is derived from a cell modified in this way. For example, recombinant cells express genes that are not found within the natural (non-recombinant) cellular form or express natural genes that are abnormally expressed, lowly expressed, or not expressed at all.

"Antigen-binding domain" encompasses a Fab, a modified Fab, a Fab', a modified Fab', F(ab')2, an Fv, a Fab-Fv, a Fab-dsFv, a single-domain antibody (e.g., a VH or VL or VHH), an scFv, a bivalent or trivalent or tetravalent antibody, a Bis-scFv, a diabody, a tribody, a triabody, a tetrabody, and an epitope-binding fragment of any one of the above (see, e.g., Holliger and Hudson, 2005, Nature Biotech. 23(9): 1126-1136; Adair and Lawson, 2005, Drug Design Reviews-Online 2(3), 209-217). Methods for producing and preparing such antigen-binding fragments are well known in the art (see, e.g., Verma et al., 1998, Journal of Immunological Methods, 216, 165-181).

"Multispecific antibody" refers to an antibody that binds to two or more different target antigens or different antigenic epitopes. The term "multispecific antibody" includes bispecific, trispecific, tetraspecific, pentaspecific, and hexaspecific. As used herein, the term "trispecific antibody" refers to an antibody that binds to three different antigens (e.g., FAP, CD40, or 4-1BB) or three different epitopes.

For the determination or definition of CDRs, the deterministic depiction of CDRs and identification of residues comprising antigen-binding sites of an antibody can be accomplished by resolving the structure of the antibody and/or resolving the structure of the antibody-ligand complex. This can be accomplished by any one of a variety of techniques known to those skilled in the art, such as X-ray crystallography. A variety of analysis methods can be used to identify CDRs, including but not limited to the Kabat numbering scheme, the Chothia numbering scheme, the AbM numbering scheme, the IMGT numbering scheme, the contact definition, and the conformational definition. The Kabat numbering scheme is a standard for numbering residues in antibodies and is generally used to identify CDRs (see, e.g., Johnson & Wu, 2000, Nucleic Acids Res., 28:214-8). The Chothia numbering scheme is similar to the Kabat numbering scheme, except that it takes into account the position of certain structural loop regions (see, e.g., Chothia et al., 1986, J. Mol. Biol., 196:901-17; Chothia et al., 1989, Nature, 342:877-83). The AbM numbering scheme adopts a computer program integration suite for modeling antibody structures manufactured by Oxford Molecular Group (see, e.g., Martin et al., 1989, Proc Natl Acad Sci (USA), 86:9268-9272; "AbMTM, A Computer Program for Modeling Variable Regions of Antibodies", Oxford, UK; Oxford Molecular, Ltd.). The AbM numbering scheme adopts a combination of a knowledge database and the de-novo method to model the tertiary structure of antibodies from basic sequences (see those described in Samudrala et al., 1999, "Ab Initio Protein Structure Prediction Using a Combined Hierarchical Approach", PROTEINS, Structure, Function and Genetics Suppl., 3:194-198). The contact definition is based on the analysis of the available complex crystal structures (see, e.g., MacCallum et al., 1996, J. Mol. Biol., 5:732-45). In the conformational definition, the positions of the CDRs can be identified as residues that contribute enthalpy to the antigen binding (see, e.g., Makabe et al., 2008, Journal of Biological Chemistry, 283:1156-1166). In addition, other CDR boundary definitions may not strictly follow one of the methods described above, but still overlap with at least a portion of the Kabat CDRs, although they may be shortened or lengthened based on predictions or experimental results that a particular residue or a particular group of residues do not significantly affect the antigen binding. As used in the present disclosure, a CDR may refer to a CDR defined by any method known in the art, including combinations of methods. The correspondence between the various numbering schemes is well known to those skilled in the art, and examples are shown in Table 1 below.

Table 1. The relationships between CDR numbering schemes

| CDR | IMGT | Kabat | AbM | Chothia | Contact |
|-------|-------|-------|-------|---------|---------|
| HCDR1 | 27-38 | 31-35 | 26-35 | 26-32 | 30-35 |

(continued)

| CDR | IMGT | Kabat | AbM | Chothia | Contact |
|---|---|---|---|---|---|
| HCDR2 | 56-65 | 50-65 | 50-58 | 52-56 | 47-58 |
| HCDR3 | 105-117 | 95-102 | 95-102 | 95-102 | 93-101 |
| LCDR1 | 27-38 | 24-34 | 24-34 | 24-34 | 30-36 |
| LCDR2 | 56-65 | 50-56 | 50-56 | 50-56 | 46-55 |
| LCDR3 | 105-117 | 89-97 | 89-97 | 89-97 | 89-96 |

The CDR amino acid residues of the VL and VH regions of the antibody of the present disclosure accord with the known Kabat numbering scheme in terms of number and positions.

"Domain" of a polypeptide or protein refers to a folded protein structure that is capable of maintaining its tertiary structure independently of the rest of the protein. In general, a domain is responsible for a single functional property of a protein, and in many cases may be added, deleted, or transferred to other proteins without loss of functions of the rest of the protein and/or the domain.

"Immunoglobulin domain" refers to a globular region of an antibody chain. Immunoglobulin domains are characterized by their maintenance of the folding feature of the antibody molecule.

"Immunoglobulin variable domain" refers to an immunoglobulin domain substantially consisting of four "framework regions", i.e., "framework region 1" or "FR1", "framework region 2" or "FR2", "framework region 3" or "FR3", and "framework region 4" or "FR4", and three "complementarity-determining regions" or "CDRs", i.e., "complementarity-determining region 1" or "CDR1", "complementarity-determining region 2" or "CDR2", and "complementarity-determining region 3" or "CDR3". Thus, the general structure or sequence of an immunoglobulin variable domain can be expressed as follows: FR1-CDR1-FR2-CDR2-FR3-CDR3-FR4. Immunoglobulin variable domains possess specificity for an antigen by virtue of having an antigen-binding site.

"Antibody framework (FR)" refers to a portion of a variable domain, which serves as a framework for the antigen-binding loops (CDRs) of the variable domain.

"Immunoglobulin single variable domain" is generally used to refer to an immunoglobulin variable domain (which may be a heavy or light chain domain, including a VH, VHH, or VL domain) that can form a functional antigen-binding site without interacting with other variable domains (e.g., without VH/VL interactions as are required between the VH and VL domains of conventional four-chain monoclonal antibodies). Examples of "immunoglobulin single variable domains" include nanobodies (including VHHs, humanized VHHs, and/or camelized VHs, e.g., camelized human VHs), IgNARs, domains, (single-domain) antibodies as VH domains or derived from VH domains (such as dAbs™) and (single-domain) antibodies as VL domains or derived from VL domains (such as dAbs™). Immunoglobulin single variable domains based on and/or derived from heavy chain variable domains (such as VH or VHH domains) are generally preferred. A specific example of an immunoglobulin single variable domain is a "VHH domain" (or "VHH" for short) as defined below.

"VHH domain", also known as heavy chain single-domain antibody, VHH, $V_{HH}$ domain, VHH antibody fragment, VHH antibody, or nanobody, is a variable domain of an antigen-binding immunoglobulin known as a "heavy-chain antibody" (i.e., "an antibody devoid of light chains") (Hamers-Casterman C, Atarhouch T, Muyldermans S, Robinson G, Hamers C, Songa EB, Bendahman N, Hamers R., "Naturally occurring antibodies devoid of light chains"; Nature 363, 446-448 (1993)). The term "VHH domain" is used to distinguish said variable domain from the VHs and VLs present in antibodies of the conventional four-peptide-chain structure. VHH domains specifically bind to an epitope without the need for an additional antigen-binding domain (as opposed to the VH or VL domain in conventional antibodies with a four-peptide-chain structure, in which case the epitope is recognized by the VL domain together with the VH domain). A VHH domain is a small, stable, and efficient antigen recognition unit formed by a single immunoglobulin domain. The terms "heavy-chain single-domain antibody", "VHH domain", "VHH", "$V_{HH}$ domain", "VHH antibody fragment", "VHH antibody", "Nanobody®", and "Nanobody® domain" ("Nanobody" is a trademark of Ablynx N.V., Ghent, Belgium) are used interchangeably. "VHH domains" include, but are not limited to, natural antibodies produced by camelids, antibodies produced by camelids and then humanized, or antibodies obtained by screening with phage display techniques. The total number of amino acid residues in a VHH domain will usually be in the range of 110 to 120, often between 112 and 115. However, it should be noted that smaller and longer sequences may also be suitable for the purposes described in the present disclosure. Methods for obtaining VHHs that bind to a particular antigen or epitope have been previously disclosed in the following documents: R. van der Linden et al., Journal of Immunological Methods, 240 (2000) 185-195; Li et al., J Biol Chem., 287 (2012) 13713-13721; Deffar et al., African Journal of Biotechnology Vol. 8 (12), pp.2645-2652, 17 June, 2009, and WO94/04678.

As is well known in the art for VH domains and VHH domains, the total number of amino acid residues in each of the CDRs may vary and may not correspond to the total number of amino acid residues indicated by the Kabat numbering (that is, one or more positions according to the Kabat numbering may not be occupied in the actual sequence, or the actual sequence may contain more amino acid residues than the number allowed for by the Kabat numbering). This means that, in general, the numbering according to Kabat may or may not correspond to the actual numbering of the amino acid residues in the actual sequence. Other numbering systems or numbering schemes include Chothia, IMGT, and AbM.

"Humanized antibody", also known as CDR-grafted antibody, refers to an antibody produced by grafting non-human CDR sequences into the framework of variable regions of a human antibody. Chimeric antibodies, due to their significant non-human protein content, can induce a strong immune response. This issue can be overcome by using humanized antibodies. To avoid the decrease in activity caused by the decrease in immunogenicity, the variable regions of a fully human antibody can be subjected to minimum reverse mutation to maintain activity. Examples of "humanization" include "humanization" of a VHH domain derived from camelidae by replacing one or more amino acid residues in the amino acid sequence of the original VHH sequence with one or more amino acid residues present at the corresponding positions in a VH domain of a human antibody of the conventional four-peptide-chain structure; the humanized VHH domain may contain one or more fully human framework region sequences, and in some specific embodiments, may contain the human framework region sequence of IGHV3. Another example of "humanization" includes an antibody produced by grafting mouse CDR sequences into a human antibody variable region framework, i.e., a different type of human germline antibody framework sequence. The strong antibody response induced by the chimeric antibody due to the large amount of mouse protein components it carries can be overcome. Methods for humanization include, e.g., protein surface amino acid humanization (resurfacing) and universal framework grafting method for antibody humanization (CDR grafting to a universal framework), i.e., "grafting" CDRs onto other "frameworks" (including but not limited to human scaffolds or non-immunoglobulin scaffolds). Scaffolds and techniques suitable for such CDR grafting are known in the art. For example, germline DNA sequences of genes of the human heavy and light chain variable regions can be found in the "VBase" human species sequence database, as well as in Kabat, E. A. et al., 1991 Sequences of Proteins of Immunological Interest, 5th edition. The humanized antibody of the present disclosure also includes humanized antibodies formed by further affinity maturation of the CDRs by phage display. Additionally, in order to avoid the decrease in activity caused by the decrease in immunogenicity, the framework sequence in the human antibody variable region can be subjected to minimum reverse mutation or back mutation to maintain activity.

"Pre-existing ADA" (i.e., pre-ADA) is an ADA already present in the subject or individual who is to be administered or given a drug (e.g., a protein or polypeptide drug, e.g., an antibody drug). Pre-existing ADAs can be present in an experiment-naive subject or individual (i.e., a subject or individual to whom a drug has never been administered before).

Generally, "specific binding" or "selective binding" refers to the binding of a binding molecule to an epitope on an antigen. The FAP/4-1BB/CD40-binding molecule of the present disclosure will bind to the antigens to be bound (i.e., FAP, CD40, and 4-1BB) or epitopes thereof with a dissociation constant ($K_D$) of preferably $10^{-7}$ to $10^{-10}$ mol/L (M), more preferably $10^{-8}$ to $10^{-10}$ mol/L, and even more preferably $10^{-9}$ to $10^{-10}$ or less, and/or with an association constant (KA) of at least $10^{-7}$ M, preferably at least $10^{-8}$ M, more preferably at least $10^{-9}$ M, and more preferably at least $10^{-10}$ M, as measured in a Biacore or KinExA or Fortibio assay. Any $K_D$ value greater than $10^{-4}$ M is generally considered to indicate non-specific binding. The specific binding of a binding molecule to an antigen or epitope can be determined in any known suitable manner, including, for example, the surface plasmon resonance (SPR), enzyme-linked immunosorbent assay (ELISA), and/or flow cytometry sorting (FACS) described in the present disclosure.

"Epitope" refers to a site on an antigen to which an immunoglobulin or an antibody binds. An epitope may be formed from contiguous amino acids, or non-contiguous amino acids juxtaposed by tertiary folding of the protein. An epitope formed from contiguous amino acids is generally retained after exposure to a denaturing solvent, while an epitope formed by tertiary folding is generally lost after a denaturing solvent treatment. An epitope generally comprises, for example, at least 3-15 amino acids in a distinctive spatial conformation. Methods for determining the binding of an epitope to a given antibody are well known in the art and include an immunoblotting assay, an immunoprecipitation assay, and the like. Methods for determining the spatial conformation of an epitope include techniques in the art and techniques described in the present disclosure, such as X-ray crystallography and two-dimensional nuclear magnetic resonance.

"Binding affinity" or "affinity" is used in the present disclosure as a measure of the strength of a non-covalent interaction between two molecules (e.g., an antibody or a portion thereof and an antigen). The binding affinity between two molecules can be quantified by determining the dissociation constant ($K_D$). $K_D$ can be determined by measuring the kinetics of complex formation and dissociation by using, e.g., the surface plasmon resonance (SPR) method (Biacore). The rate constants corresponding to the association and dissociation of a monovalent complex are referred to as the association rate constant ka (or kon) and the dissociation rate constant kd (or koff), respectively. $K_D$ is related to ka and kd by the equation $K_D = kd/ka$. The value of the dissociation constant can be determined directly by well-known methods, and calculations can even be performed for complex mixtures by, for example, those methods described in Caceci et al (1984, Byte 9:340-362). For example, $K_D$ can be determined by using a dual filtration nitrocellulose filter binding assay such as that disclosed by Wong & Lohman (1993, Proc. Natl. Acad. Sci. USA 90:5428-5432). Other standard assays for evaluating the binding ability of an antibody to a target antigen are known in the art and include, for example, ELISA, western blot, RIA, and FACS, as well as other assays exemplified elsewhere in the present disclosure. The binding kinetics and binding affinity of the antibody can also be evaluated by standard assays known in the art, such as surface plasmon resonance (SPR), e.g., by using the Biacore™ system or KinExA. The binding affinities associated with different molecular interactions, e.g., the binding affinities of different antibodies for a given antigen, can be compared by comparing the $K_D$ values of antibody/antigen complexes. Similarly, the specificity of an interaction can be evaluated by determining and comparing the $K_D$ value for the interaction of interest (e.g., a specific interaction between an antibody and an antigen) with the $K_D$ value for an interaction not of interest (e.g., a control antibody known not to bind to FAP).

"Conservative replacement" refers to a replacement with another amino acid residue having similar properties to the original amino acid residue. For example, lysine, arginine, and histidine have similar properties in that they have basic side chains, and aspartic acid and glutamic acid have similar properties in that they have acidic side chains. Additionally, glycine, asparagine, glutamine, serine, threonine, tyrosine, cysteine, and tryptophan have similar properties in that they have uncharged polar side chains, and alanine, valine, leucine, threonine, isoleucine, proline, phenylalanine, and methionine have similar properties in that they have nonpolar side chains. In addition, tyrosine, phenylalanine, tryptophan, and histidine have similar properties in that they have aromatic side chains. Thus, it will be apparent to those skilled in the art that even when an amino acid residue in a group exhibiting similar properties as described above is replaced, it will not exhibit a particular change in properties.

"Homology", "identity", or "sequence identity" refers to sequence similarity between two nucleic acid sequences or between two polypeptides. When positions in two compared sequences are occupied by identical nucleotides or amino acid monomers, e.g., if the position of each of two DNA molecules is occupied by an identical nucleotide, the molecules are homologous at that position. The homology percentage between two sequences is a function of the number of matching or homologous positions shared by the two sequences divided by the number of positions compared × 100%. For example, if 6 out of 10 positions are matched or homologous when two sequences are optimally aligned, the two sequences are 60% homologous. In general, when two sequences are aligned, a comparison is performed to obtain the maximum homology percentage.

"Nucleic acid" or "polynucleotide" are used interchangeably herein to refer to any DNA or RNA molecule, either single- or double-stranded, and, if single-stranded, the molecule of its complementary sequence, preferably a double-stranded DNA. A nucleic acid is "operably linked" when it is placed into a functional relationship with another nucleic acid sequence. For example, a promoter or enhancer is operably linked to a coding sequence if it affects the transcription of the coding sequence.

"Host cell" includes individual cells or cell cultures, which may be or have been the recipient of a vector for incorporation of a nucleic acid insert. The host cell includes progeny of a single host cell, and the progeny may not necessarily be completely identical (in morphology or genomic DNA complement) to the original parent cell due to natural, accidental, or deliberate mutations. The host cell includes cells transfected and/or transformed *in vivo* with the nucleic acid of the present disclosure. "Cell", "cell line", and "cell culture" are used interchangeably, and all such designations include their progeny. It should also be understood that all progeny may not be precisely identical in DNA content due to intentional or unintentional mutations. Mutant progeny with identical function or biological activity as those screened in the initially transformed cells are included.

The term "pharmaceutically acceptable auxiliary material" or "pharmaceutically acceptable excipient" includes any material that, when combined with an active ingredient, allows the ingredient to retain biological activity and is non-reactive with the immune system of a subject. Examples include, but are not limited to, any standard pharmaceutical carrier, such as phosphate-buffered saline, water, emulsions such as oil/water emulsions, and various types of wetting agents. In some examples, the diluent for aerosol or parenteral administration is phosphate-buffered saline (PBS) or normal (0.9%) saline. Compositions containing such carriers are formulated by well-known conventional methods (see, e.g., Remington's Pharmaceutical Sciences, 18th edition, A. Gennaro, eds., Mack Publishing Co., Easton, PA, 1990; and R Remington, The Science and Practice of Pharmacy, 20th edition, Mack Publishing, 2000).

"Inhibition" or "blocking" is used interchangeably and encompasses both partial and complete inhibition/blocking. "Inhibition of growth" (e.g., involving cells) is intended to include any measurable reduction in cell growth.

"Arresting the growth of" or "growth inhibition" refers to inhibiting cell growth or proliferation.

"Proliferative disease" refers to a disorder associated with a certain degree of abnormal cell proliferation. In one embodiment, the proliferative disorder is cancer. "Tumor" refers to all neoplastic cell growth and proliferation, whether malignant or benign, and all pre-cancerous and cancerous cells and tissues. "Cancer", "cancerous", "proliferative disorder", and "tumor" are not mutually exclusive when referred to in the present disclosure.

"Preventing a cancer" refers to delaying, inhibiting, or preventing the onset of the cancer in a subject in which the onset of oncogenesis or tumorigenesis has not been evidenced but a predisposition toward the cancer has been identified, as determined, for example, by genetic screening or otherwise. The term also encompasses treating a subject having a premalignant disorder to stop the progression of, or cause regression of, the premalignant disorder towards malignancy.

"Giving", "administering", and "treating", when applied to animals, humans, experimental subjects, cells, tissues, organs, or biological fluids, refer to contact of an exogenous drug, a therapeutic agent, a diagnostic agent, or a composition with the animals, humans, subjects, cells, tissues, organs, or biological fluids, e.g., therapeutic, pharmacokinetic, diagnostic, research, and experimental methods. The treatment of cells comprises contacting a reagent with cells and contacting the reagent with a fluid, where the fluid is in contact with the cells. "Giving", "administering", and "treating" also refer to treating, e.g., a cell, by a reagent, diagnosis, a binding composition, or by another cell *in vitro* and *ex vivo.* When applied to humans, veterinary medicine, or research subjects, they refer to therapeutic treatment, preventive or prophylactic measures, and research and diagnostic applications.

"Treating" or "treatment" refers to administering a therapeutic agent, such as a therapeutic agent comprising any one of the binding molecules of the present disclosure or a pharmaceutical composition thereof, either internally or externally, to a subject who has had, is suspected of having, or is predisposed to having one or more proliferative diseases or symptoms thereof on which the therapeutic agent is known to have a therapeutic effect. Typically, the therapeutic agent is administered in an amount effective in alleviating one or more disease symptoms in the subject or population being treated, whether by inducing regression of such symptoms or inhibiting the development of such symptoms into any clinically measurable degree. The amount of therapeutic agent effective in alleviating any specific disease symptom (also referred to as the "therapeutically effective amount") may vary depending on factors such as the disease state, age, and weight of the subject, and the capability of the drug to produce the desired therapeutic effect in the subject. Whether a disease symptom has been alleviated can be evaluated by any clinical testing methods commonly used by doctors or other health care professionals to evaluate the severity or progression of the symptom. Although embodiments of the present disclosure (e.g., treatment methods or products) may be ineffective in alleviating a disease symptom of interest in a certain subject, they shall alleviate the disease symptom of interest in a statistically significant number of subjects as determined by any statistical test method known in the art, such as the Student's t-test, Chi-square test, U-test by Mann and Whitney, Kruskal-Wallis test (H-test), Jonckheere-Terpstra test and Wilcoxon test.

"Effective amount" comprises an amount sufficient to ameliorate or prevent a symptom or sign of a medical disorder. An effective amount also means an amount sufficient to allow or facilitate diagnosis. The effective amount for a subject may vary depending on factors such as the disorder to be treated, the general health of the subject, the method, route, and dose of administration, and the severity of side effects. An effective amount may be the maximum dose or administration regimen to avoid significant side effects or toxic effects. The subject of the present disclosure may be an animal or a human subject. "Optional" or "optionally" means that the event or circumstance subsequently described may, but does not necessarily, occur, and this description includes instances where the event or circumstance occurs or does not occur. "And/or" should be taken as specific disclosure of each of the two specified features or components with or without the other. Thus, the term "and/or" as used in a phrase such as "A and/or B" in the present disclosure includes "A and B", "A or B", "A" (alone), and "B" (alone). Unless otherwise clearly stated in the context, throughout the description and the claims, the words "comprise", "have", "include", and the like should be understood in an inclusive sense as opposed to an exclusive or exhaustive sense; that is to say, in the sense of "including, but not limited to".

The "subject" or "patient" in the present disclosure means a mammal, particularly a primate, and especially a human.

## BRIEF DESCRIPTION OF THE DRAWINGS

FIG. 1 shows the FACS assay results of the binding of test antibodies to the human 4-1BB antigen.

FIG. 2 shows the binding of C5_V2, C5_V2-YTI, and C5_V2-YTIQ to human 4-1BB on the surface of HEK293 cells.

FIG. 3 shows schematic diagrams of the structures of anti-FAP/4-1BB/CD40 multispecific antibodies (a-e of FIG. 3) and an anti-4-1BB/CD40 (f of FIG. 3).

FIG. 4A shows the FACS assay results of the binding of test antibodies to human FAP on the surface of CHOK1 cells. FIG. 4B shows the FACS assay results of the binding of test antibodies to human 4-1BB on the surface of HEK293 cells. FIG. 4C shows the FACS assay results of the binding of test antibodies to human CD40 on the surface of HEK293 cells.

FIG. 5A shows the FACS assay results of the binding of test antibodies to human FAP on the surface of CHOK1 cells. FIG. 5B shows the FACS assay results of the binding of test antibodies to human 4-1BB on the surface of HEK293 cells. FIG. 5C shows the FACS assay results of the binding of test antibodies to human CD40 on the surface of HEK293 cells.

FIG. 6A shows the FACS assay results of the binding of test antibodies to human FAP on the surface of CHOK1 cells. FIG. 6B shows the FACS assay results of the binding of test antibodies to human 4-1BB on the surface of HEK293 cells. FIG. 6C shows the FACS assay results of the binding of test antibodies to human CD40 on the surface of HEK293 cells.

FIGs. 7A-7C show FAP/CD40-dependent 4-1BB/NF-$\kappa$B luciferase reporter gene assay results for test antibodies. FIG. 7A shows the results before crosslinking, FIG. 7B shows the results after FAP crosslinking, and FIG. 7C shows the results after CD40 crosslinking.

FIGs. 8A-8C show FAP/CD40-dependent 4-1BB/NF-κB luciferase reporter gene assay results for test antibodies. FIG. 8A shows the results before crosslinking, FIG. 8B shows the results after FAP crosslinking, and FIG. 8C shows the results after CD40 crosslinking.

FIGs. 9A-9C show FAP/CD40-dependent 4-1BB/NF-κB luciferase reporter gene assay results for test antibodies. FIG. 9A shows the results before crosslinking, FIG. 9B shows the results after FAP crosslinking, and FIG. 9C shows the results after CD40 crosslinking.

FIGs. 10A-10C show FAP/4-1BB-dependent CD40/NF-κB luciferase reporter gene assay results for test antibodies. FIG. 10A shows the results before crosslinking, FIG. 10B shows the results after FAP crosslinking, and FIG. 10C shows the results after 4-1BB crosslinking.

FIGs. 11A-11C show FAP/4-1BB-dependent CD40/NF-κB luciferase reporter gene assay results for test antibodies. FIG. 11A shows the results before crosslinking, FIG. 11B shows the results after FAP crosslinking, and FIG. 11C shows the results after 4-1BB crosslinking.

FIGs. 12A-12C show FAP/4-1BB-dependent CD40/NF-κB luciferase reporter gene assay results for test antibodies. FIG. 12A shows the results before crosslinking, FIG. 12B shows the results after FAP crosslinking, and FIG. 12C shows the results after 4-1BB crosslinking.

FIGs. 13 and 14 show the results of the activation of T lymphocytes from donor 1 and donor 2 by test antibodies.

FIG. 15 shows the results of the activation of CD3+ T lymphocytes from donor 1 and donor 2 by test antibodies, with RO7122290, MP0310, BNT312, and isotype IgG1 as controls.

FIGs. 16, 17, and 18 show DC maturation promotion assay results for test antibodies.

FIG. 19 shows the abilities of test antibodies to crosslink with 4-1BB/CD40-expressing cells, with BNT312 and isotype IgG1 as controls.

FIG. 20 shows MLR assay results for test antibodies, with BNT312 and isotype IgG1 as controls.

FIG. 21 shows the antitumor effects of test antibodies on MC38-mFAP graft tumors, with the vehicle, RO7122290, and Ab10-A12V2-2 as controls.

FIG. 22 shows the effects of test antibodies on ALT/AST in mice on day 17 after administration, with the vehicle, Ab10-A12V2-2, and RO7211190 as controls.

FIG. 23 shows the effects of test antibodies on the body weight of mice, with the vehicle, RO7122290, and Ab10-A12V2-2 as controls.

FIG. 24 shows the effects of test antibodies on neutrophil and platelet counts in mice, with the vehicle, RO7122290, and Ab10-A12V2-2 as controls.

FIG. 25 shows the effects of test antibodies on ALT/AST in mice on day 8 after administration, with the vehicle, Ab10-A12V2-2, and BNT312 as controls.

## DETAILED DESCRIPTION

### Example 1. Screening for and Preparation of Anti-FAP Monoclonal Antibodies

1.1. Sequences and preparation of screening and detection antigens

Human fibroblast activation protein (FAP; GeneBank accession No. AAC51668) is a serine oligopeptidase with a molecular weight of about 170 kDa and is a homodimer. The sequences, sources, and uses of the recombinant FAP proteins used in the present disclosure are shown in Table 2.

Table 2. The sources of the amino acid sequences of recombinant proteins

| Name | Start and end of amino acid sequence | Sino Biological cat. No. | Use |
|---|---|---|---|
| h-FAP-biotin | Leu26-Asp760 (GeneBank accession No. AAC51668) | 10464-H07H-B | Phage display liquid-phase magnetic bead screening |
| h-FAP-His | | 10464-H07H | ELISA screening for positive clones |
| h-FAP-FITC | | 10464-H07H-F | CHO cell surface antibody display screening |

1.2. Screening for h-FAP-specific binding antibody fragments from human natural Fab phage display library

With the antigen h-FAP-biotin as the target molecule, antigen-specific phages were captured using avidin-coated magnetic beads, and phage enrichment was performed using a magnetic rack. The antigen-specific phages were eluted with a glycine solution (pH 2.2).

After two rounds of screening, 284 clones were randomly picked and screened by phage ELISA for positive clones, which included the following specific steps: The growing colonies in the dish were counted (284 colonies in total). The phages were inoculated onto a 96-well plate, each well of which contained 400 μL of culture medium (2YT + Amp + 0.2% glucose). The plate was shaken at 37 °C at 250 rpm for 6 h. The plate was coated with the antigen h-FAP-His at 100 ng/100 μL/well and incubated overnight at 4 °C. The next day, after the 96-well plate was washed and blocked, 100 μL of overnight-incubated bacterial liquid was added to each well, and the plate was incubated at 37 °C for 1 h. After washing, a secondary antibody (an HRP-labeled anti-human IgG-Fab antibody) was added, and the plate was incubated at 37 °C for 40 min. After washing, the substrate solution was added, and the plate was incubated in a dark place for 30 min. Then the OD600 was measured using a microplate reader. A total of 122 positive clones obtained from two rounds of screening were sequenced, and 74 distinctive VHs (constituting a VH-enriched library), 48 distinctive κ light chains (constituting a KLC-enriched library), and 10 distinctive λ light chains (constituting an LLC-enriched library) were obtained.

1.3. Construction of CHO cell surface full-length antibody display library

1) A full-length antibody CHO cell display gene library was constructed, which included the following steps: The VH-enriched library, the KLC-enriched library, and the LLC-enriched library were amplified by PCR using three sets of primers, respectively. The three enriched libraries were digested with enzymes and then inserted into corresponding component vectors to construct corresponding component libraries. The KLC, LLC, and VH component libraries were digested with enzymes. The KLC, LLC, and VH fragment libraries after the digestion were analyzed and purified by electrophoresis. The full-length antibody cell display vector was digested with an enzyme, and the fragments of the vector were purified. The purified fragments of the vector and the KLC, LLC, and VH fragment libraries were mixed and ligated. The ligation products were purified and transferred into *Escherichia coli* cells by electroporation. The cells were plated on a dish and cultured overnight at 37 °C, and the colonies were counted. The library size measured 3.6 × 10E6, which is more than 800 times the theoretical diversity (74 × 58 = 4292). All colonies were collected, and the vector DNA was extracted to obtain a full-length antibody cell display gene library.

2) An h-FAP antigen-specific full-length antibody cell display library was constructed, which included: CHO cells were transformed with the vector DNA of the full-length antibody cell display gene library to construct a full-length antibody CHO cell display library. The cell library was screened under the pressure of hygromycin. A stably transformed cell library was obtained, and the cell library was double-stained with a PE-labeled mouse anti-human κ (or λ) light-chain antibody and an FITC-labeled h-FAP antigen and sorted by FACS for PE and FITC double-positive cells. Each well contained one cell. The κ light chain library sorting used 2 96-well plates, and the λ light chain library sorting used 1 96-well plate. The cells were cultured under the pressure of hygromycin.

3) FACS analysis was performed to identify single-cell clones displaying h-FAP antigen-specific antibodies, which included: The cells were cultured under the pressure of hygromycin for 14 days, and 153 stably transformed κ chain single-cell clones and 50 λ chain single-cell clones were obtained. The cells were digested with a 0.5 mM EDTA-PBS buffer solution, and the single-cell clones were double-stained with a PE-labeled mouse anti-human κ (or λ) light-chain antibody and an FITC-labeled h-FAP antigen. FACS analysis showed that 138 PE and FITC fluorescence double-positive single-cell clones were obtained.

4) Antibody genes were cloned, which included: The affinities of the positive clones were analyzed by FACS, and 45 clones were subjected to antibody gene PCR amplification. The positive clones were centrifugally collected, and the supernatant was discarded. The genomic DNA was extracted from the cells, and the VH and LC were amplified by PCR. The fragments obtained from the amplification were isolated by electrophoresis and sequenced. The variable region sequences of the anti-human FAP monoclonal antibody Ab10 are as follows:

> VH

QVQLQQSGVEVKKPGASVTVSCRASGYSFADHFIHWVRQAPGQGFQWMGWIN
PNRGVTHHAQDFQGRVAMTRDMSTDTVYMELTSLRSDDTAVYYCARDASLTA
RPYYFYGFDVWGQGTLVTVSS (SEQ ID NO: 1);

> VL

DIQMTQSPSSVSASVGDRVTITCRASQGISSWLAWYQQKPGKAPKLLIYAASSLQ SGVPSRFSGSGSGTDFTLTISSLQPEDFATYYCQQANSFPPAFGQGTKVEIK  (SEQ ID NO: 2);

The CDR sequences are as follows:

Table 3. The CDRs of the anti-FAP antibody Ab10

| HCDR1 | DHFIH (SEQ ID NO:3) | LCDR1 | RASQGISSWLA (SEQ ID NO:6) |
|-------|---------------------|-------|---------------------------|
| HCDR2 | WINPNRGVTHHAQDFQG (SEQ ID NO:4) | LCDR2 | AASSLQS (SEQ ID NO:7) |
| HCDR3 | DASLTARPYYFYGFDV (SEQ ID NO:5) | LCDR3 | QQANSFPPA (SEQ ID NO:8) |

1.4. Expression and purification of anti-FAP antibody

The heavy and light chain variable regions of the above anti-FAP antibody Ab10 sequences were linked to the constant regions human HC and LC, respectively, to form a full-length heavy chain sequence (SEQ ID NO: 9) and a full-length light chain sequence (SEQ ID NO: 10). Plasmids were constructed, transient transfection, expression, and purification were performed, and the antibody of interest was obtained. The full-length sequences of Ab10 are shown below, with the constant region sequences underlined:

> Ab10 full-length heavy chain

QVQLQQSGVEVKKPGASVTVSCRASGYSFADHFIHWVRQAPGQGFQWMGWIN PNRGVTHHAQDFQGRVAMTRDMSTDTVYMELTSLRSDDTAVYYCARDASLTA RPYYFYGFDVWGQGTLVTVSS<u>ASTKGPSVFPLAPSSKSTSGGTAALGCLVKDYF</u>

<u>PEPVTVSWNSGALTSGVHTFPAVLQSSGLYSLSSVVTVPSSSLGTQTYICNVNHK PSNTKVDKKVEPKSCDKTHTCPPCPAPE**AA**GGPSVFLFPPKPKDTLMISRTPEVT CVVVDVSHEDPEVKFNWYVDGVEVHNAKTKPREEQYNSTYRVVSVLTVLHQD WLNGKEYKCKVSNKALPAPIEKTISKAKGQPREPQVYTLPPSRDELTKNQVSLT CLVKGFYPSDIAVEWESNGQPENNYKTTPPVLDSDGSFFLYSKLTVDKSRWQQG NVFSCSVMHEALHNHYTQKSLSLSPGK</u>

SEQ ID NO: 9

> Ab10 full-length light chain

DIQMTQSPSSVSASVGDRVTITCRASQGISSWLAWYQQKPGKAPKLLIYAASSLQ SGVPSRFSGSGSGTDFTLTISSLQPEDFATYYCQQANSFPPAFGQGTKVEIK<u>RTVA APSVFIFPPSDEQLKSGTASVVCLLNNFYPREAKVQWKVDNALQSGNSQESVTE QDSKDSTYSLSSTLTLSKADYEKHKVYACEVTHQGLSSPVTKSFNRGEC</u>

SEQ ID NO: 10

> Fab heavy chain

QVQLQQSGVEVKKPGASVTVSCRASGYSFADHFIHWVRQAPGQGFQWMGWIN
PNRGVTHHAQDFQGRVAMTRDMSTDTVYMELTSLRSDDTAVYYCARDASLTA
RPYYFYGFDVWGQGTLVTVSSASTKGPSVFPLAPSSKSTSGGTAALGCLVKDYF
PEPVTVSWNSGALTSGVHTFPAVLQSSGLYSLSSVVTVPSSSLGTQTYICNVNHK
PSNTKVDKKV

SEQ ID NO: 66

> Fab light chain

DIQMTQSPSSVSASVGDRVTITCRASQGISSWLAWYQQKPGKAPKLLIYAASSLQ
SGVPSRFSGSGSGTDFTLTISSLQPEDFATYYCQQANSFPPAFGQGTKVEIKRTVA
APSVFIFPPSDEQLKSGTASVVCLLNNFYPREAKVQWKVDNALQSGNSQESVTE
QDSKDSTYSLSSTLTLSKADYEKHKVYACEVTHQGLSSPVTKSFNRGEC

SEQ ID NO: 67

### 1.5. ELISA binding assay

A plate was directly coated with a His-tagged FAP recombinant protein. After the antibodies were added, a secondary antibody (an HRP-conjugated anti-human IgG antibody) and the HRP substrate TMB were added to determine the binding activity of the antibodies to the antigen. The assay included: A 96-well microplate was coated with a 0.5 $\mu$g/mL solution of human FAP-His protein at 100 $\mu$L/well, and the plate was incubated overnight at 4 °C.After thorough washing, a blocking solution was added at 200 $\mu$L/well, and the plate was incubated at room temperature for 2 h.After thorough washing, the test anti-FAP antibodies, diluted with a diluent, were added at 100 $\mu$L/well.The plate was incubated at room temperature for 1 h. After thorough washing, an HRP-labeled goat anti-human IgG secondary antibody diluted with a diluent in a 1:20,000 ratio was added at 100 $\mu$L/well.The plate was incubated at room temperature for 1 h. After thorough washing, TMB was added at 100 $\mu$L/well, and the plate was incubated in a dark place for 15 min.0.16 M sulfuric acid was added at 50 $\mu$L/well.The OD at 450 nm was measured using a Thermo MultiSkanFc microplate reader. The binding $EC_{50}$ value of the anti-FAP antibody Ab10 was calculated to be 0.0920 mg/mL.

### 1.6. Surface plasmon resonance (SPR) binding assay

A CM5 sensor chip was used, and an HBS-EP+ buffer solution (10 mM HEPES, 150 mM NaCl, 3 mM EDTA, 0.05% surfactant P20) was used as the mobile phase. A 30 $\mu$g/mL solution of an anti-human IgG (Fc) antibody was prepared in a 10 mM sodium acetate buffer (pH 5.0), and amino coupling immobilization was performed. The test antibodies were diluted in the HBS-EP+ buffer solution and captured by the anti-human IgG (Fc) antibody on chip channels. h-FAP-His was dissolved in the HBS-EP+ buffer solution as an analyte, and the resulting solution was serially diluted 2-fold. The diluted antibodies were allowed to flow through the experimental and reference channels at a flow rate of 30 $\mu$L/min, with 1 min of association and 15 min of disassociation. 10 mM glycine pH 1.5 was run as a regeneration buffer at a flow rate of 10 $\mu$L/min for 30 s. Data were analyzed. The results in Table 4 show that the affinity of Ab10 for antigen FAP was 5.3 times higher than that of the control anti-FAP antibody 28H1 (the sequences 219 and 233 of the patent US9266938B2). However, the binding affinities of the other anti-FAP antibodies synchronously obtained by screening in the present disclosure, such as Ab2, Ab4, and Ab5 (their sequences are not shown), for the antigen were lower than that of 28H1.

Table 4. The SPR affinity Ka, Kd, and $K_D$ values of anti-FAP antibodies

| No. | ka (1/Ms) | kd (1/s) | $K_D$ (M) | Ratio of $K_D$ of 28H1 to $K_D$ of test antibody | Ratio of $K_d$ of 28H1 to $K_d$ of test antibody |
|---|---|---|---|---|---|
| 28H1 | 1.95E+05 | 2.55E-04 | 1.31E-09 | 1 | 1 |
| Ab2 | 1.62E+06 | 1.62E-02 | 9.98E-09 | 0.1 | 0.02 |
| Ab4 | 1.51E+06 | 6.17E-03 | 4.09E-09 | 0.3 | 0.04 |
| Ab5 | 1.08E+06 | 3.57E-03 | 3.32E-09 | 0.4 | 0.07 |

(continued)

| No. | ka (1/Ms) | kd (1/s) | $K_D$ (M) | Ratio of $K_D$ of 28H1 to $K_D$ of test antibody | Ratio of $K_d$ of 28H1 to $K_d$ of test antibody |
|---|---|---|---|---|---|
| Ab10 | 2.97E+05 | 7.28E-05 | 2.45E-10 | 5.3 | 3.50 |

1.7. FACS binding assay

A FACS assay was used to assess the binding properties of anti-FAP antibodies on cells. The assay included: A CHO cell line overexpressing human FAP was constructed and plated (1E5/well). The test antibodies were added at 100 μL/well. The highest concentration was 100 nM and was 5-fold diluted to obtain 8 concentrations in total. The plate was incubated at 4 °C for 1 h. Anti-hIgG Alexa Fluor-647 was added as a secondary antibody in a 1:500 ratio. The plate was incubated at 4 °C for 30 min, and a FACS assay was performed. Antibodies that bind to FAP can mark cells. The binding $EC_{50}$ value of the antibody Ab10 to human FAP was 0.0004320 μg/mL.

**Example 2. Screening for and Preparation of Anti-CD40 Nanobodies**

2.1. Sequences and preparation of immunizing and screening antigens

A His-tagged human CD40 recombinant protein (h-CD40-His), a C-terminally biotinylated human CD40 recombinant protein (h-CD40-biotin), and a C-terminally His-tagged monkey CD40 recombinant protein (cyno-CD40-His) were selected. Their sequences and sources are shown in Table 5. The protein reagents can be used in the experiments of the following examples.

Table 5. The amino acid sequences and sources of recombinant proteins

| Name | Start and end of amino acid sequence | Acrobiosystems cat. No. |
|---|---|---|
| h-CD40-His | | CD0-H5228 |
| h-CD40-biotin | Glu21-Arg193 | CD0-H82E8 |
| cyno-CD40-His | | CD0-C52H6 |

2.2. Alpaca immunization process and titer determination

An alpaca was immunized with h-CD40-his once every two weeks, and a total of four immunizations were performed. In the first immunization, 0.5 mg of antigen and 1 mL of Freund's complete adjuvant (CFA) were well mixed and injected subcutaneously. In the following three immunizations, 0.25 mg of antigen and 1 mL of Freund's incomplete adjuvant (IFA) were well mixed and injected subcutaneously. Blank serum was collected before the immunizations, and 50 mL of peripheral blood was collected one week after the third immunization and one week after the fourth immunization. Lymphocytes were isolated, and the serum titer was determined.

2.3. Titer determination and phage library construction

After the four immunizations of the alpaca, the serum titer was determined. After the titer was determined to be acceptable, PBMCs were isolated, and the total RNA was extracted. The purity was determined, and the RNA was reverse-transcribed into DNA. After two rounds of nested PCR, a purified vector and the VHH fragments of interest were digested with enzymes and ligated. Transfection was performed using electroporation, clones were selected, and phage libraries A and B were obtained.

2.4. Phage library affinity panning and ELISA identification

With the h-CD40 antigen as the target molecule, screening was performed by using a Gly-HCL acid elution method to elute specific phages. After two rounds of screening, 384 clones were randomly picked from the titer determination plates of the first round and the second round and screened by phage ELISA for positive clones, and the optical density at 450 nm was measured. Based on sequencing results, sequence alignments and phylogenetic tree analysis were performed on sequences. The sequence of A297 is shown below, with the CDRs defined using the Kabat numbering scheme underlined:

> A297

QVQLVESGGGGMVEPGGSLRLSCAASGFTFS<u>RYGMK</u>WVRQAPGKGPEWVS<u>TINS HGDTTYYADSVKG</u>RFTISRDNAQNTVYLQMNSLKPEDTALYYCLR<u>IDYSDYSS</u>R GQGTQVTVSS (SEQ ID NO: 11);

The CDR sequences are as follows:

Table 6. The CDRs of the anti-CD40 antibody A297

| Antibody No. | CDR sequences | | Sequence No. |
|---|---|---|---|
| A297 | CDR1 | RYGMK | SEQ ID NO:12 |
| | CDR2 | TINSHGDTTYYADSVKG | SEQ ID NO:13 |
| | CDR3 | IDYSDYSS | SEQ ID NO:14 |

2.5. Humanization of anti-CD40 nanobody

On the basis of the typical VHH structures of the obtained nanobody A297, the VHH variable region sequences were compared with an antibody germline database to obtain highly homologous human germline templates. A preferred human germline template for the antibody A297 of the present disclosure was IGHV3-48*03. The CDRs were then grafted into human FRs, and the key amino acids that affect the structure and function of the antibody were back-mutated to restore the binding ability and activity. The sequence of A297V3 is shown below, with the CDRs defined using the Kabat numbering scheme underlined:

> A297V3

EVQLVESGGGLVQPGGSLRLSCAASGFTFS<u>RYGMK</u>WVRQAPGKGLEWVS<u>TINS HGDTTYYADSVKG</u>RFTISRDNAKNSLYLQMNSLRAEDTAVYYCLR<u>IDYSDYSS</u>R GQGTLVTVSS (SEQ ID NO: 15)

2.6. Determination of binding abilities of anti-CD40 nanobodies to antigen

The above anti-CD40 nanobody sequences were linked to human IgG1-Fc (SEQ ID NO: 16) with mutations C220A, S267E, and L328F (numbered according to the Eu numbering scheme). Plasmids were constructed, transient transfection, expression, and purification were performed, and the antibody of interest was obtained. 9E5-SELFNS was used as a positive control CD40 agonist (see the sequences 58 and 59 of WO2020108611A1).

> IgG1-Fc

EPKSADKTHTCPPCPAPELLGGPSVFLFPPKPKDTLMISRTPEVTCVVVDV**E**HED PEVKFNWYVDGVEVHNAKTKPREEQYNSTYRVVSVLTVLHQDWLNGKEYKC KVSSKA**F**PAPIEKTISKAKGQPREPQVYTLPPSRDELTKNQVSLTCLVKGFYPSDI AVEWESNGQPENNYKTTPPVLDSDGSFFLYSKLTVDKSRWQQGNVFSCSVMHE ALHNHYTQKSLSLSPGK (SEQ ID NO: 16)

The binding abilities of the humanized antibodies to cells highly expressing the human antigen protein CD40 were determined by FACS. HEK293 cells were transiently transfected with CD40 plasmids (Sino Biological; HG10774-CF) to obtain HEK293 cells highly expressing CD40. The resulting cells were resuspended in a flow cytometry staining buffer (PBS + 2% FBS), and the test antibodies, serially diluted to different concentrations, were added. After 1 h of incubation on ice, the cells were washed with PBS and centrifuged at 400 g for 5 min. A goat anti-human Fc antibody labeled with the fluorescent group Alexa Fluor 647 was added as a secondary antibody. The cells were stained in an ice bath for 1 h and washed twice with PBS, and the fluorescent signals on the cell surface were then detected by FACS. The $EC_{50}$ values are shown in Table 7. The humanized antibodies (the sequence of A297V4 is not shown) all exhibited relatively high affinities for the cell strain highly expressing CD40.

Table 7. The $EC_{50}$ values for the affinities of humanized anti-CD40 antibodies for a cell strain highly expressing human CD40

| Antibody | $EC_{50}$ (nM) | Emax MFI |
|---|---|---|
| A297 | 1.12 | 16728 |
| A297V3 | 1.25 | 12827 |
| A297V4 | 2.70 | 8255 |
| 9E5-SELFNS | 1.12 | 12120 |

The activation of HEK-BlueTM CD40L cells. The *in vitro* agonistic activity of the CD40 antibodies was assessed by assessing the activation of HEK-Blue™CD40L cells by the CD40 antibodies when crosslinked with FcγRIIb. The mutations S267E/L328F contained in the Fc of the humanized CD40 antibodies enhance the affinity of IgG1 Fc for FcγRIIb and thus enhance the crosslinking of the antibodies by FcγRIIb. The agonist activity of the CD40 antibodies when fully crosslinked was simulated by the FcγRIIb-mediated activation of HEK-Blue™ CD40L cells by the CD40 antibodies. HEK293 cells were transiently transfected with FcγRIIb plasmids (CD32B/Fcgr2b cDNA ORF Clone, Human, N-His tag; Sino Biological; HG10259-NH) to obtain HEK293 cells highly expressing FcγRIIb. HEK-Blue™CD40L cells were plated onto a 96-well cell culture plate at 5E4/well (the culture medium was DMEM, 10% FBS, 100 μg/mL Normocin), and the HEK293 cells expressing FcγRIIb were added at 5E4/well. The test antibodies, serially diluted, were added at 100 μL/well, and the plate was incubated overnight at 37 °C. The cells were centrifuged. 20 μL of the cell supernatant was transferred to a new 96-well plate, and 180 μL of QUANTI-Blue substrate solution was added. The plate was incubated in a dark place for 30 min. The absorbance at 620 nm was measured using an Envision microplate reader, and the $EC_{50}$ values and the Emax values (relative to the fluorescence intensity of the antibody-free group) were calculated. The *in vitro* cell agonist activity of the CD40 antibodies was assessed based on the $EC_{50}$ values. The $EC_{50}$ values and the Emax values (relative fluorescence intensity) are shown in Table 8. The results show that in the above reporter gene cell system, the humanized CD40 antibodies all exhibited relatively strong agonist activity when crosslinked with FcγRIIb.

Table 8. The activation of HEK-Blue™CD40L cells by humanized anti-CD40 antibodies

| Antibody | FcγRIIb-mediated activation of HEK-Blue™CD40L cells | |
|---|---|---|
| | $EC_{50}$ (nM) | Emax (relative fluorescence intensity) |
| A297 | 0.243 | 17.34 |
| A297_V3 | 0.006 | 12.84 |
| A297_V4 | 0.038 | 13.31 |
| 9E5-SELFNS | 0.009 | 16.05 |

## Example 3. Preparation of and Screening for Anti-4-1BB Nanobodies

3.1. Alpaca immunization, titer determination, and phage library affinity panning

An alpaca was immunized with a His-tagged human 4-1BB recombinant protein (Acrobiosystems, 41B-H5227) once every two weeks, and a total of four immunizations were performed. In the first immunization, 0.5 mg of antigen and 1 mL of Freund's complete adjuvant (CFA) were well mixed and injected subcutaneously. In the following three immunizations, 0.25 mg of antigen and 1 mL of Freund's incomplete adjuvant (IFA) were well mixed and injected subcutaneously. Blank serum was collected before the immunizations, and 50 mL of peripheral blood was collected one week after the third immunization and one week after the fourth immunization. PBMCs were isolated, and the total RNA was extracted. The purity was determined, and reverse transcription was performed to obtain DNA. After two rounds of nested PCR, the nanobody fragments of interest were ligated to a phage display vector. Transfection was performed using electroporation, and a phage library was obtained.

> Human 4-1BB protein sequence

LQDPCSNCPAGTFCDNNRNQICSPCPPNSFSSAGGQRTCDICRQCKGVFRTRKEC
SSTSNAECDCTPGFHCLGAGCSMCEQDCKQGQELTKKGCKDCCFGTFNDQKR
GICRPWTNCSLDGKSVLVNGTKERDVVCGPSPADLSPGASSVTPPAPAREPGHSP
Q (SEQ ID NO: 17)

In order to obtain anti-4-1BB nanobodies that simultaneously recognize humans and monkeys, a strategy of two rounds of cross-screening with human and monkey antigens was adopted. The antigens used in the first and second rounds of screening were human 4-1BB and monkey 4-1BB, or monkey 4-1BB and human 4-1BB, respectively. In each round of screening, phages specifically binding to 4-1BB were eluted using a Gly-HCl acid elution method. Ninety-six clones (192 clones in total) were randomly picked from the titer determination plates of the first round and the second round and screened by phage ELISA for positive clones, and the optical density at 450 nm was measured. The positive clones were sequenced. Based on the sequencing results, sequence alignments and phylogenetic tree analysis were performed, and 14 distinctive sequences, including H27, C5, C145, etc., were selected. The C5 sequence is shown below.

> C5

QVQLVESGGGLVQPGGSLRLSCSASGFTFSS<u>YAMS</u>WVRQAPGKGLEWVS<u>DINS
GGESTFYEDSVKG</u>RFTISRDNAKNTLYLQMNSLKPEDTAVYYCAK<u>HPLTFTIAT
MNDYDY</u>WGQGTQVTVSS (SEQ ID NO: 18);

The CDR sequences are as follows:

Table 9. The CDRs of the anti-4-1BB nanobody C5 (Kabat numbering scheme)

| Antibody No. | CDR sequences | | Sequence No. |
|---|---|---|---|
| C5 | CDR1 | SYAMS | SEQ ID NO:19 |
| | CDR2 | DINSGGESTFYEDSVKG | SEQ ID NO:20 |
| | CDR3 | HPLTFTIATMNDYDY | SEQ ID NO:21 |

The sequence of C5 was linked to human IgG1-Fc with mutations C220A, S267E, and L328F (numbered according to the Eu numbering scheme). The resulting VHH-Fc fusion protein sequence is shown below. Furthermore, mutations such as L234A, L235A, and N297A (according to the Eu numbering scheme) were introduced into the Fc of human IgG1 to completely remove the FcγR-mediated effector function of the antibody, and S228P (according to the Eu numbering scheme) was introduced into the Fc of human IgG4 to stabilize the antibody molecule, preventing half-molecule formation. Both are alternative IgG Fcs.

The antibody sequence in which C5 is linked to an IgG1-Fc (containing mutations C220A, S267E, and L328F) is shown by way of example and set forth in SEQ ID NO: 22, with the IgG1-Fc sequence underlined:

> C5-Fc

QVQLVESGGGLVQPGGSLRLSCSASGFTFSSYAMSWVRQAPGKGLEWVSDINS
GGESTFYEDSVKGRFTISRDNAKNTLYLQMNSLKPEDTAVYYCAKHPLTFTIAT
MNDYDYWGQGTQVTVSS<u>EPKSADKTHTCPPCPAPELLGGPSVFLFPPKPKDTL
MISRTPEVTCVVVDVEHEDPEVKFNWYVDGVEVHNAKTKPREEQYNSTYRVV
SVLTVLHQDWLNGKEYKCKVSNKAFPAPIEKTISKAKGQPREPQVYTLPPSREE
MTKNQVSLTCLVKGFYPSDIAVEWESNGQPENNYKTTPPVLDSDGSFFLYSKLT
VDKSRWQQGNVFSCSVMHEALHNHYTQKSLSLSPGA</u> (SEQ ID NO: 22)

Plasmids were constructed and transiently transfected into cells, and antibodies were expressed and purified. Analysis showed that the antibodies of interest were obtained. 3.2. Assays for antigen-binding activity and agonist activity of anti-4-1BB antibodies

(1) Assay for binding ability to 4-1BB antigen

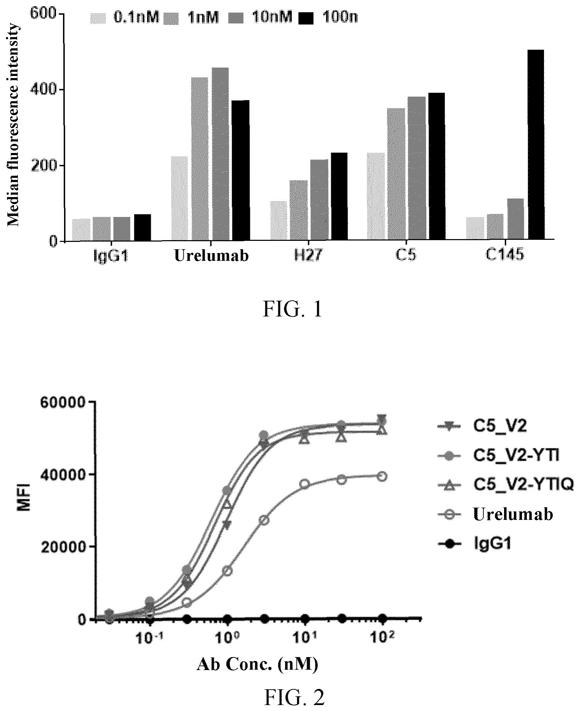

The binding activity of the anti-4-1BB single-domain antibodies to human 4-1BB protein was determined using a flow cytometer. HEK293-Hu4-1BB cells were obtained by transiently transfecting HEK293 cells (ATCC CRL-1573) with a gene expressing human 4-1BB protein (CD137 cDNA ORF Clone, Human, C-OFPSpark tag; purchased from Sino Biological, Cat#HG10041-ACR). The cell culture medium was DMEM (Gibco, Cat#11995065) containing 10% fetal bovine serum. The experimental culture medium was sterile PBS (phosphate-buffered saline, pH 7.40) containing 2% fetal bovine serum. The HEK293-Hu4-1BB cells were washed twice with the experimental culture medium and seeded into a 96-well U-bottom plate at $1 \times 10^5$ HEK293-Hu4-1BB cells/well, and the test anti-4-1BB antibody VHH-Fc samples, at different concentrations, were added. The cells were incubated at 4 °C for 1 h and then washed twice with the experimental culture medium. Subsequently, a goat anti-human IgG (H + L) Alexa Fluor 488 antibody (Thermo, Cat#A11013) was added. After two washes, the fluorescence signals were measured using a flow cytometer. Urelumab was used as a positive control. The MFI values for the antibodies are shown in FIG. 1. The results show that the related anti-4-1BB antibodies exhibited different binding abilities to 4-1BB on the surface of HEK293-Hu4-1BB cells, and C5 exhibited good cell membrane surface antigen-binding activity.

(2) Assay for agonist activity on 4-1BB signaling pathway

The agonist activity of anti-4-1BB antibodies was evaluated using a 4-1BB/NF-κB reporter gene. HEK293-Hu4-1BB/NF-κB double-transfected cells were obtained by transiently transfecting HEK293 cells (ATCC CRL-1573) with a gene expressing human 4-1BB (CD137 cDNA ORF Clone, Human, C-OFPSpark tag; purchased from Sino Biological, Cat#HG10041-ACR) and the NF-κB reporter gene (pGL4.32[luc2P/NF-κB-RE/Hygro] Vector, purchased from Promega, Cat#E849A). 4-1BB activation can be characterized by the activation level of the NF-κB signaling pathway. HEK293 cells highly expressing FcγRIIb were obtained by transiently transfecting HEK293 cells with FcγRIIb plasmids (CD32B/Fcgr2b cDNA ORF Clone, Human, N-His tag; purchased from Sino Biological, Cat#HG10259-NH). The cell culture medium was DMEM (Gibco, Cat#11995065) containing 10% fetal bovine serum. HEK293-Hu4-1BB/NF-κB cells ($2 \times 10^6$/mL) were plated onto a 96-well cell culture plate at 50 μL, 40 μL of culture medium or FcγRIIb-expressing HEK293 cells ($2.5 \times 10^6$/mL) was added, and 10× serially diluted test anti-4-1BB antibodies were added at 10 μL/well. The plate was incubated at 37 °C for 6 h. The cells were taken out. An equal volume of Bio-Glo Luciferase Assay System reagent (Promega, Cat#G7940) was added to each well, and the plate was incubated for 5 min in a dark place. The fluorescence signals were measured using an Envision microplate reader (PerkinElmer, 2150). The $EC_{50}$ values and the Emax values (relative to the fluorescence intensity of the antibody-free group) were calculated, and the *in vitro* cell agonist activity of the anti-4-1BB antibodies was assessed based on the $EC_{50}$ values. The results are shown in Table 10. The results show that when no FcγRIIb (i.e., CD32b) was added for positive crosslinking, the related anti-4-1BB antibodies exhibited much weaker activation of the 4-1BB/NF-κB luciferase reporter gene signaling pathway than the control urelumab, indicating that the anti-4-1BB antibodies of the present disclosure are safer; after FcγRIIb was added for crosslinking, the related anti-4-1BB antibodies exhibited significant activation of the 4-1BB/NF-κB luciferase reporter gene signaling pathway, and the activation ability of C5 was the strongest and comparable to that of the control urelumab. Based on these activity results, the sequence C5, which exhibited low background activation before FcγRIIb crosslinking and stronger activation activity after FcγRIIb crosslinking, was selected and humanized.

Table 10. The $EC_{50}$ values for the agonistic activity of anti-4-1BB antibodies on the 4-1BB/NF-κB luciferase reporter gene

| No. | FcγRIIb crosslinking | | No FcγRIIb crosslinking | |
|---|---|---|---|---|
| | Emax | $EC_{50}$ (nM) | Emax | $EC_{50}$ (nM) |
| IgG | 1.12 | - | 1.10 | - |
| Urelumab | 9.39 | 0.027 | 4.30 | 2.185 |
| C5 | 10.04 | ~ 0.8144 | 2.26 | - |
| (Note: "-" indicates that the measurement was beyond the limit of detection) | | | | |

3.3. Humanization engineering

The amino acids of the CDRs and FRs (human framework regions) of the C5 sequence were numbered using the Kabat numbering scheme. The FR1+CDR1, FR2+CDR2, and FR3+CDR3 sequences were matched with an antibody germline database to obtain FR human germline templates with relatively high homology. FR1 was derived from IGHV3-64*04, FR2 was derived from IGHV3-23*03, and FR3 was derived from IGHV3-74*01. The above human germline FRs were inserted into the original sequences as replacements to reduce the immunogenicity produced in the human body. The key amino acids that affect the structure and function of the antibody were back-mutated to restore the binding ability and activity. The humanized sequences are shown below.

> C5_V1

QVQLVESGGGLVQPGGSLRLSCSASGFTFS<u>SYAMS</u>WVRQAPGKGLEWVS<u>DINS GGESTFYEDSVKG</u>RFTISRDNAKNTLYLQMNSLRAEDTAVYYCAR<u>HPLTFTIAT MNDYDY</u>WGQGTLVTVSS (SEQ ID NO: 23);

> C5_V2

QVQLVESGGGLVQPGGSLRLSCSASGFTFS<u>SYAMS</u>WVRQAPGKGLEWVS<u>DINS GGESTFYEDSVKG</u>RFTISRDNAKNTLYLQMNSLRAEDTAVYYCAK<u>HPLTFTIAT MNDYDY</u>WGQGTLVTVSS (SEQ ID NO: 24);

> C5_V3

QVQLVESGGGLVQPGGSLRLSCSASGFTFS<u>SYAMS</u>WVRQAPGKGLEWVS<u>DINS GGESTFYEDSVKG</u>RFTISRDNAKNTLYLQMNSLKPEDTAVYYCAK<u>HPLTFTIAT MNDYDY</u>WGQGTLVTVSS (SEQ ID NO: 25);

(Note: The CDRs are underlined)

The above 3 humanized sequences were each linked to human IgG1-Fc (SEQ ID NO: 16). Plasmids were constructed, and transient transfection, expression, and purification were performed.

3.4. Assays for antigen-binding activity and agonist activity of humanized anti-4-1BB antibodies

(1) Assay for binding ability to 4-1BB antigen

The antigen-binding activity of the humanized antibodies was determined using a FACS assay as described in 3.2. The results are shown in Table 11. The results show that the related humanized anti-4-1BB antibodies exhibited different binding abilities to 4-1BB on the surface of HEK293-Hu4-1BB cells, and C5_V1, C5_V2, and C5_V3 all maintained good binding activity.

Table 11. The $EC_{50}$ values for the affinities of anti-4-1BB antibodies for a cell strain highly expressing human 4-1BB before/after humanization

| Antibody No. | $EC_{50}$ (nM) | Maximum fluorescence value |
|---|---|---|
| C5 | 3.196 | 1101 |
| C5_V1 | 3.789 | 1036 |
| C5_V2 | 1.624 | 1031 |
| C5_V3 | 1.651 | 1096 |
| Urelumab | 1.184 | 1005 |

(2) Assay for agonist activity on 4-1BB signaling pathway

The activation of 4-1BB/NF-κB signaling by the humanized antibodies was assessed using an NF-κB luciferase reporter gene assay as described in 3.2. The results are shown in Table 12. The results show that after FcγRIIb was added for crosslinking, the related humanized anti-4-1BB antibodies exhibited activation of the 4-1BB/NF-κB luciferase reporter gene signaling pathway, and the activation abilities of C5, C5 _V1, C5_V2, and C5_V3 were comparable to that of the control urelumab.

Table 12. The activation of the NF-κB signaling pathway by anti-4-1BB antibodies before/after humanization

| Antibody No. | Activation of HEK293-4-1BB cells | | FcγRIIb-mediated activation of HEK293-4-1BB cells | |
|---|---|---|---|---|
| | Activity | Fold change in Emax | Activity | Fold change in Emax |
| C5 | - | 1.08 | +++ | 2.71 |
| C5_V1 | - | 1.14 | ++ | 2.28 |
| C5_V2 | - | 1.37 | ++ | 2.02 |
| C5_V3 | - | 1.01 | + | 1.83 |
| Urelumab | + | 1.70 | +++ | 2.61 |
| (Note: For a <1.5-fold change in Emax, the activity is defined as "-"; for 1.5-2, as "+"; for 2-2.5, as "++"; for 2.5-3, as "+++"; and for >3, as "++++".) | | | | |

3.5. Removal of TCE sites

Subsequently, we performed T-cell Epitope (TCE) prediction on the C5_V2 sequence and engineered the CDR3 sequence of C5_V2 based on the prediction results to reduce the number of TCEs. The F in the CDR3 was mutated to Y (F99Y, according to the Kabat numbering scheme) to obtain a TCE-optimized version of the C5_V2 sequence. This version was designated C5_V2-YTI, and its sequence is as follows:

> C5_V2-YTI

QVQLVESGGGLVQPGGSLRLSCSASGFTFSS<u>SYAMS</u>WVRQAPGKGLEWVS<u>DINS GGESTFYEDSVKG</u>RFTISRDNAKNTLYLQMNSLRAEDTAVYYCAK<u>HPLTYTIAT MNDYDY</u>WGQGTLVTVSS (SEQ ID NO: 26);

That is, according to the Kabat numbering scheme, the amino acid sequence of the CDR1 in C5_V2-YTI is set forth in SEQ ID NO: 19, the amino acid sequence of the CDR2 is set forth in SEQ ID NO: 20, and the amino acid sequence of the CDR3 is HPLTYTIATMNDYDY (SEQ ID NO: 27).
The above C5_V2-YTI sequence was linked to human IgG1-Fc (SEQ ID NO: 16). Plasmids were constructed, transient transfection, expression, and purification were performed, and the antibody of interest was obtained.

3.6. Assay for binding ability of humanized 4-1BB antibody to antigen after TCE removal

The humanized antibody C5_V2 was optimized by TCE removal, and the resulting antibody was C5_V2-YTI. The antigen-binding activities of C5_V2 and C5_V2-YTI were compared using a FACS assay as described in 3.2. The results are shown in Table 13. The results show that the related antibody C5_V2-YTI maintained good binding activity to 4-1BB on the surface of HEK293-Hu4-1BB cells, and its binding activity was comparable to that of C5_V2.

Table 13. The $EC_{50}$ values for the affinities of C5_V2 and C5_V2-YTI for a cell strain highly expressing human 4-1BB

| Antibody No. | $EC_{50}$ (nM) | Maximum fluorescence value |
|---|---|---|
| C5_V2 | 0.98 | 53,669 |
| C5_V2-YTI | 0.62 | 53,825 |
| Urelumab | 1.64 | 39,666 |
| IgG1 | - | 67 |

3.7. Reduction of risk of antibody aggregation

We found that the C-terminal surface of the antibody C5_V2-YTI had a relatively large hydrophobic region that could lead to the risk of antibody aggregation. By mutating Leu (L), which is a hydrophobic amino acid, in the TLVTVSS sequence to Gln (Q), which is a hydrophilic amino acid, the area of the C-terminal hydrophobic surface region of the antibody can be effectively reduced, thereby lowering the risk of antibody aggregation. Further, to optimize the pI value of the sequence, two acidic amino acids (Glu (E)) in the sequence were mutated to Gln (Q), which is a basic amino acid, and Ala (A), which is a neutral amino acid, respectively. The engineered antibody was designated C5_V2-YTIQ, and its sequence is as follows:

> C5_V2-YTIQ

QVQLVESGGGLVQPGGSLRLSCSASGFTFSS<u>SYAMS</u>WVRQAPGKGLEWVS<u>DINS
GGQSTFYADSVKG</u>RFTISRDNAKNTLYLQMNSLRAEDTAVYYCAK<u>HPLTYTIAT
MNDYDY</u>WGQGT**Q**VTVSS (SEQ ID NO: 28);

That is, according to the Kabat numbering scheme, the amino acid sequence of the CDR1 in C5_V2-YTIQ is set forth in SEQ ID NO: 19, the amino acid sequence of the CDR2 is DINSGGQSTFYADSVKG (SEQ ID NO: 29), and the amino acid sequence of the CDR3 is set forth in SEQ ID NO: 27.
That is, the above anti-4-1BB nanobodies have structures of the following general formulas:

the CDR1 is SYAMS (SEQ ID NO: 19);
the CDR2 is DINSGGX$_1$STFYX$_2$DSVKG (SEQ ID NO: 30), where X$_1$ is E or Q, and X$_2$ is E or A;
the CDR3 is HPLTX$_3$TIATMNDYDY (SEQ ID NO: 31), where X$_3$ is F or Y.

3.8. Assay for binding abilities of engineered anti-4-1BB nanobodies to antigen

C5_V2-YTI and C5_V2-YTIQ were linked to human IgG1-Fc (SEQ ID NO: 16). Plasmids were constructed, transient transfection, expression, and purification were performed, and the antibody of interest was obtained.
The antigen-binding activities of C5_V2, C5_V2-YTI, and C5_V2-YTIQ were compared using a FACS assay. The results are shown in FIG. 2 and Table 14, with urelumab as a positive control. The results show that C5_V2, C5_V2-YTI, and C5_V2-YTIQ all maintained good binding activity to 4-1BB on the surface of HEK293-Hu4-1BB cells.

Table 14. The EC$_{50}$ values for the affinities of anti-4-1BB nanobodies for a cell strain highly expressing human 4-1BB

| Antibody No. | EC$_{50}$ (nM) | Maximum fluorescence value |
|---|---|---|
| C5_V2 | 0.98 | 53,669 |
| C5_V2-YTI | 0.62 | 53,825 |
| C5_V2-YTIQ | 0.70 | 51,664 |
| Urelumab | 1.64 | 39,666 |
| IgG1 | - | 67 |

**Example 4. Design and Preparation of Anti-FAP/4-1BB/CD40 Multispecific Antibodies**

4.1. Design, expression, and purification of anti-FAP/4-1BB/CD40 multispecific antibodies

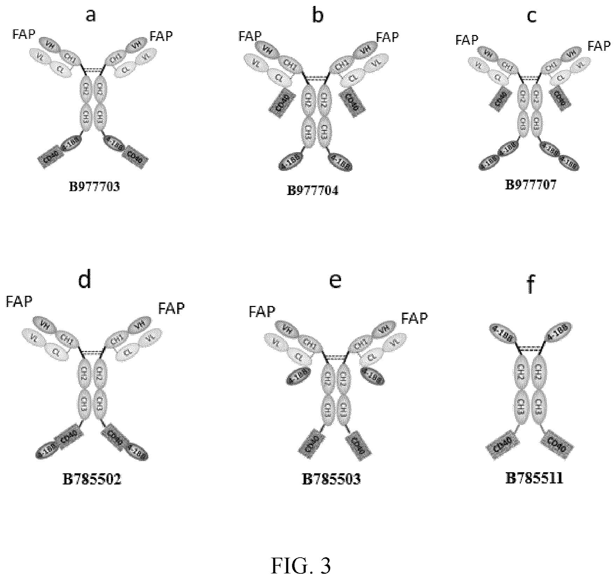

Fusion proteins of FAP, 4-1BB, and CD40 were constructed, and their molecular formats are schematically shown in FIG. 3.
In the molecule B977703 shown in a of FIG. 3, the C-termini of the two heavy chains of an anti-FAP monoclonal antibody (Ab10) are linked to the N-termini of anti-4-1BB nanobodies (C5V2-YTI), respectively, by (G$_4$S)$_2$ linkers, and the C-termini of the two anti-4-1BB nanobodies are linked to the N-termini of anti-CD40 nanobodies (A297V3), respectively, by (G$_4$S)$_2$ linkers.
In the molecule B977704 shown in b of FIG. 3, the C-termini of the two heavy chains of an anti-FAP monoclonal antibody (Ab10) are linked to the N-termini of anti-4-1BB nanobodies (C5V2-YTI), respectively, by (G$_4$S)$_2$ linkers, and the

C-termini of the two light chains of the anti-FAP monoclonal antibody (Ab10) are linked to the N-termini of anti-CD40 nanobodies (A297V3), respectively, by $(G_4S)_2$ linkers.

In the molecule B977707 shown in c of FIG. 3, two anti-4-1BB nanobodies (C5V2-YTI) were linked head to tail in sequence by a $(G_4S)_2$ linker, and the N-termini are linked to the C-termini of the two heavy chains of an anti-FAP monoclonal antibody (Ab10), respectively, by $(G_4S)_2$ linkers; the C-termini of the two light chains of the anti-FAP monoclonal antibody (Ab10) are linked to the N-termini of anti-CD40 nanobodies (A297V3), respectively, by $(G_4S)_2$ linkers.

In the molecule B785502 shown in d of FIG. 3, the C-termini of the two heavy chains of an anti-FAP monoclonal antibody (Ab10) are linked to the N-termini of anti-CD40 nanobodies (A297V3), respectively, by $(G_4S)_2$ linkers, and the C-termini of the two anti-CD40 nanobodies (A297V3) are linked to the N-termini of anti-4-1BB nanobodies (C5V2-YTI), respectively, by $(G_4S)_2$ linkers.

In the molecule B785503 shown in e of FIG. 3, the C-termini of the two heavy chains of an anti-FAP monoclonal antibody (Ab10) are linked to the N-termini of anti-CD40 nanobodies (A297V3), respectively, by $(G_4S)_2$ linkers, and the C-termini of the two light chains of the anti-FAP monoclonal antibody (Ab10) are linked to the N-termini of anti-4-1BB nanobodies (C5V2-YTI), respectively, by $(G_4S)_2$ linkers.

The molecule B785511 shown in f of FIG. 3 is a specific antibody 4-1BB/CD40 used as a control. In the molecule, two anti-4-1BB nanobodies (C5V2-YTI) are linked to the N-termini of an Fc region by the heavy chain hinge region of IgG1, and the C-termini of the Fc region are linked to the N-termini of anti-CD40 nanobodies (A297V3), respectively, by $(G_4S)_2$ linkers.

The Fc in the molecules shown in a-f of FIG. 3 is human IgG1 Fc with mutations L234A and L235A, which remove Fc$\gamma$R-mediated effector functions.

4.2. Engineering design, expression, and purification of anti-FAP/4-1BB/CD40 multispecific antibodies

To reduce binding to pre-existing anti-drug antibodies (pre-ADAs) or anti-drug antibodies (ADAs), the C-terminal nanobodies of the anti-FAP/4-1BB/CD40 multispecific antibodies were engineered by mutating the C-terminal TVSS sequences of the nanobodies to TVSAA. The optimized sequence for A297V3 was A297V3_AA, and the optimized sequence for C5_V2-YTI was C5_V2-YTI_AA.

> A297V3_AA

EVQLVESGGGLVQPGGSLRLSCAASGFTFSRYGMKWVRQAPGKGLEWVSTINS HGDTTYYADSVKGRFTISRDNAKNSLYLQMNSLRAEDTAVYYCLRIDYSDYSSR GQGTLVTVSAA (SEQ ID NO: 32)

> C5_V2-YTI_AA

QVQLVESGGGLVQPGGSLRLSCSASGFTFSSYAMSWVRQAPGKGLEWVSDINS GGESTFYEDSVKGRFTISRDNAKNTLYLQMNSLRAEDTAVYYCAKHPLTYTIAT MNDYDYWGQGTLVTVSAA (SEQ ID NO: 33)

The anti-CD40 nanobodies (A297V3) at the C-termini of the heavy chains in B977703 were engineered into A297V3_AA to obtain B21326101; the anti-CD40 nanobodies (A297V3) at the C-termini of the light chains in B977704 were engineered into A297V3_AA, and the anti-4-1BB nanobodies (C5_V2-YTI) at the C-termini of the heavy chains in B977704 were engineered into C5_V2-YTI_AA to obtain B21326102. The specific molecular sequences are shown in Table 32.

4.3. Engineering design, expression, and purification of anti-FAP/4-1BB/CD40 multispecific antibodies with reduced antibody aggregation

To reduce aggregation, we further engineered B21326101 by replacing C5_V2-YTI with C5_V2-YTIQ (SEQ ID NO: 28). The optimized molecule was designated B21546901.

4.4. Control molecules

BNT312 was a CD40/4-1BB bispecific antibody molecule, Ab10-A12V2-2 was a FAP/CD40 bispecific antibody molecule, RO7300490 was a FAP/CD40 bispecific antibody molecule, RO7122290 was a FAP/4-1BBL bispecific antibody molecule, and MP0310 was a FAP/4-1BB bispecific antibody molecule. All were used as control molecules.

The sequences of the above multispecific antibodies are shown in Table 32. The sequences of the control molecules are detailed in Table 33.

4.5. Expression and purification

Transient transfection and expression: Taking a 30 mL expression system as an example, 60 μL of transfection reagent was diluted with the culture medium and then mixed well with 15 μg of plasmids. After 15 min of incubation at 37 °C, the mixed transfection solution was added dropwise to a cell suspension with shaking. The cells were cultured for expression on a shaker for one week, and the supernatant was collected and centrifuged at 8000 rpm for 5 min.

Antibody purification: First, a Protein A affinity chromatography column was equilibrated with 20 mL of 1× PBS at a flow rate of 1 mL/min. The sample was loaded at a flow rate of 1 mL/min. 20 mL of 1× PBS was passed through the column at a flow rate of 1 mL/min to remove impurities. Elution was performed with a citric acid buffer (pH 3.4) at 1 mL/min, and the eluate was collected in tubes. The absorbance at 280 nm was measured using a NanoDrop spectrophotometer. Finally, the high-concentration protein was transferred to a dialysis bag and dialyzed against 1× PBS in a beaker. Analysis showed that the antibodies of interest were obtained.

**Example 5. Assays for Antigen-Binding Activity of Anti-FAP/4-1BB/CD40 Multispecific Antibodies**

5.1. Assay for antigen-binding activity of anti-FAP/4-1BB/CD40 multispecific antibodies

The binding activity of anti-FAP/4-1BB/CD40 multispecific antibodies to human FAP/4-1BB/CD40 proteins was determined by FACS. CHO-K1-HuFAP cells were obtained by stably transfecting CHO-K1 cells with a gene expressing human FAP protein. The cell culture medium was Ham's F12 + GlutaMAXTM-I (Gibco, Cat#31765035) containing 10% fetal bovine serum and 800 μg/mL hygromycin B. HEK293-Hu4-1BB cells and HEK293-HuCD40 cells were HEK293 cells overexpressing human 4-1BB protein and HEK293 cells overexpressing human CD40 protein, respectively. The cell culture medium was DMEM (Gibco, Cat#11965092) containing 10% fetal bovine serum and 100 μg/mL hygromycin B. The experimental culture medium was sterile PBS (phosphate-buffered saline, pH 7.40) containing 2% fetal bovine serum. The CHO-K1-HuFAP, HEK293-Hu4-1BB, or HEK293-HuCD40 cells were washed twice with the experimental culture medium and seeded into a 96-well U-bottom plate at $1 \times 10^5$ cells/well, and test samples at different concentrations were added. The cells were incubated at 4 °C for 1 h and then washed twice with the experimental culture medium. Subsequently, the Alexa Fluor 647-mouse anti-human (IgG, Fcγ fragment specific) antibody (Jackson, Cat#209-605-098) was added. After two washes, the fluorescence signals were measured using a flow cytometer.

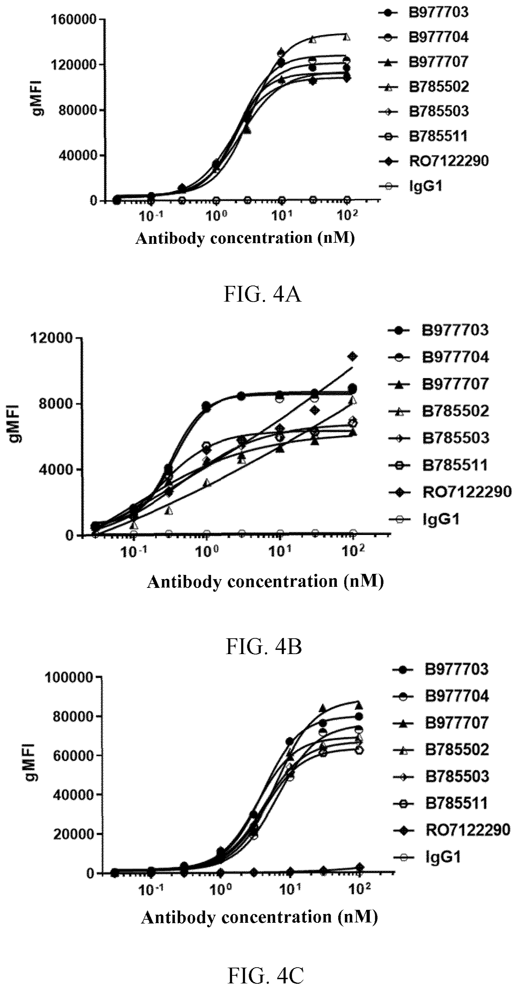

The FACS assay results in Table 15 and FIG. 4A show that the FAP/4-1BB/CD40 multispecific antibodies exhibited strong binding abilities to FAP on the surface of CHO-K1-HuFAP cells, and the binding abilities were comparable to that of the control antibody RO7122290, indicating that the binding of the antibody to FAP was not affected after the antibody was prepared into the multispecific antibodies. The results in Table 16 and FIG. 4B show that the FAP/4-1BB/CD40 multispecific antibodies exhibited good binding abilities to 4-1BB on the surface of HEK293-Hu4-1BB cells. The results in Table 17 and FIG. 4C show that the FAP/4-1BB/CD40 multispecific antibodies bound well to CD40 on the surface of HEK293-HuCD40 cells, indicating that the binding of the antibody to CD40 was not affected after the antibody was prepared into the multispecific antibodies.

Table 15. The results of the binding of the FAP/4-1BB/CD40 multispecific antibodies to CHO-K1-HuFAP cells

| Antibody name | EC$_{50}$ (nM) | Maximum fluorescence value |
|---|---|---|
| B977703 | 2.23 | 120,837 |
| B977704 | 2.24 | 127,457 |
| B977707 | 2.40 | 113,142 |
| B785502 | 3.38 | 146,921 |
| B785503 | 1.78 | 107,922 |
| B785511 | - | 74 |
| RO7122290 | 1.92 | 111,818 |

(continued)

| Antibody name | EC$_{50}$ (nM) | Maximum fluorescence value |
|---|---|---|
| IgG1 | / | 63 |

Table 16. The results of the binding of the FAP/4-1BB/CD40 multispecific antibodies to HEK293-Hu4-1BB cells

| Antibody name | EC$_{50}$ (nM) | Maximum fluorescence value |
|---|---|---|
| B977703 | 0.360 | 8,636 |
| B977704 | 0.332 | 8,516 |
| B977707 | 0.093 | 6,196 |
| B785503 | 0.439 | 6,730 |
| B785511 | 0.251 | 6,285 |
| RO7122290 | - | 10,807 |
| IgG1 | - | 69 |

Table 17. The results of the binding of the FAP/4-1BB/CD40 multispecific antibodies to HEK293-HuCD40 cells

| Antibody name | EC$_{50}$ (nM) | Maximum fluorescence value |
|---|---|---|
| B977703 | 4.05 | 79,686 |
| B977704 | 6.54 | 75,577 |
| B977707 | 6.23 | 88,116 |
| B785502 | 3.39 | 68,919 |
| B785503 | 4.22 | 66,687 |
| B785511 | 4.21 | 63,184 |
| RO7122290 | - | 2334 |
| IgGl | - | 61 |

5.2. Assay for antigen-binding activity of anti-FAP/4-1BB/CD40 multispecific antibodies

The antigen-binding activity of the FAP/4-1BB/CD40 multispecific antibodies B977703, B977704, B21326101, and B21326102 was determined using the method provided in Example 5.1, with RO7122290, Ab10-A12V2-2, BNT312, and IgG1 as controls.

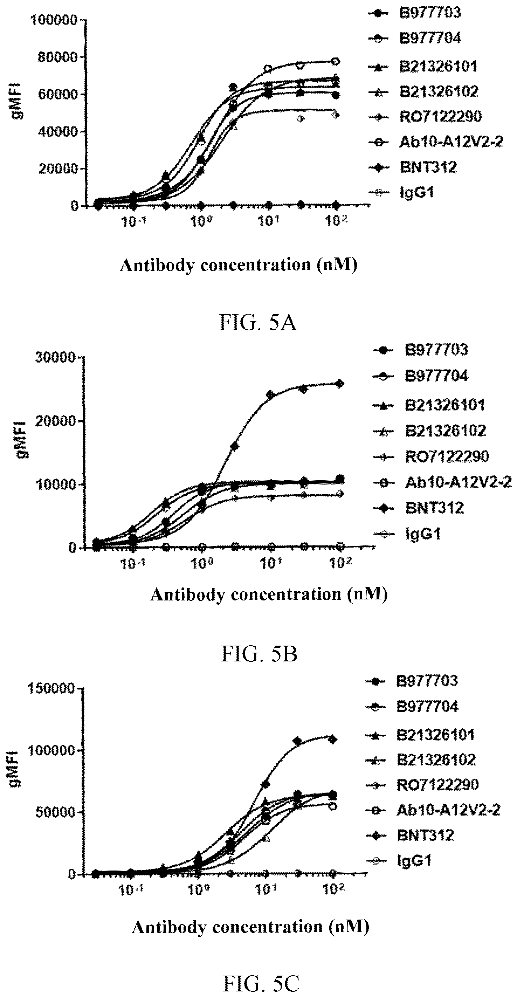

The FACS assay results (Tables 18-20 and FIGs. 5A-5C) show that the FAP/4-1BB/CD40 multispecific antibodies exhibited strong binding abilities to FAP on the surface of CHO-K1-HuFAP cells (Table 18 and FIG. 5A). Table 19 and FIG. 5B show that the FAP/4-1BB/CD40 multispecific antibodies exhibited good binding abilities to 4-1BB on the surface of HEK293-Hu4-1BB cells, and the multispecific antibodies after 4-1BB sequence engineering did not differ in 4-1BB binding, indicating that the 4-1BB sequence alteration did not affect target binding. Table 20 and FIG. 5C show that the FAP/4-1BB/CD40 multispecific antibodies exhibited binding abilities to CD40 on the surface of HEK293-HuCD40 cells, indicating that the pre-ADA removal engineering in the CD40 sequence did not significantly affect target binding.

Table 18. The results of the binding of the FAP/4-1BB/CD40 multispecific antibodies to CHO-K1-HuFAP cells

| Antibody name | EC$_{50}$ (nM) | Maximum fluorescence value |
|---|---|---|
| B977703 | 1.24 | 60,689 |
| B977704 | 0.92 | 66,984 |
| B21326101 | 0.72 | 65,348 |
| B21326102 | 2.05 | 68,740 |
| RO7122290 | 1.33 | 48,388 |

(continued)

| Antibody name | EC$_{50}$ (nM) | Maximum fluorescence value |
|---|---|---|
| Ab10-A12V2-2 | 1.72 | 77,189 |
| BNT312 | - | 77 |
| IgGl | - | 62 |

Table 19. The results of the binding of the FAP/4-1BB/CD40 multifunctional antibodies to HEK293-Hu4-1BB cells

| Antibody name | EC$_{50}$ (nM) | Maximum fluorescence value |
|---|---|---|
| B977703 | 0.402 | 10,846 |
| B977704 | 0.227 | 10,626 |
| B21326101 | 0.190 | 10,809 |
| B21326102 | 0.548 | 10,621 |
| RO7122290 | 0.573 | 8,437 |
| Ab10-A12V2-2 | - | 73 |
| BNT312 | 2.173 | 25,705 |
| IgGl | - | 63 |

Table 20. The results of the binding of the FAP/4-1BB/CD40 multifunctional antibodies to HEK293-HuCD40 cells

| Antibody name | EC$_{50}$ (nM) | Maximum fluorescence value |
|---|---|---|
| B977703 | 5.29 | 63,346 |
| B977704 | 4.24 | 63,779 |
| B21326101 | 2.55 | 62,345 |
| B21326102 | 12.08 | 64,684 |
| RO7122290 | - | 89 |
| Ab10-A12V2-2 | 4.77 | 53,987 |
| BNT312 | 6.78 | 108,106 |
| IgGl | - | 62 |

5.3. Assay for antigen-binding activity of anti-FAP/4-1BB/CD40 multispecific antibodies

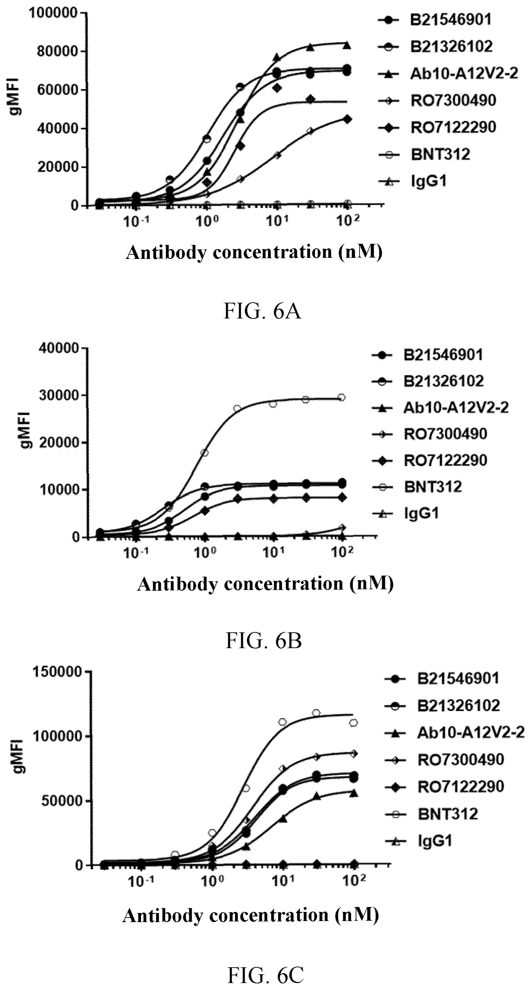

The antigen-binding activity of the FAP/4-1BB/CD40 multispecific antibodies B21546901 and B21326102 was determined using the method provided in Example 5.1, with RO7122290, RO7300490, BNT312, and IgG1 as controls.
The FACS assay results (Tables 21-23 and FIGs. 6A-6C) show that the FAP/4-1BB/CD40 multispecific antibodies exhibited strong binding abilities to FAP on the surface of CHO-K1-HuFAP cells, and the binding abilities were stronger than that of RO7300490 and comparable to that of the control antibody RO7122290. Table 22 shows that the FAP/4-1BB/CD40 multispecific antibodies exhibited good binding abilities to 4-1BB on the surface of HEK293-Hu4-1BB cells, indicating that the antibody aggregation risk reduction engineering in the 4-1BB sequence did not significantly affect binding to 4-1BB. Table 23 shows that the FAP/4-1BB/CD40 multispecific antibodies exhibited good binding abilities to CD40 on the surface of HEK293-HuCD40 cells.

Table 21. The results of the binding of the FAP/4-1BB/CD40 multifunctional antibodies to CHO-K1-HuFAP cells

| Antibody name | EC$_{50}$ (nM) | Maximum fluorescence value |
|---|---|---|
| B21546901 | 1.71 | 68,669 |
| B21326102 | 1.03 | 70,629 |
| Ab10-A12V2-2 | 2.73 | 82,738 |

(continued)

| Antibody name | EC$_{50}$ (nM) | Maximum fluorescence value |
|---|---|---|
| RO7300490 | 8.68 | 44,437 |
| RO7122290 | 2.48 | 44,190 |
| BNT312 | - | 449 |
| IgG1 | - | 64 |

Table 22. The results of the binding of the FAP/4-1BB/CD40 multifunctional antibodies to HEK293-Hu4-1BB cells

| Antibody name | EC$_{50}$ (nM) | Maximum fluorescence value |
|---|---|---|
| B21546901 | 0.50 | 10,839 |
| B21326102 | 0.25 | 11,340 |
| Ab10-A12V2-2 | - | 70 |
| RO7300490 | - | 1,787 |
| RO7122290 | 0.67 | 8,197 |
| BNT312 | 0.77 | 29,314 |
| IgG1 | - | 65 |

Table 23. The results of the binding of the FAP/4-1BB/CD40 multifunctional antibodies to HEK293-HuCD40 cells

| Antibody name | EC$_{50}$ (nM) | Maximum fluorescence value |
|---|---|---|
| B21546901 | 4.17 | 66,548 |
| B21326102 | 3.98 | 69,374 |
| Ab10-A12V2-2 | 6.85 | 55,203 |
| RO7300490 | 3.71 | 85,545 |
| RO7122290 | - | 106 |
| BNT312 | 2.77 | 109,469 |
| IgG1 | - | 62 |

The above results show that: the anti-FAP/4-1BB/CD40 multispecific antibodies exhibited strong binding abilities to FAP on the surface of CHO-K1-HuFAP cells, and the binding abilities were comparable to that of the control antibody RO7122290, indicating that the binding of the antibody to FAP was not affected after the antibody was prepared into the trispecific antibodies. The anti-FAP/4-1BB/CD40 multispecific antibodies exhibited good binding abilities to 4-1BB on the surface of HEK293-Hu4-1BB cells, and B977704 and the B21326102 after engineering did not differ in 4-1BB binding, indicating that the 4-1BB sequence alteration did not affect target binding. The anti-FAP/4-1BB/CD40 multispecific antibodies exhibited binding abilities to CD40 on the surface of HEK293-HuCD40 cells, indicating that the CD40 sequence alteration did not significantly affect target binding.

## Example 6. Luciferase Reporter Gene Assays of FAP/4-1BB/CD40 Multispecific Antibodies

6.1. 4-1BB/NF-κB luciferase reporter gene assay of FAP/4-1BB/CD40 multispecific antibodies

The agonist activity of the antibodies was evaluated using a 4-1BB/NF-κB reporter gene assay. HEK293-Hu4-1BB/NF-κB double-transfected cells were obtained by transiently transfecting HEK293 cells (ATCC CRL-1573) with a gene expressing human 4-1BB (CD137 cDNA ORF Clone, Human, C-OFPSpark tag; Sino Biological, Cat#HG10041-ACR) and the NF-κB genome (pGL4.32[luc2P/NF-κB-RE/Hygro] Vector, Promega, Cat#E849A). Hu4-1BB activation can be characterized by the activation level of the NF-κB signaling pathway. CHO-K1-HuFAP cells were obtained by stably transfecting CHO-K1 cells with a gene expressing human FAP protein. The cell culture medium was Ham's F12 + GlutaMAXTM-I (Gibco, Cat#31765035) containing 10% fetal bovine serum and 800 μg/mL hygromycin B. HEK293-HuCD40 cells were HEK293 cells overexpressing human CD40 protein. The cell culture medium was DMEM (Gibco, Cat#11965092) containing 10% fetal bovine serum and 100 $\mu$g/mL hygromycin B. HEK293-Hu4-1BB/NF-$\kappa$B cells (2 $\times$ 10$^6$/mL) were plated onto a 96-well cell culture plate at 50 $\mu$L, 40 $\mu$L of culture medium, FAP-expressing CHO-K1-HuFAP cells, or CD40-expressing HEK293-HuCD40 cells (2.5 $\times$ 10$^6$ cells/mL) were added, and 10$\times$ serially diluted test antibodies were added at 10 $\mu$L/well. The plate was incubated at 37 °C for 6 h. The cells were taken out. An equal volume of Bio-Glo Luciferase Assay System reagent (Promega, Cat#G7940) was added to each well, and the plate was incubated for 5 min in a dark place. The fluorescence signals were measured using an Envision microplate reader. The EC50 values and the Emax values (relative to the fluorescence intensity of the antibody-free group) were calculated, and the *in vitro* cell agonist activity of the anti-4-1BB antibodies was assessed.

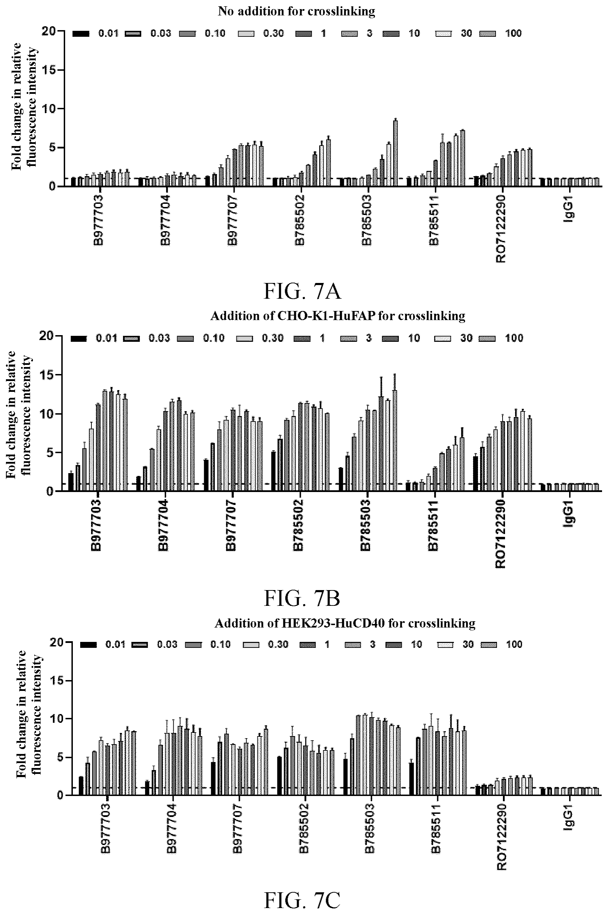

The results (Table 24 and FIGs. 7A-7C) show that the uncrosslinked FAP/4-1BB/CD40 multispecific antibodies B977703 and B977704 exhibited no background activation of 4-1BB, and the other antibodies all exhibited some background activation. The FAP/4-1BB/CD40 multifunctional antibodies all exhibited FAP or CD40 crosslinking-activated 4-1BB activity.

Table 24. FAP/CD40-dependent 4-1BB/NF-$\kappa$B luciferase reporter gene assay results for the FAP/4-1BB/CD40 multispecific antibodies (uncrosslinked, FAP-crosslinked, and CD40-crosslinked)

| Antibody name | Uncrosslinked | FAP-crosslinked | | CD40-crosslinked | |
|---|---|---|---|---|---|
| | Fold change in Emax | EC$_{50}$ (nM) | Fold change in Emax | EC$_{50}$ (nM) | Fold change in Emax |
| B977703 | 1.53 | 0.221 | 12.68 | - | 8.23 |
| B977704 | 1.54 | 0.142 | 10.92 | 0.054 | 8.41 |
| B977707 | 5.28 | 0.033 | 9.74 | - | 7.27 |
| B785502 | 5.97 | 0.042 | 10.86 | 1.063 | 6.51 |
| B785503 | 8.42 | 0.020 | 13.12 | 0.030 | 9.80 |
| B785511 | 7.20 | - | 6.93 | 0.026 | 8.48 |
| RO7122290 | 5.97 | 0.040 | 9.95 | - | 2.30 |
| IgG1 | 1.05 | - | 1.04 | - | 1.06 |

6.2. 4-1BB/NF-$\kappa$B luciferase reporter gene assay of FAP/4-1BB/CD40 multispecific antibodies

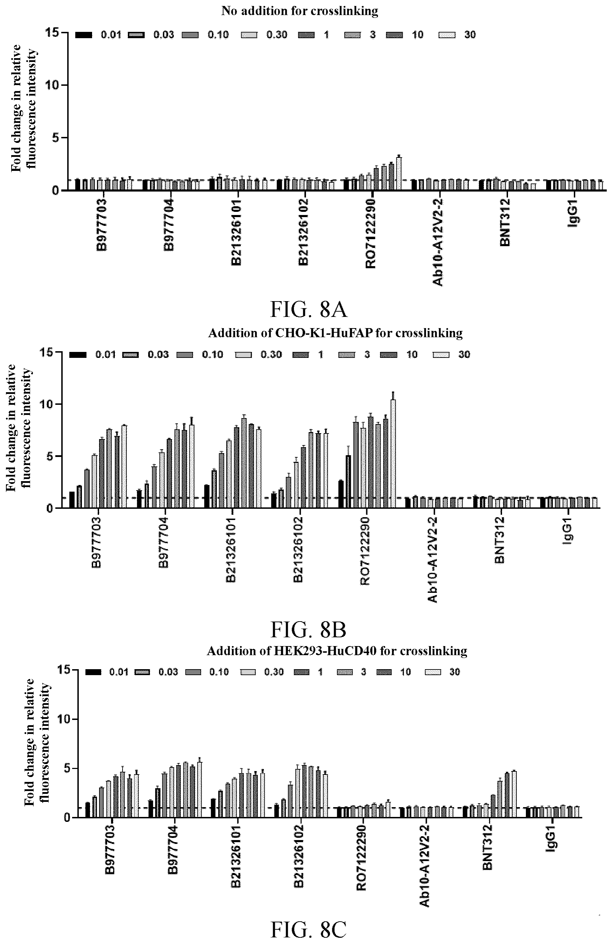

The 4-1BB agonist activity of the FAP/4-1BB/CD40 multispecific antibodies was determined using the method provided in Example 6.1. The results are shown in FIGs. 8A-8C and Table 25.

The results in Table 25 and FIG. 8A show that the uncrosslinked FAP/4-1BB/CD40 multispecific antibodies exhibited no background activation of 4-1BB. The results in Table 25 and FIG. 8B show that after FAP crosslinking, the 4-1BB agonistic activity of the antibodies B977703 and B977704 was comparable to the activation abilities of B21326101 and B21326102, indicating that the 4-1BB sequence engineering did not affect 4-1BB activation activity. The results in Table 25 and FIG. 8C show that the FAP/4-1BB/CD40 multispecific antibodies all exhibited CD40 crosslinking-activated 4-1BB activity, and after being crosslinked with CD40, the FAP/4-1BB/CD40 multispecific antibodies were superior to BNT312 in 4-1BB agonistic activity.

Table 25. FAP/CD40-dependent 4-1BB/NF-$\kappa$B luciferase reporter gene assay results for the FAP/4-1BB/CD40 multifunctional antibodies

| Antibody name | Uncrosslinked | FAP-crosslinked | | CD40-crosslinked | |
|---|---|---|---|---|---|
| | Fold change in Emax | EC$_{50}$ (nM) | Fold change in Emax | EC$_{50}$ (nM) | Fold change in Emax |
| B977703 | 1.14 | 0.173 | 7.71 | 0.070 | 4.37 |
| B977704 | 1.01 | 0.146 | 7.98 | 0.033 | 5.50 |
| B21326101 | 1.05 | 0.071 | 8.14 | 0.035 | 4.53 |
| B21326102 | 1.06 | 0.266 | 7.43 | 0.090 | 4.97 |
| RO7122290 | 3.60 | 0.032 | 9.382 | - | 1.22 |
| Ab10-A12V2-2 | 1.06 | - | 1.05 | - | 1.10 |

(continued)

| Antibody name | Uncrosslinked | FAP-crosslinked | | CD40-crosslinked | |
|---|---|---|---|---|---|
| | Fold change in Emax | EC$_{50}$ (nM) | Fold change in Emax | EC$_{50}$ (nM) | Fold change in Emax |
| BNT312 | 1.09 | 0.293 | 1.19 | 1.651 | 4.76 |
| IgG1 | 1.02 | - | 1.05 | - | 1.18 |

6.3. 4-1BB/NF-κB luciferase reporter gene assay of FAP/4-1BB/CD40 multifunctional antibodies

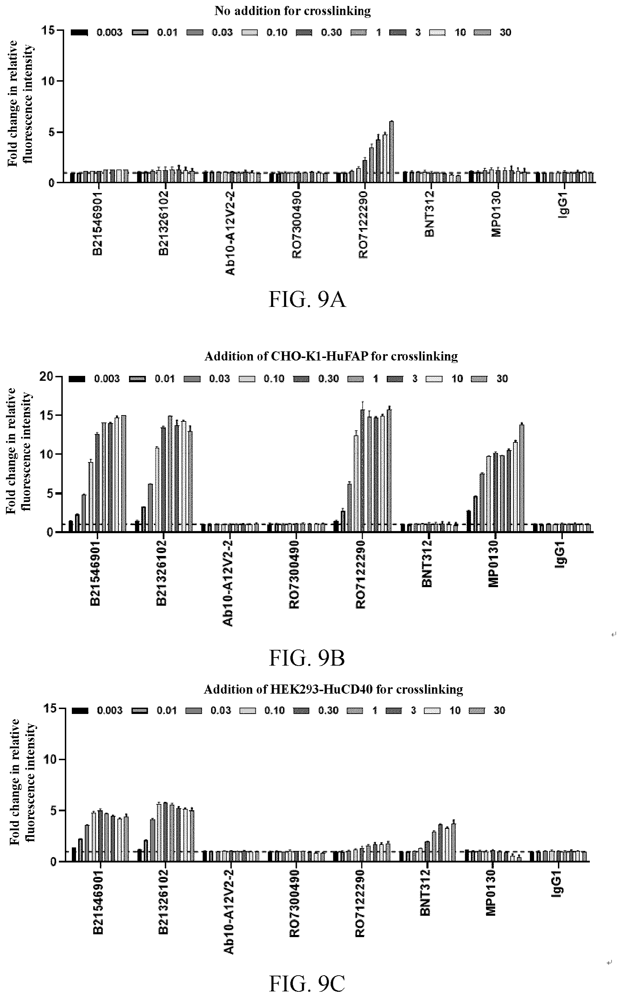

The 4-1BB agonist activity of the FAP/4-1BB/CD40 multispecific antibodies was determined using the method provided in Example 6.1. The results are shown in FIGs. 9A-9C and Table 26.

Table 26 and FIG. 9A show that the uncrosslinked antibodies B21546901 and B21326102 exhibited no background activation activity. Table 26 and FIG. 9B show that after FAP crosslinking, the 4-1BB agonistic activity of the antibodies was comparable to the activation ability of RO7122290. Table 26 and FIG. 9C show that the FAP/4-1BB/CD40 multispecific antibodies all exhibited CD40 crosslinking-activated 4-1BB activity, and after being crosslinked with CD40, the antibodies were superior to BNT312 in 4-1BB agonistic activity.

Table 26. FAP/CD40-dependent 4-1BB/NF-κB luciferase reporter gene assay results for the FAP/4-1BB/CD40 multifunctional antibodies (uncrosslinked, FAP-crosslinked, and CD40-crosslinked)

| Antibody name | Uncrosslinked | FAP-crosslinked | | CD40-crosslinked | |
|---|---|---|---|---|---|
| | Fold change in Emax | EC$_{50}$ (nM) | Fold change in Emax | EC$_{50}$ (nM) | Fold change in Emax |
| B21546901 | 1.34 | 0.07 | 14.98 | 0.02 | 4.61 |
| B21326102 | 1.33 | 0.04 | 14.91 | 0.02 | 5.04 |
| Ab10-A12V2-2 | 1.10 | - | 1.01 | - | 1.07 |
| RO7300490 | 1.01 | - | 1.02 | - | 1.06 |
| RO7122290 | 6.03 | 0.04 | 15.40 | - | 1.77 |
| BNT312 | 1.10 | - | 1.01 | 0.41 | 3.56 |
| MP0310 | 1.01 | 0.02 | 13.85 | - | 1.11 |
| IgG1 | 1.10 | - | 1.08 | - | 1.05 |

The above results show that: the uncrosslinked anti-FAP/4-1BB/CD40 multispecific antibodies B977703, B977704, B21326101, B21326102, and B21546901 exhibited no background activation of 4-1BB; after being crosslinked with FAP, several anti-FAP/4-1BB/CD40 multispecific antibodies did not significantly differ in 4-1BB agonistic activity, indicating that the 4-1BB sequence engineering did not affect 4-1BB activation activity; after being crosslinked with CD40, several anti-FAP/4-1BB/CD40 multispecific antibodies were comparable in 4-1BB agonistic activity, and after being crosslinked with CD40, the antibodies were superior to BNT312 in 4-1BB agonistic activity.

## Example 7. Luciferase Reporter Gene Assays of FAP/4-1BB/CD40 Multispecific Antibodies

7.1. CD40/NF-κB luciferase reporter gene assay of FAP/4-1BB/CD40 multispecific antibodies

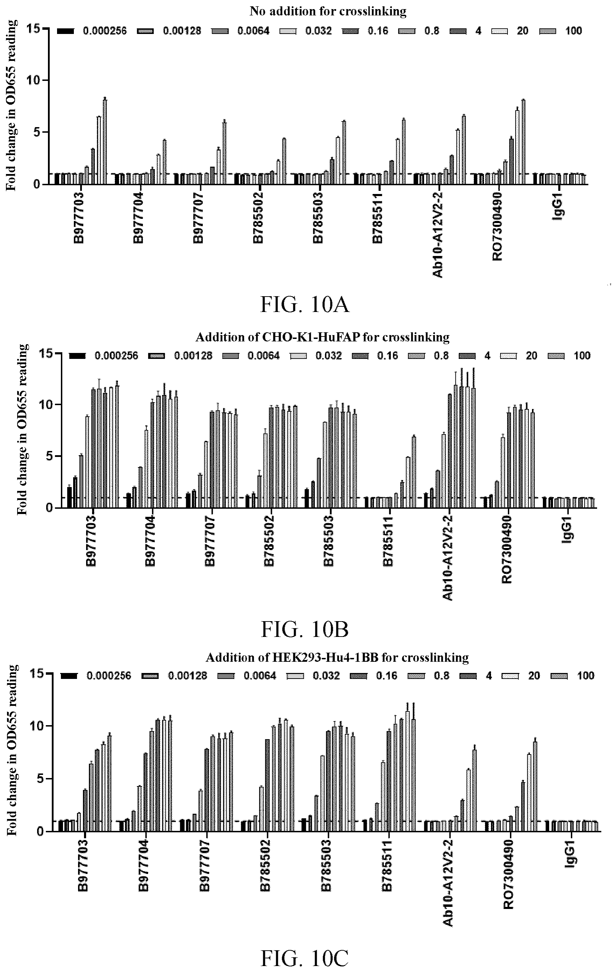

The agonist activity of the antibodies was evaluated using a CD40/NF-κB reporter gene. HEK-Blue™CD40L (InvivoGen, hkb-cd40) cells were cultured in DMEM (Gibco, Cat#11965092) containing 10% heat-inactivated fetal bovine serum 100 μg/mL Normocin™ (InvivoGen, ant-nr-1). HuCD40 activation can be characterized by the activation level of the NF-κB signaling pathway. CHO-K1-HuFAP cells were obtained by stably transfecting CHO-K1 cells with a gene expressing human FAP protein. The cell culture medium was Ham's F12 + GlutaMAXTM-I (Gibco, Cat#31765035) containing 10% fetal bovine serum and 800 μg/mL hygromycin B. HEK293-Hu4-1BB cells were HEK293 cells overexpressing human 4-1BB protein. The cell culture medium was DMEM (Gibco, Cat#11965092) containing 10% fetal bovine serum and 100 μg/mL hygromycin B. HEK-Blue™CD40L cells ($1 \times 10^6$ cells/mL) were plated onto a 96-well cell culture plate at 100 μL, 80 μL of culture medium, FAP-expressing CHO-K1-HuFAP cells, or 4-1BB-expressing HEK293-Hu4-1BB cells ($1.25 \times 10^6$/mL) was added, and 20× serially diluted test antibodies were added at 10 μL/well.The plate was incubated overnight at 37 °C. 20 μL of cells was taken out from each well, and 180 μL of QUANTI-Blue™ (InvivoGen, re-qbs3) was added. The plate was incubated in a dark place at 37 °C for 30 min. The OD655 was measured using an Envision microplate reader. The EC50 values and the Emax values (relative to the reading of the antibody-free group) were calculated, and the *in vitro* cell agonist activity of the anti-CD40 antibodies was assessed. The results are shown in FIGs. 10A-10C and Table 27.

The results in Table 27 and FIG. 10B show that after FAP crosslinking, the CD40 agonistic activity of the FAP/4-1BB/CD40 multispecific antibodies was comparable to the activation abilities of RO7300490 and the Ab10-A12V2-2 molecule. The results in Table 27 and FIG. 10C show that the FAP/4-1BB/CD40 multispecific antibodies all exhibited 4-1BB crosslinking-activated CD40 activity.

Table 27. FAP/4-1BB-dependent CD40/NF-κB luciferase reporter gene assay results for the FAP/4-1BB/CD40 multispecific antibodies (uncrosslinked, FAP-crosslinked, and 4-1BB-crosslinked)

| Antibody name | Uncrosslinked | FAP-crosslinked | | 4-1BB-crosslinked | |
|---|---|---|---|---|---|
| | Fold change in Emax | $EC_{50}$ (nM) | Fold change in Emax | $EC_{50}$ (nM) | Fold change in Emax |
| B977703 | 8.63 | 0.013 | 11.68 | 0.309 | 8.79 |
| B977704 | 4.79 | 0.017 | 10.88 | 0.071 | 10.68 |
| B977707 | 7.78 | 0.019 | 9.32 | 0.049 | 9.06 |
| B785502 | 6.97 | 0.016 | 9.72 | 0.050 | 10.26 |
| B785503 | 6.67 | 0.09 | 9.47 | 0.015 | 9.64 |
| B785511 | 7.26 | - | 7.77 | 0.025 | 10.82 |
| Ab10-A12V2-2 | 7.05 | 0.025 | 11.88 | - | 8.42 |
| RO7300490 | 8.54 | 0.019 | 9.58 | - | 9.07 |
| IgG1 | 1.01 | - | 1.04 | - | 1.03 |

7.2. CD40/NF-κB luciferase reporter gene assay of FAP/4-1BB/CD40 multispecific antibodies

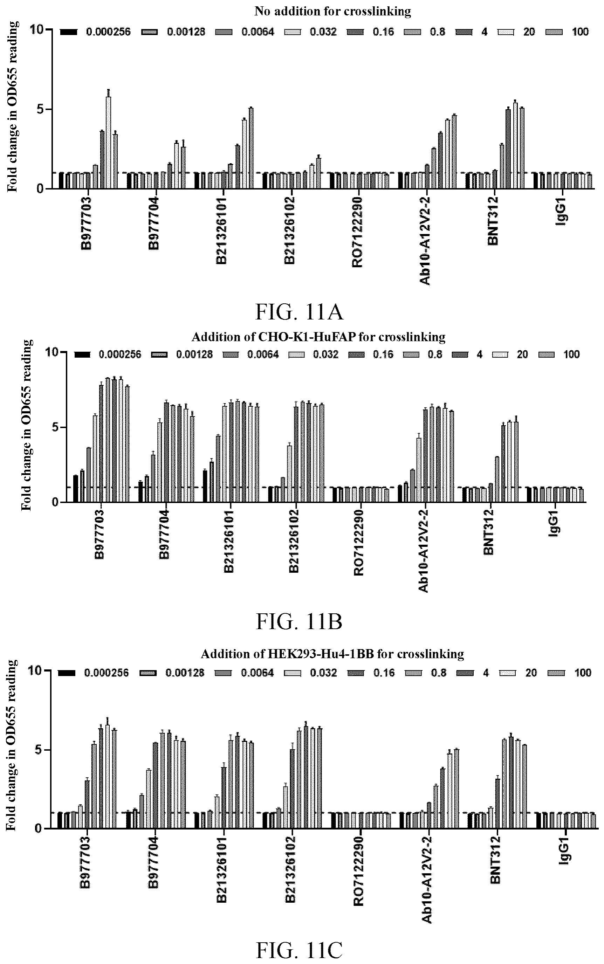

The CD40 agonist activity of the FAP/4-1BB/CD40 multispecific antibodies was determined using the method provided in Example 7.1. The results are shown in FIGs. 11A-11C and Table 28.

The results in Table 28 and FIG. 11B show that after FAP crosslinking, the CD40 agonistic activity of the FAP/4-1BB/CD40 multispecific antibodies was comparable to the activation ability of the Ab10-A12V2-2 molecule; meanwhile, the activation abilities of B977703 and B977704 were comparable to those of B21326101 and B21326102, indicating that the CD40 sequence engineering did not affect CD40 activation activity. The results in Table 28 and FIG. 11C show that the FAP/4-1BB/CD40 multispecific antibodies all exhibited 4-1BB crosslinking-activated activity and were comparable to BNT312 in activation activity.

Table 28. FAP/4-1BB-dependent CD40/NF-κB luciferase reporter gene assay results for the FAP/4-1BB/CD40 multifunctional antibodies

| Antibody name | Uncrosslinked | FAP-crosslinked | | 4-1BB-crosslinked | |
|---|---|---|---|---|---|
| | Fold change in Emax | $EC_{50}$ (nM) | Fold change in Emax | $EC_{50}$ (nM) | Fold change in Emax |
| B977703 | 6.09 | 0.190 | 8.30 | 1.335 | 6.52 |
| B977704 | 2.92 | 0.137 | 6.75 | 0.253 | 5.92 |
| B21326101 | 5.06 | 0.095 | 6.79 | 0.688 | 6.01 |
| B21326102 | 2.05 | 0.301 | 6.89 | 0.499 | 6.38 |
| RO7122290 | 0.98 | - | 1.00 | - | 1.00 |
| Ab10-A12V2-2 | 4.57 | 0.230 | 6.47 | - | 5.00 |
| BNT312 | 5.06 | - | 5.41 | 1.033 | 5.97 |
| IgG1 | 1.00 | - | 0.99 | - | 0.99 |

7.3. CD40/NF-κB luciferase reporter gene assay of FAP/4-1BB/CD40 multispecific antibodies

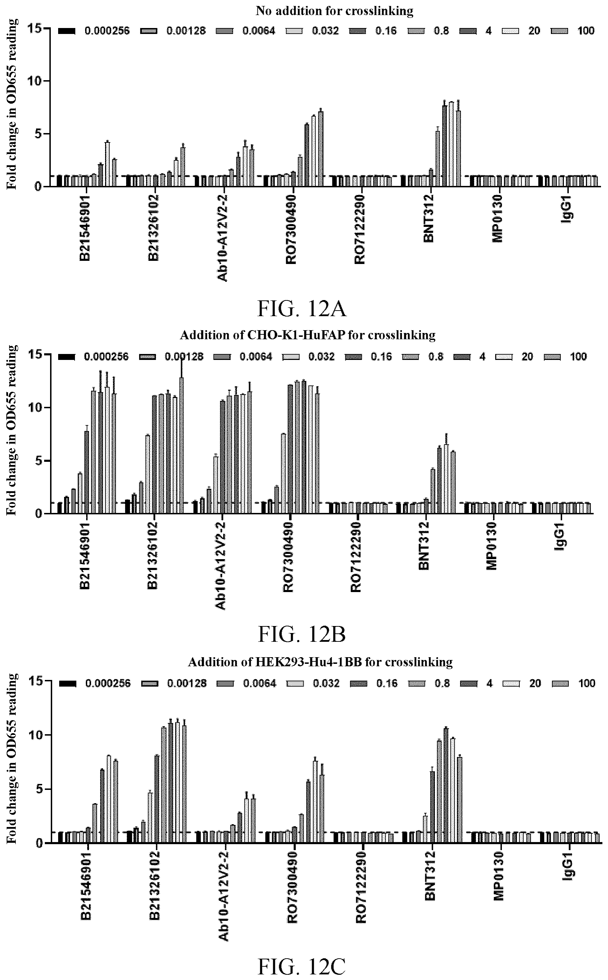

The CD40 agonist activity of the FAP/4-1BB/CD40 multispecific antibodies was determined using the method provided in Example 7.1. The results are shown in FIGs. 12A-12C and Table 29.

The results in Table 29 and FIG. 12B show that in the assay for the CD40 agonistic activity of the related FAP/4-1BB/CD40 multispecific antibodies after FAP crosslinking, their activity was comparable to the activation abilities of RO7300490 and the Ab10-A12V2-2 molecule. The results in Table 29 and FIG. 12C show that the FAP/4-1BB/CD40 multispecific antibodies all exhibited 4-1BB crosslinking-activated activity, with the activity of B21546901 slightly weaker than that of BNT312 and the activity of B21326102 comparable to that of BNT312.

Table 29. FAP/4-1BB-dependent CD40/NF-κB luciferase reporter gene assay results for the FAP/4-1BB/CD40 multifunctional antibodies (uncrosslinked, FAP-crosslinked, and 4-1BB-crosslinked)

| Antibody name | Uncrosslinked | FAP-crosslinked | | 4-1BB-crosslinked | |
|---|---|---|---|---|---|
| | Fold change in Emax | $EC_{50}$ (nM) | Fold change in Emax | $EC_{50}$ (nM) | Fold change in Emax |
| B21546901 | 3.41 | 0.66 | 11.79 | 3.97 | 7.87 |
| B21326102 | 4.06 | 0.25 | 11.66 | 0.50 | 11.19 |
| Ab10-A12V2-2 | 3.73 | 0.35 | 11.34 | - | 4.27 |
| RO7300490 | 7.03 | 0.26 | 12.18 | - | 6.97 |
| RO7122290 | 0.99 | - | 1.00 | - | 1.00 |
| BNT312 | 7.66 | - | 6.27 | 0.69 | 9.51 |
| MP0310 | 1.03 | - | 0.99 | - | 1.01 |
| IgG1 | 0.99 | - | 0.99 | - | 0.99 |

The above results show that: after being crosslinked with FAP, several anti-FAP/4-1BB/CD40 multispecific antibodies did not significantly differ in CD40 agonistic activity, and their activity was comparable to the activation ability of the Ab10-A12V2-2 molecule, indicating that the CD40 sequence engineering did not affect CD40 activation activity; the 4-1BB-crosslinked anti-FAP/4-1BB/CD40 multispecific antibodies all exhibited CD40 activation activity.

**Example 8. T Lymphocyte Activation Assays of FAP/4-1BB/CD40 Multispecific Antibodies**

8.1. T lymphocyte activation assay of FAP/4-1BB/CD40 multispecific antibodies

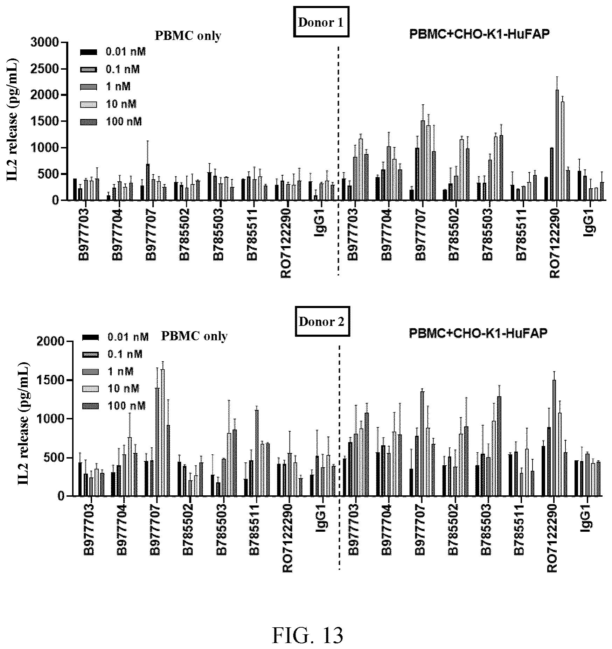

The abilities of the antibodies to stimulate T cells in the presence or absence of FAP were determined using a T lymphocyte activation assay. Freshly isolated and purified PBMCs were resuspended in RPMI1640 (Gibco, Cat#10491A-01) containing 10% fetal bovine serum, and the density was adjusted to $1 \times 10^6$ cells/mL. CHO-K1-HuFAP cells were obtained by stably transfecting CHO-K1 cells with a gene expressing human FAP protein. The cell culture medium was Ham's F12 + GlutaMAXTM-I (Gibco, Cat#31765035) containing 10% fetal bovine serum and 800 μg/mL hygromycin B. After being thawed, the cells were passaged to adjust their state, and the density was adjusted to $1.25 \times 10^6$ cells/mL. A 96-well plate was coated with 0.25 μg/mL anti-CD3 antibody OKT3 (Invitrogen, Cat#16-0037-85) at 100 μL/well, incubated at 37 °C for 2 h, and then washed with PBS to remove the antibody residue. Subsequently, 100 μL of PBMCs ($1 \times 105$ cells/well) and 80 μL of culture medium or CHO-K1-HuFAP cells ($1 \times 10^5$ cells/well) were added to each well of the OKT3-coated 96-well plate, and $10\times$ test samples at different concentrations were added at 20 μL/well. The plate was incubated in an incubator at 37 °C with 5% $CO_2$ for 3 days. The cell culture plate was taken out and centrifuged at 400 g for 5 min, and the cell culture supernatants were collected and assayed for IL-2 levels using a human IL2 assay kit (Cisbio, Cat#62HIL02PEG). See the reagent instructions for specific procedures. The results are shown in FIG. 13.

The results in FIG. 13 show that in the two donors, the related FAP/4-1BB/CD40 multispecific antibody B977707 exhibited a certain background activation activity. After FAP crosslinking, the FAP/4-1BB/CD40 multispecific antibodies all activated IL-2 secretion, and their T lymphocyte activation activity was significantly improved.

8.2. T lymphocyte activation assay of FAP/4-1BB/CD40 multispecific antibodies

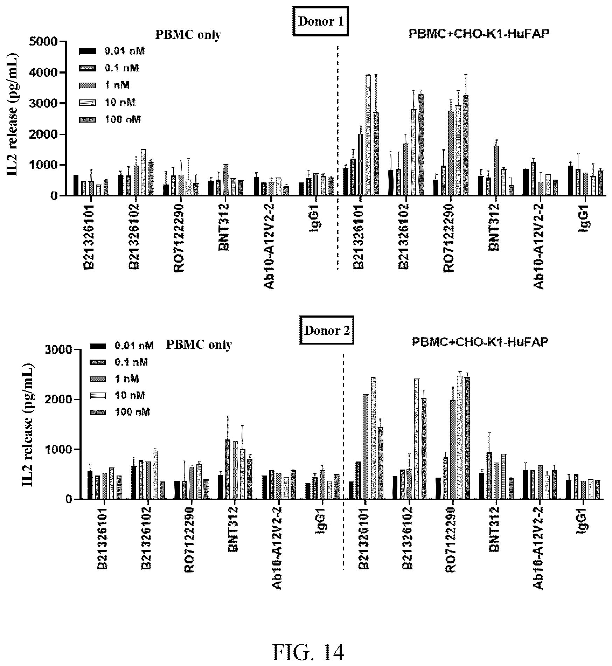

The abilities of the FAP/4-1BB/CD40 multispecific antibodies to stimulate T cells were determined using the experimental method provided in Example 8.1. The results are shown in FIG. 14.

The results in FIG. 14 show that in the two donors, both the FAP/4-1BB/CD40 multispecific antibody B21326102 and the control antibody BNT312 exhibited a certain background T cell activation activity. After FAP crosslinking, the related FAP/4-1BB/CD40 multispecific antibodies all well activated T cells to secrete IL-2, and their abilities to activate T cells were significantly improved.

8.3. T lymphocyte activation assay of FAP/4-1BB/CD40 multispecific antibodies

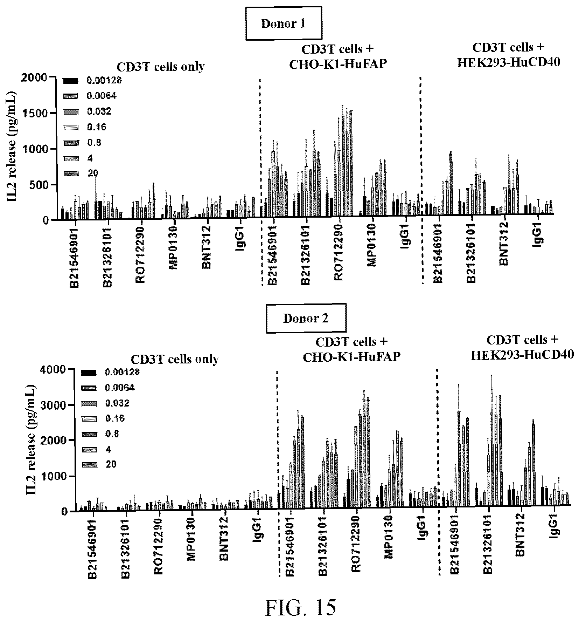

Freshly isolated and purified PBMCs were resuspended in MACS Buffer (Miltenyi Biotec, Cat#130-091-221), and CD3 T cells were isolated using an EasySepTM Human T Cell Isolation Kit (Stemcell, Cat#17951). See the kit instructions for specific procedures. The isolated and purified CD3 T cells were resuspended in RPMI1640 (Gibco, Cat#10491A-01) containing 10% fetal bovine serum, and the density was adjusted to $1 \times 10^6$ cells/mL. CHO-K1-HuFAP cells were obtained by stably transfecting CHO-K1 cells with a gene expressing human FAP protein. The cell culture medium was Ham's F12 + GlutaMAXTM-I (Gibco, Cat#31765035) containing 10% fetal bovine serum and 800 $\mu$g/mL hygromycin B. HEK293-HuCD40 cells were HEK293 cells overexpressing human CD40 protein. The cell culture medium was DMEM (Gibco, Cat#11965092) containing 10% fetal bovine serum and 100 $\mu$g/mL hygromycin B. After being thawed, the cells were passaged to adjust their state, and the density was adjusted to $1.25 \times 10^6$ cells/mL. A 96-well plate was coated with 0.25 $\mu$g/mL anti-CD3 antibody OKT3 (Invitrogen, Cat#16-0037-85) at 100 $\mu$L/well, incubated at 37 °C for 2 h, and then washed with PBS to remove the antibody residue. Subsequently, 100 $\mu$L of PBMCs ($1 \times 10^5$ cells/well) and 80 $\mu$L of culture medium, CHO-K1-FAP cells, or HEK293-HuCD40 ($1 \times 10^5$ cells/well) were added to each well of the OKT3-coated 96-well plate, and $10\times$ test samples at different concentrations were added at 20 $\mu$L/well. The plate was incubated in an incubator at 37 °C with 5% $CO_2$ for 3 days. The cell culture plate was taken out and centrifuged at 400 g for 5 min, and the cell culture supernatants were collected and assayed for IL-2 levels using a human IL-2 assay kit (Cisbio, Cat#62HIL02PEG). See the reagent instructions for specific procedures. The results are shown in FIG. 15.

The results in FIG. 15 show that in the two donors, after FAP crosslinking, the FAP/4-1BB/CD40 multispecific antibodies all activated IL-2 secretion and outperformed MP0310. After CD40 crosslinking, the FAP/4-1BB/CD40 multispecific antibodies all activated IL-2 secretion and outperformed the control antibody BNT312.

**Example 9. DC Maturation Promotion Assays of FAP/4-1BB/CD40 Multispecific Antibodies**

9.1. DC maturation promotion assay of FAP/4-1BB/CD40 multispecific antibodies

The abilities of the antibodies to promote DC maturation in the presence or absence of FAP were verified using a DC maturation promotion experiment. Freshly isolated and purified PBMCs were resuspended in MACS Buffer (Miltenyi Biotec, Cat#130-091-221), and monocytes were isolated using an EasySep™ Human Monocyte Isolation Kit (Stemcell, Cat#19359). See the kit instructions for specific procedures. The isolated monocytes were resuspended in RPMI1640 (Gibco, Cat#10491A-01) containing 10% fetal bovine serum, 50 ng/mL GM-CSF (Peprotech, 200-04), and 50 ng/mL IL4 (Peprotech, 300-03), and the density was adjusted to $1 \times 10^6$ cells/mL. 10 mL of the cell suspension was added to each 100 mm TC-treated Culture Dish (Corning, 430167). After 5 days of culture (the culture medium was changed every 2-3 days), iDCs were obtained. The iDCs were collected centrifugally ($400\times$ g, 10 min) and resuspended in RPMI1640 (Gibco, Cat#10491A-01) containing 10% fetal bovine serum, and the density was adjusted to $1 \times 10^6$ cells/mL. The cell suspension was added at 50 $\mu$L/well. $3\times$ test samples at different concentrations were added at 50 $\mu$L/well. CHO-K1-HuFAP cells were obtained by stably transfecting CHO-K1 cells with a gene expressing human FAP protein. The cell culture medium was Ham's F12 + GlutaMAXTM-I (Gibco, Cat#31765035) containing 10% fetal bovine serum and 800 $\mu$g/mL hygromycin B. CHO-K1-WT cells were cultured in Ham's F12 + GlutaMAXTM-I (Gibco, Cat#31765035) containing 10% fetal bovine serum. CHO-K1-HuFAP and CHO-K1-WT were collected centrifugally ($300\times$ g, 5 min), washed twice with sterile PBS (phosphate-buffered saline, pH 7.40), and resuspended in RPMI1640 (Gibco, Cat#10491A-01), and the density was adjusted to $1 \times 10^6$ cells/mL. 10 $\mu$g/mL Mitomycin C (Sigma, Cat#50-07-7) was added, and the cells were incubated at 37 °C for 45 min. After the incubation, the cells were washed twice with PBS and resuspended in sterile PBS (phosphate-buffered saline, pH 7.40), and the density was adjusted to $2 \times 10^6$ cells/mL. 5 $\mu$M Cell Trace Violet (ThermoFisher, Cat#C345557 A) was added, and the cells were incubated in a dark place at 37 °C for 10 min. After 10 min, an equal volume of RPMI1640 (Gibco, Cat#10491A-01) containing 40% fetal bovine serum was added, and the reaction was stopped in a dark place for 10 min. The cells were washed twice with sterile PBS and resuspended in RPMI1640 (Gibco, Cat#10491A-01) containing 10% fetal bovine serum, and the density was adjusted to $1 \times 10^6$ cells/mL. The cell suspensions were added at 50 $\mu$L/well, with a total volume of 150 $\mu$L. The plate was incubated in an incubator at 37 °C with 5% $CO_2$ for 48 h.

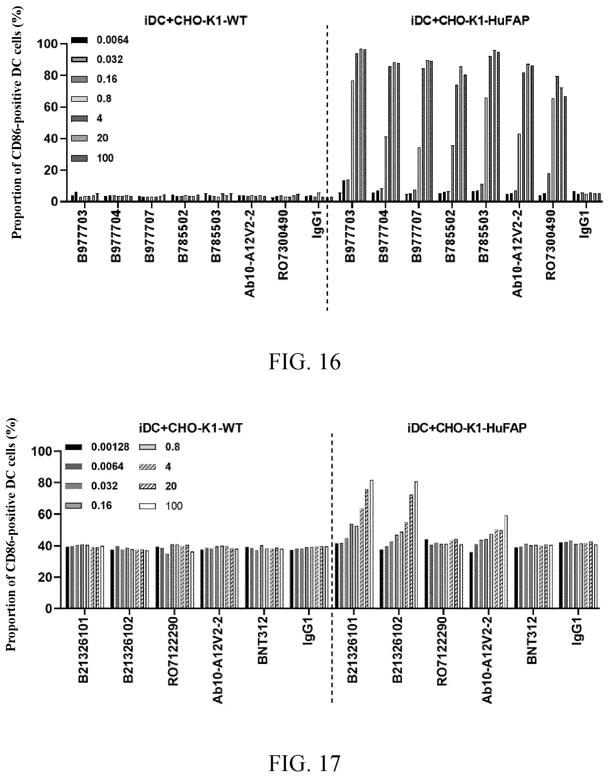

After the 48-hour incubation, the DCs were transferred to a new 96-well U-bottom plate. The plate was washed centrifugally ($300\times$ g, 5 min) twice, and 100 $\mu$L of FACS Buffer (sterile PBS (phosphate-buffered saline, pH 7.40) containing 2% fetal bovine serum) containing 0.1 $\mu$L of live/dead dye APC-efluor780 (BD Pharmingen, Cat#565388) was added to each well. The plate was incubated in a dark place at room temperature for 15 min and washed centrifugally ($300\times$ g, 5 min) twice. 50 $\mu$L of FACS Buffer (sterile PBS (phosphate-buffered saline, pH 7.40) containing 2% fetal bovine serum) containing 1 $\mu$L of Human Trustain FCXTM (Biolegend, Cat#422302) was added to each well, and the plate was incubated in a dark place at room temperature for 10 min. 50 $\mu$L of FACS Buffer (sterile PBS (phosphate-buffered saline, pH 7.40) containing 2% fetal bovine serum) containing 2.5 $\mu$L of PE Mouse Anti-Human CD86 (BD Pharmingen, Cat#555658) was added, and the plate was incubated on ice in a dark place for 30 min. After the incubation, the cells were centrifugally washed twice with FACS Buffer and resuspended in FACS Buffer, and the fluorescence signals were measured using a flow cytometer. The results are shown in FIG. 16.

The results show that none of the FAP/4-1BB/CD40 multispecific antibodies exhibited background DC maturation promotion activity. After FAP crosslinking, the FAP/4-1BB/CD40 multispecific antibodies all promoted DC maturation, and their activity was comparable to that of the Ab10-A12V2-2 molecule and RO7300490.

9.2. DC maturation promotion assay of FAP/4-1BB/CD40 multispecific antibodies

The DC maturation promotion activity of the FAP/4-1BB/CD40 multispecific antibodies was determined using the experimental method provided in Example 8.1. The results are shown in FIG. 17.

The results show that none of the related FAP/4-1BB/CD40 multifunctional antibodies exhibited background DC maturation activation activity. After FAP crosslinking, the FAP/4-1BB/CD40 multifunctional antibodies all promoted DC maturation, and their activity was comparable to that of the Ab10-A12V2-2 molecule.

9.3. DC maturation promotion assay of FAP/4-1BB/CD40 multispecific antibodies

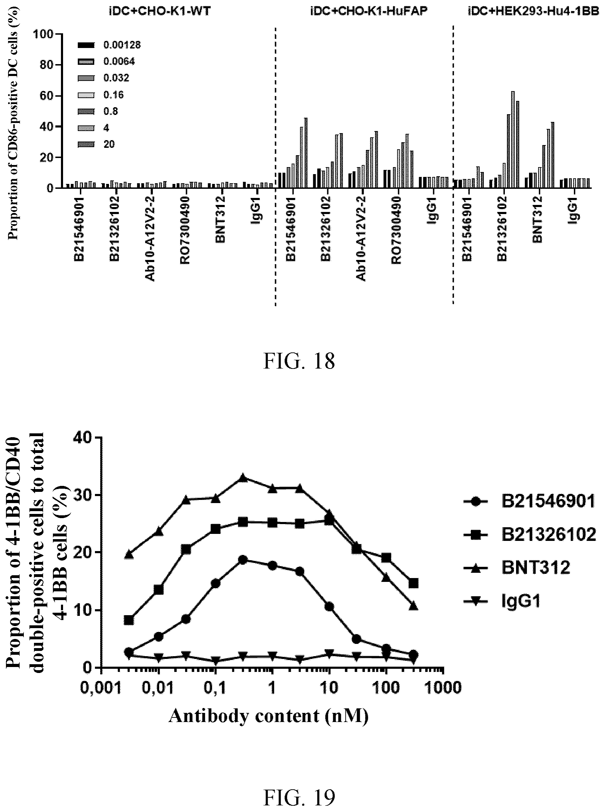

The DC maturation promotion activity of the FAP/4-1BB/CD40 multispecific antibodies was determined using the experimental method provided in Example 8.1. The results are shown in FIG. 18.

The results show that none of the FAP/4-1BB/CD40 multispecific antibodies exhibited background DC maturation promotion activity. After FAP crosslinking, the FAP/4-1BB/CD40 multispecific antibodies all promoted DC maturation and were comparable to the Ab10-A12V2-2 molecule and RO7300490. After 4-1BB crosslinking, the FAP/4-1BB/CD40 multispecific antibodies all promoted DC maturation; B21326102 outperformed the control antibody BNT312, and B21546901 was outperformed by the control antibody BNT312.

## Example 10. Assay for Abilities of FAP/4-1BB/CD40 Multispecific Antibodies to Crosslink with 4-1BB/CD40-Expressing Cells

From the clinical results of BNT312, the crosslinking and activation of CD40-4-1BB in the periphery can cause severe hepatotoxicity. Therefore, our trispecific antibody molecules need strong FAP binding abilities to achieve tumor targeting effects. Meanwhile, they reduce the crosslinking ability between CD40-4-1BB to reduce peripheral activation, thereby reducing hepatotoxicity. The abilities of the FAP/4-1BB/CD40 multifunctional antibodies to crosslink with 4-1BB/CD40-expressing cells were determined by FACS. HEK293-Hu4-1BB cells and HEK293-HuCD40 cells were HEK293 cells overexpressing human 4-1BB protein and HEK293 cells overexpressing human CD40 protein, respectively. The cell culture medium was DMEM (Gibco, Cat#11965092) containing 10% fetal bovine serum and 100 $\mu$g/mL hygromycin B. The experimental culture medium was sterile PBS (phosphate-buffered saline, pH 7.40) containing 2% fetal bovine serum. HEK293-Hu4-1BB and HEK293-HuCD40 cells were collected centrifugally ($300\times$ g, 5 min), washed twice with sterile PBS (phosphate-buffered saline, pH 7.40), and resuspended in sterile PBS (phosphate-buffered saline, pH 7.40), and the density was adjusted to $2 \times 10^6$ cells/mL. 5 $\mu$M Cell Trace Violet (ThermoFisher, Cat#C345557 A) and 5 $\mu$M Cell Trace Far red (ThermoFisher, Cat#C34564 A) were added, and the cells were incubated in a dark place at 37 °C for 10 min. After 10 min, an equal volume of RPMI1640 (Gibco, Cat#10491A-01) containing 40% fetal bovine serum was added, and the reaction was stopped in a dark place for 10 min. The cells were washed twice with sterile PBS and resuspended in FACS Buffer (sterile PBS (phosphate-buffered saline, pH 7.40) containing 2% fetal bovine serum), and the density was adjusted to $2 \times 10^6$ cells/mL. 50 $\mu$L of HEK293-Hu4-1BB cells and 50 $\mu$L of HEK293-HuCD40 cells were added to each well, and $3\times$ test samples at different concentrations were added at 50 $\mu$L/well, with a total volume of 150 $\mu$L. The plate was incubated at 4 °C for 1 h. After the incubation, the fluorescence signals were measured using a flow cytometer. The results are shown in FIG. 19.

The results show that the FAP/4-1BB/CD40 multispecific antibodies all crosslinked with 4-1BB/CD40-expressing cells. The crosslinking abilities of the FAP/4-1BB/CD40 multispecific antibodies were all weaker than that of the control antibody BNT312.

**Example 11. Mixed Lymphocyte Culture Assay of FAP/4-1BB/CD40 Multispecific Antibodies**

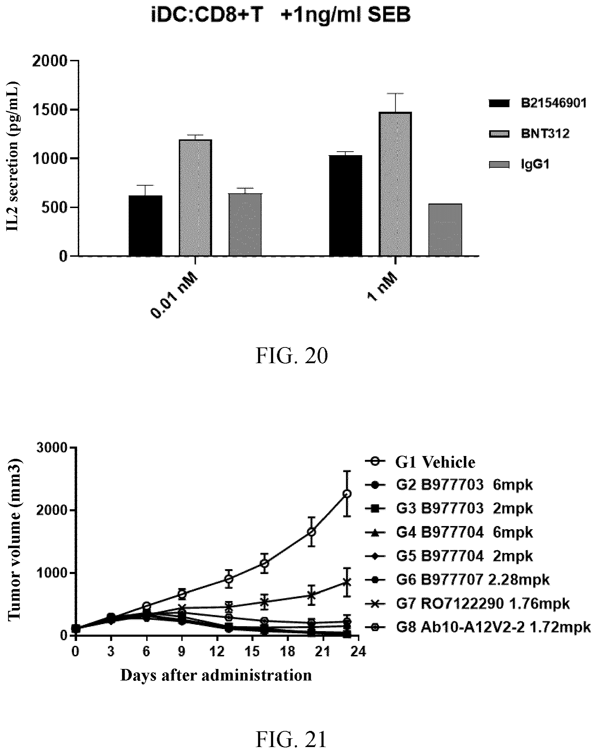

The abilities of the trispecific antibody molecules and the control molecule BNT312 to crosslink with CD40/4-1BB were determined using mixed lymphocyte culture. Cryopreserved PBMCs were resuspended in MACS Buffer (Miltenyi Biotec, Cat#130-091-221), and monocytes were isolated using an EasySep™ Human Monocyte Isolation Kit (Stemcell, Cat#19359). See the kit instructions for specific procedures. The isolated monocytes were resuspended in RPMI1640 (Gibco, Cat#10491A-01) containing 10% fetal bovine serum, 50 ng/mL GM-CSF (Peprotech, 200-04), and 50 ng/mL IL4 (Peprotech, 300-03), and the density was adjusted to $1 \times 10^6$ cells/mL. 10 mL of the cell suspension was added to each 100 mm TC-treated Culture Dish (Corning, 430167). After 5 days of culture at 37 °C (the culture medium was changed every 2-3 days), iDCs were obtained. The iDCs were collected centrifugally ($400\times$ g, 10 min) and resuspended in RPMI1640 (Gibco, Cat#10491A-01) containing 10% fetal bovine serum and 1 ng/mL *Staphylococcus aureus* enterotoxin B (SEB, Toxin Technology, Cat#BT202), and the density was adjusted to $2 \times 10^5$ cells/mL. The cell suspension was added at 50 μL/well. Cryopreserved PBMCs from another donor were resuspended in MACS Buffer (Miltenyi Biotec, Cat#130-091-221), and CD8 T cells were isolated using a human CD8 T cell isolation kit (Miltenyi Biotec, Cat#130-096-495). See the kit instructions for specific procedures. The isolated human CD 8T cells were resuspended in RPMI1640 (Gibco, Cat#10491A-01) containing 10% fetal bovine serum, and the density was adjusted to $1 \times 10^6$ cells/mL. The cell suspension was added at 50 μL/well. $3\times$ test samples at different concentrations were added at 50 μL/well, with a total volume of 150 μL. The plate was incubated at 37 °C for 5 days. The cell culture plate was taken out and centrifuged at 400 g for 5 min, and the cell culture supernatants were collected and assayed for IL-2 levels using a human IL-2 assay kit (Cisbio, Cat#62HIL02PEG). See the reagent instructions for specific procedures. The results are shown in FIG. 20.

The results show that the IL-2 secretion levels induced by the control antibody BNT312 at two concentrations were both higher than those induced by the related FAP/4-1BB/CD40 multispecific antibody.

In conclusion, the experiments of the FAP/4-1BB/CD40 multispecific antibodies crosslinking with 4-1BB/CD40-expressing cells and stimulating IL-2 secretion were non-FAP-dependent molecular characterizations. The result that the FAP/4-1BB/CD40 multispecific antibodies were all weaker than the control antibody BNT312 indicates that in the case that the 4-1BB end and the CD40 end function simultaneously, the background activation of the FAP/4-1BB/CD40 multispecific antibodies in non-FAP dependent cases is low. The FAP/4-1BB/CD40-specific antibodies have a FAP-mediated tumor locating ability. Meanwhile, they can reduce peripheral non-FAP-dependent activation, thereby reducing toxicity.

**Example 12. Study of *In Vivo* Efficacy and Toxicity of Anti-FAP/4-1BB/CD40 Multispecific Antibodies**

*12.1. In vivo* efficacy of FAP/4-1BB/CD40 multispecific antibodies in mouse colon cancer MC38-mFAP model

MC38-mFAP cells (constructed in-house) were subcutaneously inoculated into B-h4-1BB/CD40 humanized mice (provided by Biocytogen) at $2 \times 10^5$ cells/100 μL/mouse. When the tumors grew to about 110 mm$^3$, 48 mice were selected according to tumor volume and randomized into 8 groups of 6, which were the vehicle, B977703 (6 mg/kg), B977703 (2 mg/kg), B977704 (6 mg/kg), B977704 (2 mg/kg), B977707 (2.28 mg/kg), RO7122290 (1.76 mg/kg), and Ab10-A12V2-2 (1.72 mg/kg), respectively. The mice were dosed twice weekly. During the dosing and observation periods, the tumor volume was measured twice weekly, and the measurements were recorded. B977703 (2 mg/kg), B977704 (2 mg/kg), B977707 (2.28 mg/kg), RO7122290 (1.76 mg/kg), and Ab10-A12V2-2 (1.72 mg/kg) were administered at an equimolar dose.

The tumor volume (TV) was calculated using the formula TV = $1/2 \times a \times b_2$, where a and b represent the long and short diameters of the measured tumor, respectively. Relative tumor proliferation rate T/C% = (T - T0) / (C - C0) $\times$ 100%; tumor growth inhibition rate TGI% = 1 - T/C%.

CR% (complete tumor regression proportion) = the number of mice with complete tumor regression (<110 mm$^3$)/the number of mice in the group.

The results are shown in FIG. 21 and Table 30. The results show that at the different doses, the FAP/4-1BB/CD40 multispecific antibodies B977703, B977704, and B977707 all exhibited good antitumor activity, and their antitumor activity was better than that of the control antibodies RO7122290 and Ab10-A12V2-2.

Table 30. The tumor growth inhibition effects of the different FAP/4-1BB/CD40 multifunctional antibodies on mouse graft tumors

| Group | Administration regimen | Dose (mg/kg) | TGI (%) | CR% (<110 mm$^3$) | P value (VS. RO7122290) |
|---|---|---|---|---|---|
| G1 | Vehicle | - | - | - | - |
| G2 | B977703 | 6 | 104.0 | 100% | * |
| G3 | B977703 | 2 | 104.3 | 100% | * |
| G4 | B977704 | 6 | 103.9 | 100% | * |
| G5 | B977704 | 2 | 103.0 | 83.3% | * |
| G6 | B977707 | 2.28 | 98.1 | 66.7% | * |
| G7 | RO7122290 | 1.76 | 65.6 | 0% | - |
| G8 | Ab10-A12V2-2 | 1.72 | 95.0 | 66.6% | - |
| (P values: * was p < 0.05, **$P$ < 0.01, and ***$P$ < 0.001) | | | | | |

12.2. Determination of hepatotoxicity of FAP/4-1BB/CD40 multispecific antibodies in mouse colon cancer MC38-mFAP model

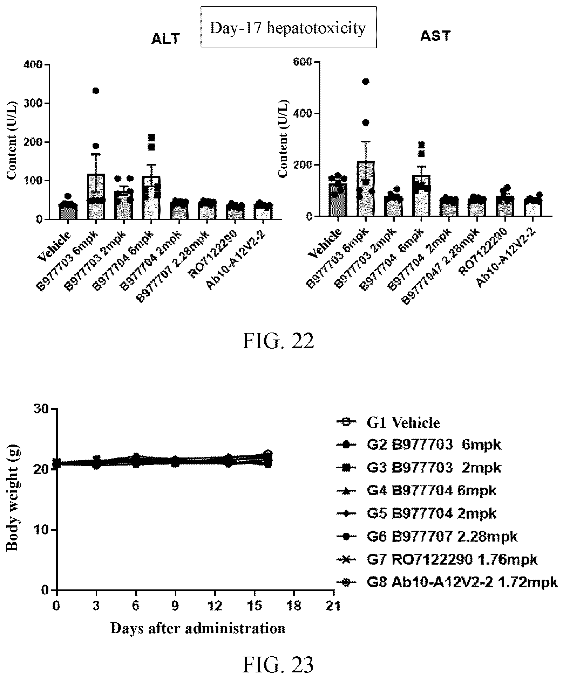

In the 12.1 test, blood was collected on day 17 and assayed for aspartate aminotransferase (AST) and alanine aminotransferase (ALT), and the results are shown in FIG. 22; the body weight was measured twice weekly, and the results are shown in FIG. 23.

The results show that the mouse body weight was stable during the administration period, suggesting that the related FAP/4-1BB/CD40 multispecific antibodies have no significant toxic or side effects (FIG. 23). The day-17 ALT/AST assay results (FIG. 22) show that none of the related FAP/4-1BB/CD40 multispecific antibodies exhibited significant hepatotoxicity.

12.3. Determination of hematotoxicity of FAP/4-1BB/CD40 multifunctional antibodies in mouse colon cancer MC38-mFAP model

In the 12.1 test, blood was collected 24 h after the first administration, and complete blood count analysis was performed.

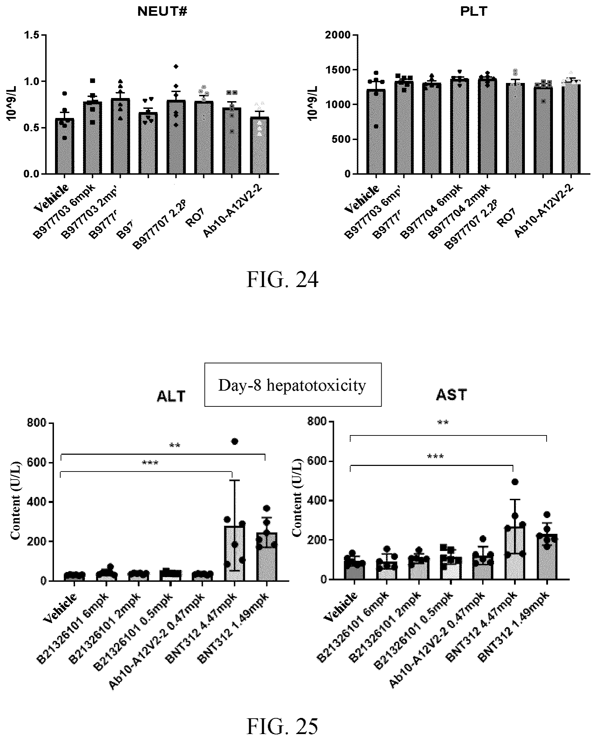

The results (FIG. 24) show that none of the related FAP/4-1BB/CD40 multispecific antibodies caused decreases in neutrophil (NEUT#) or platelet counts (PLT), indicating that they have no hematotoxicity.

12.4. *In vivo* efficacy of FAP/4-1BB/CD40 multispecific antibodies in mouse colon cancer MC38-mFAP model

MC38-mFAP cells (constructed in-house) were subcutaneously inoculated into B-h4-1BB/CD40 humanized mice (provided by Biocytogen) at $2 \times 10^5$ cells/100 µL/mouse. When the tumors grew to about 110 mm$^3$, 42 mice were selected according to tumor volume and randomized into 7 groups of 6, which were the vehicle, B21326101 (6 mg/kg), B21326101 (2 mg/kg), B21326101 (0.5 mg/kg), Ab10-A12V2-2 (0.43 mg/kg), BNT312 (4.47 mg/kg), and BNT312 (1.49 mg/kg), respectively. B21326101 (6 mg/kg), B21326101 (2 mg/kg), BNT312 (4.47 mg/kg), and BNT312 (1.49 mg/kg) were administered once every 3 days, and three administrations were performed. B21326101 (6 mg/kg) and BNT312 (4.47 mg/kg) were administered at an equimolar dose, B21326101 (2 mg/kg) and NT312 (1.49 mg/kg) were administered at an equimolar dose, and B21326101 (0.5 mg/kg) and Ab10-A12V2-2 (0.43 mg/kg) were administered at an equimolar dose. B21326101 (0.5 mg/kg) and Ab10-A12V2-2 (0.43 mg/kg) were administered once every 3 days, and four administrations were performed. During the dosing and observation periods, the tumor volume was measured twice weekly, and the measurements were recorded. At 46 days after administration, MC38-mFAP cells ($2 \times 10^5$ cells/100 µL/mouse) were subcutaneously inoculated on the opposite side of the CR mice and into Naive B-h4-1BB/CD40 humanized mice. The tumor volume of the mice continued to be measured, and the measurements were recorded. The calculation methods of the tumor volume (TV), the relative tumor proliferation rate T/C%, the tumor growth inhibition rate TGI%, and CR% (complete tumor regression proportion) are shown in 12.1.

The results are shown in Table 31. The results show that at the different doses, the related FAP/4-1BB/CD40 multispecific antibody B21326101 exhibited good antitumor activity, and its antitumor activity was better than that of the control antibodies BNT312 and Ab10-A12V2-2.

Table 31. The tumor growth inhibition effects of the different FAP/4-1BB/CD40 multifunctional antibodies on mouse graft tumors

| Group | Administration regimen | Dose (mg/kg) | TGI (%) | CR% (<110 mm$^3$) |
|---|---|---|---|---|
| G1 | Vehicle | - | - | - |
| G2 | B21326101 | 6 | 103.7 | 100% |
| G3 | B21326101 | 2 | 100.2 | 83.3% |
| G4 | B21326101 | 0.5 | 68.7 | 50% |
| G5 | Ab10-A12V2-2 | 0.43 | 26.7 | 0% |
| G6 | BNT312 | 1.49 | 44.2 | 0% |

12.5. Determination of hepatotoxicity of FAP/4-1BB/CD40 multispecific antibodies in mouse colon cancer MC38-mFAP model

In the 12.4 test, blood was collected on day 8 and assayed for AST and ALT.

The results are shown in FIG. 25. The day-8 ALT/AST assay results show that none of the related FAP/4-1BB/CD40 multifunctional antibodies exhibited hepatotoxicity, suggesting relatively good safety. BNT312 exhibited significant hepatotoxicity at both high and low doses.

Table 32. The amino acid sequences of the anti-FAP/4-1BB/CD40 trispecific antibodies

| No. | | Amino acid sequence | SEQ ID NO: |
|---|---|---|---|
| B977703 | Heavy chain | QVQLQQSGVEVKKPGASVTVSCRASGYSFADHFIHWVRQAP GQGFQWMGWINPNRGVTHHAQDFQGRVAMTRDMSTDTVY MELTSLRSDDTAVYYCARDASLTARPYYFYGFDVWGQGTLV TVSS ASTKGPSVFPLAPSSKSTSGGTAALGCLVKDYFPEPVTVSWN SGALTSGVHTFPAVLQSSGLYSLSSVVTVPSSSLGTQTYICNV NHKPSNTKVDKKVEPKSCDKTHTCPPCPAPEAAGGPSVFLFP PKPKDTLMISRTPEVTCVVVDVSHEDPEVKFNWYVDGVEVH NAKTKPREEQYNSTYRVVSVLTVLHQDWLNGKEYKCKVSN KALPAPIEKTISKAKGQPREPQVYTLPPSRDELTKNQVSLTCL VKGFYPSDIAVEWESNGQPENNYKTTPPVLDSDGSFFLYSKLT VDKSRWQQGNVFSCSVMHEALHNHYTQKSLSLSPGK GGGGSGGGGS QVQLVESGGGLVQPGGSLRLSCSASGFTFSSYAMSWVRQAPG KGLEWVSDINSGGESTFYEDSVKGRFTISRDNAKNTLYLQMN SLRAEDTAVYYCAKHPLTYTIATMNDYDYWGQGTLVTVSS GGGGSGGGGS EVQLVESGGGLVQPGGSLRLSCAASGFTFSRYGMKWVRQAP GKGLEWVSTINSHGDTTYYADSVKGRFTISRDNAKNSLYLQ MNSLRAEDTAVYYCLRIDYSDYSSRGQGTLVTVSS | 34 |

(continued)

| No. | | Amino acid sequence | SEQ ID NO: |
|---|---|---|---|
| | Light chain | DIQMTQSPSSVSASVGDRVTITCRASQGISSWLAWYQQKPGK APKLLIYAASSLQSGVPSRFSGSGSGTDFTLTISSLQPEDFATY YCQQANSFPPAFGQGTKVEIK RTVAAPSVFIFPPSDEQLKSGTASVVCLLNNFYPREAKVQWK VDNALQSGNSQESVTEQDSKDSTYSLSSTLTLSKADYEKHKV YACEVTHQGLSSPVTKSFNRGEC | 35 |
| B977704 | Heavy chain | QVQLQQSGVEVKKPGASVTVSCRASGYSFADHFIHWVRQAP GQGFQWMGWINPNRGVTHHAQDFQGRVAMTRDMSTDTVY MELTSLRSDDTAVYYCARDASLTARPYYFYGFDVWGQGTLV TVSS ASTKGPSVFPLAPSSKSTSGGTAALGCLVKDYFPEPVTVSWN SGALTSGVHTFPAVLQSSGLYSLSSVVTVPSSSLGTQTYICNV NHKPSNTKVDKKVEPKSCDKTHTCPPCPAPEAAGGPSVFLFP PKPKDTLMISRTPEVTCVVVDVSHEDPEVKFNWYVDGVEVH NAKTKPREEQYNSTYRVVSVLTVLHQDWLNGKEYKCKVSN KALPAPIEKTISKAKGQPREPQVYTLPPSRDELTKNQVSLTCL VKGFYPSDIAVEWESNGQPENNYKTTPPVLDSDGSFFLYSKLT VDKSRWQQGNVFSCSVMHEALHNHYTQKSLSLSPGK GGGGSGGGGS QVQLVESGGGLVQPGGSLRLSCSASGFTFSSYAMSWVRQAPG KGLEWVSDINSGGESTFYEDSVKGRFTISRDNAKNTLYLQMN SLRAEDTAVYYCAKHPLTYTIATMNDYDYWGQGTLVTVSS | 36 |
| | Light chain | DIQMTQSPSSVSASVGDRVTITCRASQGISSWLAWYQQKPGK APKLLIYAASSLQSGVPSRFSGSGSGTDFTLTISSLQPEDFATY YCQQANSFPPAFGQGTKVEIK RTVAAPSVFIFPPSDEQLKSGTASVVCLLNNFYPREAKVQWK VDNALQSGNSQESVTEQDSKDSTYSLSSTLTLSKADYEKHKV YACEVTHQGLSSPVTKSFNRGEC GGGGSGGGGS EVQLVESGGGLVQPGGSLRLSCAASGFTFSRYGMKWVRQAP GKGLEWVSTINSHGDTTYYADSVKGRFTISRDNAKNSLYLQ MNSLRAEDTAVYYCLRIDYSDYSSRGQGTLVTVSS | 37 |

(continued)

| No. | | Amino acid sequence | SEQ ID NO: |
|---|---|---|---|
| B977707 | Heavy chain | QVQLQQSGVEVKKPGASVTVSCRASGYSFADHFIHWVRQAP GQGFQWMGWINPNRGVTHHAQDFQGRVAMTRDMSTDTVY MELTSLRSDDTAVYYCARDASLTARPYYFYGFDVWGQGTLV TVSS ASTKGPSVFPLAPSSKSTSGGTAALGCLVKDYFPEPVTVSWN SGALTSGVHTFPAVLQSSGLYSLSSVVTVPSSSLGTQTYICNV NHKPSNTKVDKKVEPKSCDKTHTCPPCPAPE<u>AA</u>GGPSVFLFP PKPKDTLMISRTPEVTCVVVDVSHEDPEVKFNWYVDGVEVH NAKTKPREEQYNSTYRVVSVLTVLHQDWLNGKEYKCKVSN KALPAPIEKTISKAKGQPREPQVYTLPPSRDELTKNQVSLTCL VKGFYPSDIAVEWESNGQPENNYKTTPPVLDSDGSFFLYSKLT VDKSRWQQGNVFSCSVMHEALHNHYTQKSLSLSPGK GGGGSGGGGS QVQLVESGGGLVQPGGSLRLSCSASGFTFSSYAMSWVRQAPG KGLEWVSDINSGGESTFYEDSVKGRFTISRDNAKNTLYLQMN SLRAEDTAVYYCAKHPLTYTIATMNDYDYWGQGTLVTVSS GGGGSGGGGS QVQLVESGGGLVQPGGSLRLSCSASGFTFSSYAMSWVRQAPG KGLEWVSDINSGGESTFYEDSVKGRFTISRDNAKNTLYLQMN SLRAEDTAVYYCAKHPLTYTIATMNDYDYWGQGTLVTVSS | 38 |
| | Light chain | DIQMTQSPSSVSASVGDRVTITCRASQGISSWLAWYQQKPGK APKLLIYAASSLQSGVPSRFSGSGSGTDFTLTISSLQPEDFATY YCQQANSFPPAFGQGTKVEIK RTVAAPSVFIFPPSDEQLKSGTASVVCLLNNFYPREAKVQWK VDNALQSGNSQESVTEQDSKDSTYSLSSTLTLSKADYEKHKV YACEVTHQGLSSPVTKSFNRGEC GGGGSGGGGS EVQLVESGGGLVQPGGSLRLSCAASGFTFSRYGMKWVRQAP GKGLEWVSTINSHGDTTYYADSVKGRFTISRDNAKNSLYLQ MNSLRAEDTAVYYCLRIDYSDYSSRGQGTLVTVSS | 39 |
| B785502 | Heavy chain | QVQLQQSGVEVKKPGASVTVSCRASGYSFADHFIHWVRQAP GQGFQWMGWINPNRGVTHHAQDFQGRVAMTRDMSTDTVY | 40 |

(continued)

| No. | | Amino acid sequence | SEQ ID NO: |
|---|---|---|---|
| | | MELTSLRSDDTAVYYCARDASLTARPYYFYGFDVWGQGTLV TVSS ASTKGPSVFPLAPSSKSTSGGTAALGCLVKDYFPEPVTVSWN SGALTSGVHTFPAVLQSSGLYSLSSVVTVPSSSLGTQTYICNV NHKPSNTKVDKKVEPKSCDKTHTCPPCPAPEAAGGPSVFLFP PKPKDTLMISRTPEVTCVVVDVSHEDPEVKFNWYVDGVEVH NAKTKPREEQYNSTYRVVSVLTVLHQDWLNGKEYKCKVSN KALPAPIEKTISKAKGQPREPQVYTLPPSRDELTKNQVSLTCL VKGFYPSDIAVEWESNGQPENNYKTTPPVLDSDGSFFLYSKLT VDKSRWQQGNVFSCSVMHEALHNHYTQKSLSLSPGK GGGGSGGGGS EVQLVESGGGLVQPGGSLRLSCAASGFTFSRYGMKWVRQAP GKGLEWVSTINSHGDTTYYADSVKGRFTISRDNAKNSLYLQ MNSLRAEDTAVYYCLRIDYSDYSSRGQGTLVTVSS GGGGSGGGGS QVQLVESGGGLVQPGGSLRLSCSASGFTFSSYAMSWVRQAPG KGLEWVSDINSGGESTFYEDSVKGRFTISRDNAKNTLYLQMN SLRAEDTAVYYCAKHPLTFTIATMNDYDYWGQGTLVTVSS | |
| | Light chain | DIQMTQSPSSVSASVGDRVTITCRASQGISSWLAWYQQKPGK APKLLIYAASSLQSGVPSRFSGSGSGTDFTLTISSLQPEDFATY YCQQANSFPPAFGQGTKVEIK RTVAAPSVFIFPPSDEQLKSGTASVVCLLNNFYPREAKVQWK VDNALQSGNSQESVTEQDSKDSTYSLSSTLTLSKADYEKHKV YACEVTHQGLSSPVTKSFNRGEC | 41 |
| B785503 | Heavy chain | QVQLQQSGVEVKKPGASVTVSCRASGYSFADHFIHWVRQAP GQGFQWMGWINPNRGVTHHAQDFQGRVAMTRDMSTDTVY MELTSLRSDDTAVYYCARDASLTARPYYFYGFDVWGQGTLV TVSS ASTKGPSVFPLAPSSKSTSGGTAALGCLVKDYFPEPVTVSWN SGALTSGVHTFPAVLQSSGLYSLSSVVTVPSSSLGTQTYICNV NHKPSNTKVDKKVEPKSCDKTHTCPPCPAPEAAGGPSVFLFP PKPKDTLMISRTPEVTCVVVDVSHEDPEVKFNWYVDGVEVH NAKTKPREEQYNSTYRVVSVLTVLHQDWLNGKEYKCKVSN KALPAPIEKTISKAKGQPREPQVYTLPPSRDELTKNQVSLTCL VKGFYPSDIAVEWESNGQPENNYKTTPPVLDSDGSFFLYSKLT VDKSRWQQGNVFSCSVMHEALHNHYTQKSLSLSPGK GGGGSGGGGS EVQLVESGGGLVQPGGSLRLSCAASGFTFSRYGMKWVRQAP GKGLEWVSTINSHGDTTYYADSVKGRFTISRDNAKNSLYLQ MNSLRAEDTAVYYCLRIDYSDYSSRGQGTLVTVSS | 42 |

(continued)

| No. | | Amino acid sequence | SEQ ID NO: |
|---|---|---|---|
| | Light chain | DIQMTQSPSSVSASVGDRVTITCRASQGISSWLAWYQQKPGK APKLLIYAASSLQSGVPSRFSGSGSGTDFTLTISSLQPEDFATY YCQQANSFPPAFGQGTKVEIK RTVAAPSVFIFPPSDEQLKSGTASVVCLLNNFYPREAKVQWK VDNALQSGNSQESVTEQDSKDSTYSLSSTLTLSKADYEKHKV YACEVTHQGLSSPVTKSFNRGEC GGGGSGGGGS QVQLVESGGGLVQPGGSLRLSCSASGFTFSSYAMSWVRQAPG KGLEWVSDINSGGESTFYEDSVKGRFTISRDNAKNTLYLQMN SLRAEDTAVYYCAKHPLTFTIATMNDYDYWGQGTLVTVSS | 43 |
| B785511 | Heavy chain | QVQLVESGGGLVQPGGSLRLSCSASGFTFSSYAMSWVRQAPG KGLEWVSDINSGGESTFYEDSVKGRFTISRDNAKNTLYLQMN SLRAEDTAVYYCAKHPLTFTIATMNDYDYWGQGTLVTVSS EPKSADKTHTCPPCPAPE<u>AA</u>GGPSVFLFPPKPKDTLMISRTPE VTCVVVDVSHEDPEVKFNWYVDGVEVHNAKTKPREEQYNS TYRVVSVLTVLHQDWLNGKEYKCKVSNKALPAPIEKTISKAK GQPREPQVYTLPPSRDELTKNQVSLTCLVKGFYPSDIAVEWES NGQPENNYKTTPPVLDSDGSFFLYSKLTVDKSRWQQGNVFSC SVMHEALHNHYTQKSLSLSPGK GGGGSGGGGS EVQLVESGGGLVQPGGSLRLSCAASGFTFSRYGMKWVRQAP GKGLEWVSTINSHGDTTYYADSVKGRFTISRDNAKNSLYLQ MNSLRAEDTAVYYCLRIDYSDYSSRGQGTLVTVSS | 44 |

(continued)

| No. | | Amino acid sequence | SEQ ID NO: |
|---|---|---|---|
| B21326101 | Heavy chain | QVQLQQSGVEVKKPGASVTVSCRASGYSFADHFIHWVRQAP GQGFQWMGWINPNRGVTHHAQDFQGRVAMTRDMSTDTVY MELTSLRSDDTAVYYCARDASLTARPYYFYGFDVWGQGTLV TVSS ASTKGPSVFPLAPSSKSTSGGTAALGCLVKDYFPEPVTVSWN SGALTSGVHTFPAVLQSSGLYSLSSVVTVPSSSLGTQTYICNV NHKPSNTKVDKKVEPKSCDKTHTCPPCPAPEAAGGPSVFLFP PKPKDTLMISRTPEVTCVVVDVSHEDPEVKFNWYVDGVEVH NAKTKPREEQYNSTYRVVSVLTVLHQDWLNGKEYKCKVSN KALPAPIEKTISKAKGQPREPQVYTLPPSRDELTKNQVSLTCL VKGFYPSDIAVEWESNGQPENNYKTTPPVLDSDGSFFLYSKLT VDKSRWQQGNVFSCSVMHEALHNHYTQKSLSLSPGK GGGGSGGGGS QVQLVESGGGLVQPGGSLRLSCSASGFTFSSYAMSWVRQAPG KGLEWVSDINSGGESTFYEDSVKGRFTISRDNAKNTLYLQMN SLRAEDTAVYYCAKHPLTYTIATMNDYDYWGQGTLVTVSS GGGGSGGGGS EVQLVESGGGLVQPGGSLRLSCAASGFTFSRYGMKWVRQAP GKGLEWVSTINSHGDTTYYADSVKGRFTISRDNAKNSLYLQ MNSLRAEDTAVYYCLRIDYSDYSSRGQGTLVTVS<u>AA</u> | 45 |
| | Light chain | DIQMTQSPSSVSASVGDRVTITCRASQGISSWLAWYQQKPGK APKLLIYAASSLQSGVPSRFSGSGSGTDFTLTISSLQPEDFATY YCQQANSFPPAFGQGTKVEIK RTVAAPSVFIFPPSDEQLKSGTASVVCLLNNFYPREAKVQWK VDNALQSGNSQESVTEQDSKDSTYSLSSTLTLSKADYEKHKV YACEVTHQGLSSPVTKSFNRGEC | 46 |
| B21326102 | Heavy chain | QVQLQQSGVEVKKPGASVTVSCRASGYSFADHFIHWVRQAP GQGFQWMGWINPNRGVTHHAQDFQGRVAMTRDMSTDTVY MELTSLRSDDTAVYYCARDASLTARPYYFYGFDVWGQGTLV TVSS ASTKGPSVFPLAPSSKSTSGGTAALGCLVKDYFPEPVTVSWN SGALTSGVHTFPAVLQSSGLYSLSSVVTVPSSSLGTQTYICNV NHKPSNTKVDKKVEPKSCDKTHTCPPCPAPEAAGGPSVFLFP PKPKDTLMISRTPEVTCVVVDVSHEDPEVKFNWYVDGVEVH NAKTKPREEQYNSTYRVVSVLTVLHQDWLNGKEYKCKVSN KALPAPIEKTISKAKGQPREPQVYTLPPSRDELTKNQVSLTCL VKGFYPSDIAVEWESNGQPENNYKTTPPVLDSDGSFFLYSKLT VDKSRWQQGNVFSCSVMHEALHNHYTQKSLSLSPGK GGGGSGGGGS QVQLVESGGGLVQPGGSLRLSCSASGFTFSSYAMSWVRQAPG KGLEWVSDINSGGESTFYEDSVKGRFTISRDNAKNTLYLQMN SLRAEDTAVYYCAKHPLTYTIATMNDYDYWGQGTLVTVS<u>AA</u> | 47 |

(continued)

| No. | | Amino acid sequence | SEQ ID NO: |
|---|---|---|---|
| | Light chain | DIQMTQSPSSVSASVGDRVTITCRASQGISSWLAWYQQKPGK APKLLIYAASSLQSGVPSRFSGSGSGTDFTLTISSLQPEDFATY YCQQANSFPPAFGQGTKVEIK RTVAAPSVFIFPPSDEQLKSGTASVVCLLNNFYPREAKVQWK VDNALQSGNSQESVTEQDSKDSTYSLSSTLTLSKADYEKHKV YACEVTHQGLSSPVTKSFNRGEC GGGGSGGGGS EVQLVESGGGLVQPGGSLRLSCAASGFTFSRYGMKWVRQAP GKGLEWVSTINSHGDTTYYADSVKGRFTISRDNAKNSLYLQ MNSLRAEDTAVYYCLRIDYSDYSSRGQGTLVTVS<u>AA</u> | 48 |
| B21546901 | Heavy chain | QVQLQQSGVEVKKPGASVTVSCRASGYSFADHFIHWVRQAP GQGFQWMGWINPNRGVTHHAQDFQGRVAMTRDMSTDTVY MELTSLRSDDTAVYYCARDASLTARPYYFYGFDVWGQGTLV TVSS ASTKGPSVFPLAPSSKSTSGGTAALGCLVKDYFPEPVTVSWN SGALTSGVHTFPAVLQSSGLYSLSSVVTVPSSSLGTQTYICNV NHKPSNTKVDKKVEPKSCDKTHTCPPCPAPEAAGGPSVFLFP PKPKDTLMISRTPEVTCVVVDVSHEDPEVKFNWYVDGVEVH NAKTKPREEQYNSTYRVVSVLTVLHQDWLNGKEYKCKVSN KALPAPIEKTISKAKGQPREPQVYTLPPSRDELTKNQVSLTCL VKGFYPSDIAVEWESNGQPENNYKTTPPVLDSDGSFFLYSKLT VDKSRWQQGNVFSCSVMHEALHNHYTQKSLSLSPGK GGGGSGGGGS QVQLVESGGGLVQPGGSLRLSCSASGFTFSSYAMSWVRQAPG KGLEWVSDINSGGQSTFYADSVKGRFTISRDNAKNTLYLQM NSLRAEDTAVYYCAKHPLTYTIATMNDYDYWGQGTQVTVSS GGGGSGGGGS EVQLVESGGGLVQPGGSLRLSCAASGFTFSRYGMKWVRQAP GKGLEWVSTINSHGDTTYYADSVKGRFTISRDNAKNSLYLQ MNSLRAEDTAVYYCLRIDYSDYSSRGQGTQVTVS<u>AA</u> | 49 |
| | Light chain | DIQMTQSPSSVSASVGDRVTITCRASQGISSWLAWYQQKPGK APKLLIYAASSLQSGVPSRFSGSGSGTDFTLTISSLQPEDFATY YCQQANSFPPAFGQGTKVEIK RTVAAPSVFIFPPSDEQLKSGTASVVCLLNNFYPREAKVQWK VDNALQSGNSQESVTEQDSKDSTYSLSSTLTLSKADYEKHKV YACEVTHQGLSSPVTKSFNRGEC | 50 |

Table 33. The amino acid sequences of the control antibodies

| No. | | Amino acid sequence | SEQ ID |
|---|---|---|---|
| RO7122290 FAP/4-1BBL bispe-cific antibody | Heavy chain 1 | REGPELSPDDPAGLLDLRQGMFAQLVAQNVLLIDGPLS WYSDPGLAGVSLTGGLSYKEDTKELVVAKAGVYYVFF QLELRRVVAGEGSGSVSLALHLQPLRSAAGAAALALTV DLPPASSEARNSAFGFQGRLLHLSAGQRLGVHLHTEAR ARHAWQLTQGATVLGLFRVTPEIPAGL GGGGSGGGGS REGPELSPDDPAGLLDLRQGMFAQLVAQNVLLIDGPLS WYSDPGLAGVSLTGGLSYKEDTKELVVAKAGVYYVFF QLELRRVVAGEGSGSVSLALHLQPLRSAAGAAALALTV DLPPASSEARNSAFGFQGRLLHLSAGQRLGVHLHTEAR ARHAWQLTQGATVLGLFRVTPEIPAGL GGGGSGGGGS RTVAAPSVFIFPPSDRKLKSGTASVVCLLNNFYPREAKV QWKVDNALQSGNSQESVTEQDSKDSTYSLSSTLTLSKA DYEKHKVYACEVTHQGLSSPVTKSFNRGEC DKTHTCPPCPAPEAAGGPSVFLFPPKPKDTLMISRTPEV TCVVVDVSHEDPEVKFNWYVDGVEVHNAKTKPREEQ YNSTYRVVSVLTVLHQDWLNGKEYKCKVSNKALGAPI EKTISKAKGQPREPQVYTLPPCRDELTKNQVSLWCLVK GFYPSDIAVEWESNGQPENNYKTTPPVLDSDGSFFLYSK LTVDKSRWQQGNVFSCSVMHEALHNHYTQKSLSLSPG K | 51 |
| | Heavy chain 2 | EVQLLESGGGLVQPGGSLRLSCAASGFTFSSYAMSWVR QAPGKGLEWVSAIIGSGASTYYADSVKGRFTISRDNSK NTLYLQMNSLRAEDTAVYYCAKGWFGGFNYWGQGTL VTVSS ASTKGPSVFPLAPSSKSTSGGTAALGCLVKDYFPEPVTV SWNSGALTSGVHTFPAVLQSSGLYSLSSVVTVPSSSLGT QTYICNVNHKPSNTKVDKKVEPKSCDKTHTCPPCPAPE AAGGPSVFLFPPKPKDTLMISRTPEVTCVVVDVSHEDPE VKFNWYVDGVEVHNAKTKPREEQYNSTYRVVSVLTVL HQDWLNGKEYKCKVSNKALGAPIEKTISKAKGQPREP QVCTLPPSRDELTKNQVSLSCAVKGFYPSDIAVEWESNG QPENNYKTTPPVLDSDGSFFLVSKLTVDKSRWQQGNVF SCSVMHEALHNHYTQKSLSLSPGK | 52 |
| | Light chain 1 | REGPELSPDDPAGLLDLRQGMFAQLVAQNVLLIDGPLS WYSDPGLAGVSLTGGLSYKEDTKELVVAKAGVYYVFF QLELRRVVAGEGSGSVSLALHLQPLRSAAGAAALALTV DLPPASSEARNSAFGFQGRLLHLSAGQRLGVHLHTEAR ARHAWQLTQGATVLGLFRVTPEIPAGL | 53 |

(continued)

| No. | | Amino acid sequence | SEQ ID |
|---|---|---|---|
| | Light chain 2 | EIVLTQSPGTLSLSPGERATLSCRASQSVTSSYLAWYQQ KPGQAPRLLINVGSRRATGIPDRFSGSGSGTDFTLTISRL EPEDFAVYYCQQGIMLPPTFGQGTKVEIK RTVAAPSVFIFPPSDEQLKSGTASVVCLLNNFYPREAKV QWKVDNALQSGNSQESVTEQDSKDSTYSLSSTLTLSKA DYEKHKVYACEVTHQGLSSPVTKSFNRGEC | 54 |
| RO7300490 (2302(2+1)PG) FAP/CD40 bispecific antibody | Heavy chain 1 | QVQLVQSGAEVKKPGASVKVSCKASGYSFTGYYIHWV RQAPGQSLEWMGRVIPNAGGTSYNQKFKGRVTLTVDK SISTAYMELSRLRSDDTAVYYCAREGIYWWGQGTTVTV SS ASTKGPSVFPLAPSSKSTSGGTAALGCLVEDYFPEPVTV SWNSGALTSGVHTFPAVLQSSGLYSLSSVVTVPSSSLGT QTYICNVNHKPSNTKVDEKVEPKSCDKTHTCPPCPAPE AAGGPSVFLFPPKPKDTLMISRTPEVTCVVVDVSHEDPE VKFNWYVDGVEVHNAKTKPREEQYNSTYRVVSVLTVL HQDWLNGKEYKCKVSNKALGAPIEKTISKAKGQPREP QVYTLPPCRDELTKNQVSLWCLVKGFYPSDIAVEWESN GQPENNYKTTPPVLDSDGSFFLYSKLTVDKSRWQQGNV FSCSVMHEALHNHYTQKSLSLSPG GGGGSGGGGSGGGGSGGGGS EIVLTQSPGTLSLSPGERATLSCRASQSVSRSYLAWYQQ KPGQAPRLLIIGASTRATGIPDRFSGSGSGTDFTLTISRLE PEDFAVYYCQQGQVIPPTFGQGTKVEIK SSASTKGPSVFPLAPSSKSTSGGTAALGCLVKDYFPEPV TVSWNSGALTSGVHTFPAVLQSSGLYSLSSVVTVPSSSL GTQTYICNVNHKPSNTKVDKKVEPKSC | 55 |
| | Light chain 1 | EVQLLESGGGLVQPGGSLRLSCAASGFTFSSHAMSWVR QAPGKGLEWVSAIWASGEQYYADSVKGRFTISRDNSK NTLYLQMNSLRAEDTAVYYCAKGWLGNFDYWGQGTL VTVSS ASVAAPSVFIFPPSDEQLKSGTASVVCLLNNFYPREAKV QWKVDNALQSGNSQESVTEQDSKDSTYSLSSTLTLSKA DYEKHKVYACEVTHQGLSSPVTKSFNRGEC | 56 |

(continued)

| No. | | Amino acid sequence | SEQ ID |
|---|---|---|---|
| | Heavy chain 2 | QVQLVQSGAEVKKPGASVKVSCKASGYSFTGYYIHWV RQAPGQSLEWMGRVIPNAGGTSYNQKFKGRVTLTVDK SISTAYMELSRLRSDDTAVYYCAREGIYWWGQGTTVTV SS ASTKGPSVFPLAPSSKSTSGGTAALGCLVEDYFPEPVTV SWNSGALTSGVHTFPAVLQSSGLYSLSSVVTVPSSSLGT QTYICNVNHKPSNTKVDEKVEPKSCDKTHTCPPCPAPE AAGGPSVFLFPPKPKDTLMISRTPEVTCVVVDVSHEDPE VKFNWYVDGVEVHNAKTKPREEQYNSTYRVVSVLTVL HQDWLNGKEYKCKVSNKALGAPIEKTISKAKGQPREP QVCTLPPSRDELTKNQVSLSCAVKGFYPSDIAVEWESNG QPENNYKTTPPVLDSDGSFFLVSKLTVDKSRWQQGNVF SCSVMHEALHNHYTQKSLSLSPG | 57 |
| | Light chain 2 | DIVMTQTPLSLSVTPGQPASISCRSSQSLVHSNGNTFLH WYLQKPGQSPQLLIYTVSNRFSGVPDRFSGSGSGTDFTL KISRVEAEDVGVYFCSQTTHVPWTFGGGTKVEIK RTVAAPSVFIFPPSDRKLKSGTASVVCLLNNFYPREAKV QWKVDNALQSGNSQESVTEQDSKDSTYSLSSTLTLSKA DYEKHKVYACEVTHQGLSSPVTKSFNRGEC | 58 |
| Ab10-A12V2-2 | Heavy chain | QVQLQQSGVEVKKPGASVTVSCRASGYSFADHFIHWV RQAPGQGFQWMGWINPNRGVTHHAQDFQGRVAMTRD | 59 |
| FAP/CD40 bispecific antibody | | MSTDTVYMELTSLRSDDTAVYYCARDASLTARPYYFYG FDVWGQGTLVTVSS ASTKGPSVFPLAPSSKSTSGGTAALGCLVKDYFPEPVTV SWNSGALTSGVHTFPAVLQSSGLYSLSSVVTVPSSSLGT QTYICNVNHKPSNTKVDKKVEPKSCDKTHTCPPCPAPE AAGGPSVFLFPPKPKDTLMISRTPEVTCVVVDVSHEDPE VKFNWYVDGVEVHNAKTKPREEQYNSTYRVVSVLTVL HQDWLNGKEYKCKVSNKALPAPIEKTISKAKGQPREPQ VYTLPPSRDELTKNQVSLTCLVKGFYPSDIAVEWESNGQ PENNYKTTPPVLDSDGSFFLYSKLTVDKSRWQQGNVFS CSVMHEALHNHYTQKSLSLSPGK GGGGSGGGGS EVQLVESGGGLVQPGGSLRLSCAASGFTFSNYGMKWV RQAPGKGLEWVSTILSQGGSTYYADSVKGRFTISRDNA KNSLYLQMNSLRAEDTALYYCLRVDWSDYSPRGQGTQ VTVSS | |

(continued)

| No. | | Amino acid sequence | SEQ ID |
|---|---|---|---|
| | Light chain | DIQMTQSPSSVSASVGDRVTITCRASQGISSWLAWYQQ KPGKAPKLLIYAASSLQSGVPSRFSGSGSGTDFTLTISSL QPEDFATYYCQQANSFPPAFGQGTKVEIK RTVAAPSVFIFPPSDEQLKSGTASVVCLLNNFYPREAKV QWKVDNALQSGNSQESVTEQDSKDSTYSLSSTLTLSKA DYEKHKVYACEVTHQGLSSPVTKSFNRGEC | 60 |
| MP0310 FAP/4-1BB | | GSDLGKKLLEAARAGQDDEVRELLKAGADVNAKDYF SHTPLHLAARNGHLKIVEVLLKAGADVNAKDFAGKTP LHLAAADGHLEIVEVLLKAGADVNAQDIFGKTPADIAA DAGHEDIAEVLQKAA GSPTPTPTTPTPTPTTPTPTPTGS DLGKKLLEAARAGQDDEVRELLKAGADVNAKDVLGW TPLHLAAFEGHLEIVEVLLKAGADVNAKDKKGWTPLQ LAARTGHLEIVEVLLKAGADVNAKDHIGATPLHLAAW QGHPEIVEVLLKAGADVNAQDKSGKTPADLAADAGHE DIAEVLQKAA GSPTPTPTTPTPTPTTPTPTPTGS DLGQKLLKAAQEGQDDEVRELLKAGADVNAKDLRGIT PLHVAAWQGHLEIVEVLLKAGADVNAKDSKGETPLHL AAFRGHLEIVEVLLKAGADVNAQDQQGETPADLAALA GHVDIAEVLQKAA GSPTPTPTTPTPTPTTPTPTPTGS DLGQKLLKAAQEGQDDEVRELLKAGADVNAKDLRGIT PLHVAAWQGHLEIVEVLLKAGADVNAKDSKGETPLHL AAFRGHLEIVEVLLKAGADVNAQDQQGETPADLAALA GHVDIAEVLQKAA GSPTPTPTTPTPTPTTPTPTPTGS DLGKKLLEAARAGQDDEVRELLKAGADVNAKDYFSH TPLHLAARNGHLKIVEVLLKAGADVNAKDFAGKTPLH LAAADGHLEIVEVLLKAGADVNAQDIFGKTPADIAADA GHEDIAEVLQKAA HHHHHH | 61 |

(continued)

| No. | | Amino acid sequence | SEQ ID |
|---|---|---|---|
| BNT312 CD40/4-1BB bispe-cific antibody mole-cule | Heavy chain 1 | EVQLVQSGAEVKKPGASVKVSCKTSGYTFTEYIMHWV RQAPGQGLEWMGGIIPNNGGTSYNQKFQGRVTMTVDK STSTGYMELSSLRSEDTAVYYCTRREVYGRNYYALDY WGQGTLVTVSSASTKGPSVFPLAPSSKSTSGGTAALGCL VKDYFPEPVTVSWNSGALTSGVHTFPAVLQSSGLYSLSS VVTVPSSSLGTQTYICNVNHKPSNTKVDKRVEPKSCDK THTCPPCPAPEFEGGPSVFLFPPKPKDTLMISRTPEVTCV VVAVSHEDPEVKFNWYVDGVEVHNAKTKPREEQYNST YRVVSVLTVLHQDWLNGKEYKCKVSNKALPAPIEKTIS KAKGQPREPQVYTLPPSREEMTKNQVSLTCLVKGFYPS DIAVEWESNGQPENNYKTTPPVLDSDGSFLLYSKLTVD KSRWQQGNVFSCSVMHEALHNHYTQKSLSLSPGK | 62 |
| | Light chain 1 | DIQMTQSPSSLSASVGDRVTITCSASQGINNYLNWYQQ KPGKAVKLLIYYTSSLHSGVPSRFSGSGSGTDYTFTISSL QPEDIATYYCQQYSNLPYTFGGGTKVEIKRTVAAPSVFI FPPSDEQLKSGTASVVCLLNNFYPREAKVQWKVDNAL QSGNSQESVTEQDSKDSTYSLSSTLTLSKADYEKHKVY ACEVTHQGLSSPVTKSFNRGEC | 63 |
| | Heavy chain 2 | EVQLVESGGGLVQPGRSLRLSCTASGFSLNDYWMSWV RQAPGKGLEWVGYIDVGGSLYYAASVKGRFTISRDDSK SIAYLQMNSLKTEDTAVYYCARGGLTYGFDLWGQGTL VTVSSASTKGPSVFPLAPSSKSTSGGTAALGCLVKDYFP EPVTVSWNSGALTSGVHTFPAVLQSSGLYSLSSVVTVPS SSLGTQTYICNVNHKPSNTKVDKRVEPKSCDKTHTCPP CPAPEFEGGPSVFLFPPKPKDTLMISRTPEVTCVVVAVSH EDPEVKFNWYVDGVEVHNAKTKPREEQYNSTYRVVSV LTVLHQDWLNGKEYKCKVSNKALPAPIEKTISKAKGQP REPQVYTLPPSREEMTKNQVSLTCLVKGFYPSDIAVEWE SNGQPENNYKTTPPVLDSDGSFFLYSRLTVDKSRWQQG NVFSCSVMHEALHNHYTQKSLSLSPGK | 64 |
| | Light chain 2 | DIVMTQSPSSLSASVGDRVTITCQASEDISSYLAWYQQK PGKAPKRLIYGASDLASGVPSRFSASGSGTDYTFTISSL QPEDIATYYCHYYATISGLGVAFGGGTKVEIKRTVAAPS VFIFPPSDEQLKSGTASVVCLLNNFYPREAKVQWKVDN ALQSGNSQESVTEQDSKDSTYSLSSTLTLSKADYEKHK VYACEVTHQGLSSPVTKSFNRGEC | 65 |

**Claims**

1. A FAP/4-1BB/CD40-binding molecule, comprising a first antigen-binding domain that specifically binds to FAP, a second antigen-binding domain that specifically binds to CD40, and a third antigen-binding domain that specifically binds to 4-1BB, wherein the third antigen-binding domain comprises at least one immunoglobulin single variable domain;

the immunoglobulin single variable domain in the third antigen-binding domain comprises a CDR1, a CDR2, and a CDR3 from the amino acid sequence set forth in any one of SEQ ID NOs: 28, 18, 23-26, and 33, and the CDRs are defined according to the Kabat, IMGT, Chothia, AbM, or Contact numbering scheme.

2. The FAP/4-1BB/CD40-binding molecule according to claim 1, wherein the immunoglobulin single variable domain in the third antigen-binding domain comprises a CDR1, a CDR2, and a CDR3 set forth in SEQ ID NOs: 19, 30, and 31, respectively; preferably, the immunoglobulin single variable domain in the third antigen-binding domain comprises a CDR1, a CDR2, and a CDR3 as shown in any one of the following:

   a-1) a CDR1, a CDR2, and a CDR3 set forth in SEQ ID NOs: 19, 29, and 27, respectively;
   a-2) a CDR1, a CDR2, and a CDR3 set forth in SEQ ID NOs: 19, 20, and 27, respectively; or
   a-3) a CDR1, a CDR2, and a CDR3 set forth in SEQ ID NOs: 19, 20, and 21, respectively.

3. The FAP/4-1BB/CD40-binding molecule according to claim 1 or 2, wherein the immunoglobulin single variable domain in the third antigen-binding domain is engineered by humanization, avidity maturation, T-cell epitope removal, reduction of antibody deamidation, reduction of antibody aggregation, reduction of antibody isomerization, reduction of binding to pre-existing anti-drug antibodies (pre-ADAs), and/or reduction of binding to anti-drug antibodies (ADAs); preferably, a framework region template of a human germline gene for the humanization engineering is derived from IGHV3-64*04, IGHV3-23*03, and/or IGHV3-74*01.

4. The FAP/4-1BB/CD40-binding molecule according to any one of claims 1-3, wherein the immunoglobulin single variable domain in the third antigen-binding domain comprises the amino acid sequence set forth in any one of SEQ ID NOs: 28, 18, 23-26, and 33 or an amino acid sequence having at least 90% sequence identity thereto.

5. The FAP/4-1BB/CD40-binding molecule according to any one of claims 1-4, wherein the second antigen-binding domain comprises at least one immunoglobulin single variable domain; the immunoglobulin single variable domain in the second antigen-binding domain comprises a CDR1, a CDR2, and a CDR3 from the amino acid sequence set forth in SEQ ID NO: 32, 11, or 15, and the CDRs are defined according to the Kabat, IMGT, Chothia, AbM, or Contact numbering scheme.

6. The FAP/4-1BB/CD40-binding molecule according to claim 5, wherein the immunoglobulin single variable domain in the second antigen-binding domain comprises a CDR1, a CDR2, and a CDR3 set forth in SEQ ID NOs: 12, 13, and 14, respectively;

   preferably, the immunoglobulin single variable domain in the second antigen-binding domain is engineered by humanization, avidity maturation, T-cell epitope removal, reduction of antibody deamidation, reduction of antibody aggregation, reduction of antibody isomerization, reduction of binding to pre-existing anti-drug antibodies (pre-ADAs), and/or reduction of binding to anti-drug antibodies (ADAs);
   preferably, a heavy chain framework region moiety of a human germline gene for the humanization engineering is derived from IGHV3-48*03.

7. The FAP/4-1BB/CD40-binding molecule according to claim 5 or 6, wherein the immunoglobulin single variable domain in the second antigen-binding domain comprises the amino acid sequence set forth in SEQ ID NO: 32, 11, or 15 or an amino acid sequence having at least 90% sequence identity thereto.

8. The FAP/4-1BB/CD40-binding molecule according to any one of claims 1-7, wherein the first antigen-binding domain comprises a heavy chain variable region (VH) and a light chain variable region (VL), wherein:

   the VH comprises a HCDR1, a HCDR2, and a HCDR3 set forth in SEQ ID NOs: 3, 4, and 5, respectively; and
   the VL comprises a LCDR1, a LCDR2, and a LCDR3 set forth in SEQ ID NOs: 6, 7, and 8, respectively.

9. The FAP/4-1BB/CD40-binding molecule according to claim 8, wherein:

   the heavy chain variable region comprises the amino acid sequence set forth in SEQ ID NO: 1 or an amino acid sequence having at least 90% sequence identity thereto; and,
   the light chain variable region comprises the amino acid sequence set forth in SEQ ID NO: 2 or an amino acid sequence having at least 90% sequence identity thereto;

preferably, the first antigen-binding domain is selected from the group consisting of a Fab, a Fab', an Fv, and an ScFv; more preferably, the first antigen-binding domain is a Fab;

preferably, the first antigen-binding domain comprises a Fab heavy chain and a Fab light chain, wherein:

the Fab heavy chain comprises the amino acid sequence set forth in SEQ ID NO: 66 or an amino acid sequence having at least 90% sequence identity thereto; and,

the Fab light chain comprises the amino acid sequence set forth in SEQ ID NO: 67 or an amino acid sequence having at least 90% sequence identity thereto.

10. A FAP/4-1BB/CD40-binding molecule, comprising a first antigen-binding domain that specifically binds to FAP, a second antigen-binding domain that specifically binds to CD40, and a third antigen-binding domain that specifically binds to 4-1BB, wherein the first antigen-binding domain comprises a heavy chain variable region (VH) and a light chain variable region (VL), wherein:

the VH comprises a HCDR1, a HCDR2, and a HCDR3 set forth in SEQ ID NOs: 3, 4, and 5, respectively;

the VL comprises a LCDR1, a LCDR2, and a LCDR3 set forth in SEQ ID NOs: 6, 7, and 8, respectively;

preferably, the heavy chain variable region comprises the amino acid sequence set forth in SEQ ID NO: 1 or an amino acid sequence having at least 90% sequence identity thereto; and,

the light chain variable region comprises the amino acid sequence set forth in SEQ ID NO: 2 or an amino acid sequence having at least 90% sequence identity thereto;

preferably, the first antigen-binding domain comprises a Fab heavy chain and a Fab light chain, wherein:

the Fab heavy chain comprises the amino acid sequence set forth in SEQ ID NO: 66 or an amino acid sequence having at least 90% sequence identity thereto; and,

the Fab light chain comprises the amino acid sequence set forth in SEQ ID NO: 67 or an amino acid sequence having at least 90% sequence identity thereto;

preferably, the third antigen-binding domain is as defined in any one of claims 1-4;

preferably, the second antigen-binding domain is as defined in any one of claims 5-7.

11. A FAP/4-1BB/CD40-binding molecule, comprising a first antigen-binding domain that specifically binds to FAP, a second antigen-binding domain that specifically binds to CD40, and a third antigen-binding domain that specifically binds to 4-1BB, wherein the second antigen-binding domain comprises at least one immunoglobulin single variable domain, and the immunoglobulin single variable domain in the second antigen-binding domain comprises a CDR1, a CDR2, and a CDR3 set forth in SEQ ID NOs: 12, 13, and 14, respectively;

the CDRs are defined according to the Kabat, IMGT, Chothia, AbM, or Contact numbering scheme;

preferably, the immunoglobulin single variable domain in the second antigen-binding domain comprises the amino acid sequence set forth in SEQ ID NO: 11, 15, or 32 or an amino acid sequence having at least 90% sequence identity thereto;

preferably, the third antigen-binding domain is as defined in any one of claims 1-4;

preferably, the first antigen-binding domain is as defined in any one of claims 8-9.

12. The FAP/4-1BB/CD40-binding molecule according to any one of claims 1-11, further comprising an Fc region, wherein:

preferably, the Fc region is derived from a human IgG1, IgG2, or IgG4 Fc region;

more preferably, the human IgG1 Fc region comprises at least one of the following mutations: 234A, 235A, 220A, 297A or 297Q, 267E, and 328F;

more preferably, the human IgG4 Fc region comprises at least one of the following mutations: 228P, 234A, 235A, and 447A;

the above mutations are numbered according to the EU numbering scheme.

13. The FAP/4-1BB/CD40-binding molecule according to any one of the preceding claims, further comprising a linker, wherein the linker is located between any two of the first, second, and third antigen-binding domains;

preferably, the linker is a $(G_xS)_y$ linker, wherein x is selected from the group consisting of integers of 1-5, and y is selected from the group consisting of integers of 1-6;

more preferably, x is 4, and y is 1, 2, or 3.

14. The FAP/4-1BB/CD40-binding molecule according to any one of the preceding claims, wherein the valence ratio of the first antigen-binding domain to the second antigen-binding domain to the third antigen-binding domain is (1-2):(1-5):(1-5), preferably 1:2:2, 1:2:4, 1:1:1, or 1:1:2.

15. A FAP/4-1BB/CD40-binding molecule, comprising a first polypeptide chain and a second polypeptide chain, wherein in the direction from an N-terminus to a C-terminus, the FAP/4-1BB/CD40-binding molecule comprises any one or a combination of the following groups:

(I) the first polypeptide chain: [a Fab heavy chain of a first antigen-binding domain that specifically binds to FAP]-[an Fc region]-[a linker]a-[a third antigen-binding domain that specifically binds to 4-1BB]-[a linker]b-[a second antigen-binding domain that specifically binds to CD40]; and, the second polypeptide chain: [a Fab light chain of a first antigen-binding domain that specifically binds to FAP]; (II) the first polypeptide chain: [a Fab heavy chain of a first antigen-binding domain that specifically binds to FAP]-[an Fc region]-[a linker]a-[a third antigen-binding domain that specifically binds to 4-1BB]; and, the second polypeptide chain: [a Fab light chain of a first antigen-binding domain that specifically binds to FAP]-[a linker]b-[a second antigen-binding domain that specifically binds to CD40]; (III) the first polypeptide chain: [a Fab heavy chain of a first antigen-binding domain that specifically binds to FAP]-[an Fc region]-[a linker]a-[a third antigen-binding domain that specifically binds to 4-1BB]-[a linker]b-[a third antigen-binding domain that specifically binds to 4-1BB]; and, the second polypeptide chain: [a Fab light chain of a first antigen-binding domain that specifically binds to FAP]-[a linker]c-[a second antigen-binding domain that specifically binds to CD40]; (IV) the first polypeptide chain: [a Fab heavy chain of a first antigen-binding domain that specifically binds to FAP]-[an Fc region]-[a linker]a-[a second antigen-binding domain that specifically binds to CD40]-[a linker]b-[a third antigen-binding domain that specifically binds to 4-1BB]; and, the second polypeptide chain: [a Fab light chain of a first antigen-binding domain that specifically binds to FAP]; and (V) the first polypeptide chain: [a Fab heavy chain of a first antigen-binding domain that specifically binds to FAP]-[an Fc region]-[a linker]a-[a second antigen-binding domain that specifically binds to CD40]; and, the second polypeptide chain: [a Fab light chain of a first antigen-binding domain that specifically binds to FAP]-[a linker]b-[a third antigen-binding domain that specifically binds to 4-1BB]; wherein - represents a peptide bond, and any two linkers may be identical or different; a, b, and c are each independently selected from the group consisting of 0 and 1; preferably, the linkers are $(G_xS)_y$ linkers, wherein x is selected from the group consisting of integers of 1-5, and y is selected from the group consisting of integers of 1-6; more preferably, the linkers are each independently selected from the group consisting of $G_4S$, $(G_4S)_2$, $(G_4S)_3$, and $(G_4S)_4$; preferably, the first antigen-binding domain that specifically binds to FAP is selected from the group consisting of the first antigen-binding domain according to any one of claims 8-9; preferably, the second antigen-binding domain that specifically binds to CD40 is selected from the group consisting of the second antigen-binding domain according to any one of claims 5-7; preferably, the third antigen-binding domain that specifically binds to 4-1BB is selected from the group consisting of the first antigen-binding domain according to any one of claims 1-4.

16. The FAP/4-1BB/CD40-binding molecule according to any one of the preceding claims, wherein the FAP/4-1BB/CD40-binding molecule comprises a first polypeptide chain and a second polypeptide chain as shown in any one of the following:

the first polypeptide chain and the second polypeptide chain comprise the amino acid sequences set forth in SEQ ID NOs: 49 and 50, respectively; the first polypeptide chain and the second polypeptide chain comprise the amino acid sequences set forth in SEQ ID NOs: 34 and 35, respectively; the first polypeptide chain and the second polypeptide chain comprise the amino acid sequences set forth in SEQ ID NOs: 36 and 37, respectively; the first polypeptide chain and the second polypeptide chain comprise the amino acid sequences set forth in SEQ ID NOs: 38 and 39, respectively; the first polypeptide chain and the second polypeptide chain comprise the amino acid sequences set forth in SEQ ID NOs: 40 and 41, respectively; the first polypeptide chain and the second polypeptide chain comprise the amino acid sequences set forth in SEQ ID NOs: 42 and 43, respectively;

the first polypeptide chain and the second polypeptide chain comprise the amino acid sequences set forth in SEQ ID NOs: 45 and 46, respectively;

the first polypeptide chain and the second polypeptide chain comprise the amino acid sequences set forth in SEQ ID NOs: 47 and 48, respectively; or,

a combination of amino acid sequences having at least 90% sequence identity to the first polypeptide chain and the second polypeptide chain of any one of the aforementioned groups, respectively.

17. A 4-1BB-binding molecule, comprising:

i) a third antigen-binding domain that specifically binds to 4-1BB, wherein the third antigen-binding domain comprises a CDR1, a CDR2, and a CDR3 from the amino acid sequence set forth in any one of SEQ ID NOs: 18, 23-26, 28, and 33, and the CDRs are defined according to the Kabat, IMGT, Chothia, AbM, or Contact numbering scheme,

wherein preferably, the 4-1BB-binding molecule further comprises:

ii) a first antigen-binding domain that specifically binds to FAP, or
iii) a second antigen-binding domain that specifically binds to CD40;
more preferably, the first antigen-binding domain is as defined in any one of claims 8-9;
more preferably, the second antigen-binding domain is as defined in any one of claims 5-7.

18. The 4-1BB-binding molecule according to claim 17, wherein the third antigen-binding domain comprises a CDR1, a CDR2, and a CDR3 set forth in SEQ ID NOs: 19, 30, and 31, respectively;
preferably, the third antigen-binding domain comprises a CDR1, a CDR2, and a CDR3 as shown in any one of the following:

a-1) a CDR1, a CDR2, and a CDR3 set forth in SEQ ID NOs: 19, 29, and 27, respectively;
a-2) a CDR1, a CDR2, and a CDR3 set forth in SEQ ID NOs: 19, 20, and 27, respectively; or
a-3) a CDR1, a CDR2, and a CDR3 set forth in SEQ ID NOs: 19, 20, and 21, respectively.

19. The 4-1BB-binding molecule according to claim 17 or 18, wherein the immunoglobulin single variable domain is engineered by humanization, avidity maturation, T-cell epitope removal, reduction of antibody deamidation, reduction of antibody aggregation, reduction of binding to pre-existing anti-drug antibodies (pre-ADAs), and/or reduction of antibody isomerization;
preferably, a heavy chain framework region moiety of a human germline gene for the humanization engineering is derived from IGHV3-64*04, IGHV3-23*03, and/or IGHV3-74*01.

20. The 4-1BB-binding molecule according to any one of claims 17-19, wherein the 4-1BB-binding molecule comprises an immunoglobulin single variable domain represented by the following amino acid sequence:

the amino acid sequence set forth in any one of SEQ ID NOs: 18, 23-26, 28, and 33 or an amino acid sequence having at least 90% sequence identity thereto;
optionally,
the 4-1BB-binding molecule comprises:
the amino acid sequence set forth in SEQ ID NO: 44 or an amino acid sequence having at least 90% sequence identity thereto.

21. A polynucleotide, wherein the polynucleotide encodes the FAP/4-1BB/CD40-binding molecule according to any one of claims 1-16 or the 4-1BB-binding molecule according to any one of claims 17-20.

22. A vector, comprising the polynucleotide according to claim 21.

23. A host cell, comprising the polynucleotide according to claim 21 or the vector according to claim 22.

24. A method for preparing a FAP/4-1BB/CD40-binding molecule or a 4-1BB-binding molecule, comprising the following steps:

expressing the FAP/4-1BB/CD40-binding molecule according to any one of claims 1-16 or the 4-1BB-binding molecule according to any one of claims 17-20 in a host cell, and, isolating the FAP/4-1BB/CD40-binding molecule or the 4-1BB-binding molecule from the host cell,

wherein optionally, the method further comprises a step of purifying the FAP/4-1BB/CD40-binding molecule or the 4-1BB-binding molecule.

25. A pharmaceutical composition, comprising the FAP/4-1BB/CD40-binding molecule according to any one of claims 1-16, the 4-1BB-binding molecule according to any one of claims 17-20, the polynucleotide according to claim 21, or the vector according to claim 22, wherein preferably, the pharmaceutical composition further comprises one or more pharmaceutically acceptable excipients, diluents, or auxiliary materials.

26. Use of the FAP/4-1BB/CD40-binding molecule according to any one of claims 1-16, the 4-1BB-binding molecule according to any one of claims 17-20, the polynucleotide according to claim 21, the vector according to claim 22, or the pharmaceutical composition according to claim 25 in any one of (1)-(3) below:

(1) for preventing or treating cancer, or manufacturing a medicament for preventing or treating cancer,
(2) for activating T cells, or manufacturing a medicament for activating T cells, and
(3) for promoting DC activation, or manufacturing a medicament for promoting DC activation,

wherein preferably, the cancer is selected from the group consisting of lung cancer, prostate cancer, breast cancer, head and neck cancer, esophageal cancer, gastric cancer, colorectal cancer, bladder cancer, cervical cancer, uterine cancer, ovarian cancer, liver cancer, melanoma, renal cancer, squamous cell carcinoma, and hematological cancer.

27. A method for treating or alleviating cancer, comprising: administering to a subject in need thereof the FAP/4-1BB/CD40-binding molecule according to any one of claims 1-16, the 4-1BB-binding molecule according to any one of claims 17-20, the polynucleotide according to claim 21, the vector according to claim 22, or the pharmaceutical composition according to claim 25,

wherein preferably, the cancer is selected from the group consisting of lung cancer, prostate cancer, breast cancer, head and neck cancer, esophageal cancer, gastric cancer, colorectal cancer, bladder cancer, cervical cancer, uterine cancer, ovarian cancer, liver cancer, melanoma, renal cancer, squamous cell carcinoma, and hematological cancer.

FIG. 1

FIG. 2

FIG. 3

FIG. 4A

FIG. 4B

FIG. 4C

FIG. 5A

FIG. 5B

FIG. 5C

FIG. 6A

FIG. 6B

FIG. 6C

No addition for crosslinking

FIG. 7A

Addition of CHO-K1-HuFAP for crosslinking

FIG. 7B

Addition of HEK293-HuCD40 for crosslinking

FIG. 7C

FIG. 8A

FIG. 8B

FIG. 8C

FIG. 9A

FIG. 9B

FIG. 9C

FIG. 10A

FIG. 10B

FIG. 10C

No addition for crosslinking

FIG. 11A

Addition of CHO-K1-HuFAP for crosslinking

FIG. 11B

Addition of HEK293-Hu4-1BB for crosslinking

FIG. 11C

FIG. 12A

FIG. 12B

FIG. 12C

FIG. 13

FIG. 14

FIG. 15

FIG. 16

FIG. 17

FIG. 18

FIG. 19

FIG. 20

FIG. 21

ALT

Day-17 hepatotoxicity

AST

FIG. 22

FIG. 23

FIG. 24

FIG. 25

## INTERNATIONAL SEARCH REPORT

| International application No. |
| --- |
| **PCT/CN2024/079551** |

**A.      CLASSIFICATION OF SUBJECT MATTER**

C07K16/28(2006.01)i;  A61P35/00(2006.01)i;  C07K16/46(2006.01)i

According to International Patent Classification (IPC) or to both national classification and IPC

**B.      FIELDS SEARCHED**

Minimum documentation searched (classification system followed by classification symbols)

IPC:  C07K;  A61P

Documentation searched other than minimum documentation to the extent that such documents are included in the fields searched

Electronic data base consulted during the international search (name of data base and, where practicable, search terms used)

CJFD, CNTXT, ENTXTC, ENTXT, VENWFB, WFCCP, WFCD, WFCJ, WFCOA, WFCSTA, WFFCP, WFFD, WFFJ, WFFOA, WFS, WFSTR, PubMed, CNKI, GenBank, STN, Baidu, ISI Web of Knowledge: 恒瑞, 4-1BB, CD137, CD40, TNFRSF5, FAP, 单一可变结构域, 单域, 第三抗原, 抗体, 三特异, 多特异, CDR, VH, VL, 癌症, cancer

**C.      DOCUMENTS CONSIDERED TO BE RELEVANT**

| Category* | Citation of document, with indication, where appropriate, of the relevant passages | Relevant to claim No. |
| --- | --- | --- |
| A | CN 111148761 A (SYSTIMMUNE, INC. et al.) 12 May 2020 (2020-05-12) <br> description, paragraphs 17-19 | 1-27 |
| A | CN 111655731 A (F. HOFFMANN-LA ROCHE AG) 11 September 2020 (2020-09-11) <br> claims 1 and 2 | 1-27 |
| A | WO 2022057875 A1 (BIOTHEUS (ZHUHAI) CO., LTD.) 24 March 2022 (2022-03-24) <br> claim 1 | 1-27 |
| A | US 2022025060 A1 (JIANGSU HENGRUI MEDICINE CO., LTD. et al.) 27 January 2022 (2022-01-27) <br> description, paragraphs 9-12 | 1-27 |
| A | CN 115066440 A (SHANGHAI HENLIUS BIOTECH CO., LTD.) 16 September 2022 (2022-09-16) <br> entire document | 1-27 |
| A | US 2022112296 A1 (CHUGAI SEIYAKU KABUSHIKI KAISHA) 14 April 2022 (2022-04-14) <br> entire document | 1-27 |

☐ Further documents are listed in the continuation of Box C.      ☑ See patent family annex.

| * | Special categories of cited documents: |
| --- | --- |
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
| --- | --- |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
| --- | --- |
| **06 May 2024** | **16 May 2024** |

| Name and mailing address of the ISA/CN | Authorized officer |
| --- | --- |
| **China National Intellectual Property Administration (ISA/ CN)** <br> **China No. 6, Xitucheng Road, Jimenqiao, Haidian District, Beijing 100088** | |
| | Telephone No. |

Form PCT/ISA/210 (second sheet) (July 2022)

**INTERNATIONAL SEARCH REPORT**

International application No.

**PCT/CN2024/079551**

| Box No. I | Nucleotide and/or amino acid sequence(s) (Continuation of item 1.c of the first sheet) |

1. With regard to any nucleotide and/or amino acid sequence disclosed in the international application, the international search was carried out on the basis of a sequence listing:

    a. ☑ forming part of the international application as filed.

    b. ☐ furnished subsequent to the international filing date for the purposes of international search (Rule 13*ter*.1(a)),

        ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

## INTERNATIONAL SEARCH REPORT

| International application No. |
| --- |
| **PCT/CN2024/079551** |

**Box No. II        Observations where certain claims were found unsearchable (Continuation of item 2 of first sheet)**

This international search report has not been established in respect of certain claims under Article 17(2)(a) for the following reasons:

1. ☑ Claims Nos.: **26, 27**
   because they relate to subject matter not required to be searched by this Authority, namely:

   Claims 26 and 27 relate to a method for preventing or treating cancer, for activating T cells, and for promoting DC cell activation or treating or relieving cancer, and therefore do not comply with PCT Rule 39.1(iv). A search is performed on the basis of the corresponding pharmaceutical use thereof.

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

Form PCT/ISA/210 (continuation of first sheet) (July 2022)

## INTERNATIONAL SEARCH REPORT
### Information on patent family members

| International application No. |
|---|
| **PCT/CN2024/079551** |

| Patent document cited in search report | | | Publication date (day/month/year) | Patent family member(s) | | | Publication date (day/month/year) |
|---|---|---|---|---|---|---|---|
| CN | 111148761 | A | 12 May 2020 | EP | 3645050 | A2 | 06 May 2020 |
| | | | | EP | 3645050 | A4 | 11 August 2021 |
| | | | | WO | 2019005637 | A2 | 03 January 2019 |
| | | | | WO | 2019005637 | A3 | 26 March 2020 |
| | | | | US | 2023322920 | A1 | 12 October 2023 |
| | | | | US | 2020157213 | A1 | 21 May 2020 |
| | | | | US | 11649286 | B2 | 16 May 2023 |
| | | | | JP | 2020529864 | A | 15 October 2020 |
| | | | | JP | 7356970 | B2 | 05 October 2023 |
| CN | 111655731 | A | 11 September 2020 | EP | 3765501 | A1 | 20 January 2021 |
| | | | | US | 2021095002 | A1 | 01 April 2021 |
| | | | | BR | 112020013425 | A2 | 01 December 2020 |
| | | | | AU | 2019235330 | A1 | 16 July 2020 |
| | | | | CA | 3090232 | A1 | 19 September 2019 |
| | | | | WO | 2019175125 | A1 | 19 September 2019 |
| | | | | IL | 275706 | A | 31 August 2020 |
| | | | | JP | 2021515781 | A | 24 June 2021 |
| | | | | JP | 7098741 | B2 | 11 July 2022 |
| | | | | MX | 2020009512 | A | 22 October 2020 |
| | | | | TW | 202003561 | A | 16 January 2020 |
| | | | | KR | 20200131282 | A | 23 November 2020 |
| WO | 2022057875 | A1 | 24 March 2022 | US | 2023357422 | A1 | 09 November 2023 |
| | | | | CA | 3195676 | A1 | 24 March 2022 |
| | | | | JP | 2023542900 | A | 12 October 2023 |
| | | | | KR | 20230070238 | A | 22 May 2023 |
| | | | | AU | 2021342525 | A1 | 27 April 2023 |
| | | | | AU | 2021342525 | A9 | 06 July 2023 |
| | | | | EP | 4215547 | A1 | 26 July 2023 |
| US | 2022025060 | A1 | 27 January 2022 | EP | 3892634 | A1 | 13 October 2021 |
| | | | | EP | 3892634 | A4 | 16 November 2022 |
| | | | | BR | 112021009835 | A2 | 17 August 2021 |
| | | | | AU | 2019389354 | A1 | 15 July 2021 |
| | | | | WO | 2020108611 | A1 | 04 June 2020 |
| | | | | KR | 20210099027 | A | 11 August 2021 |
| | | | | CA | 3120793 | A1 | 04 June 2020 |
| | | | | JP | 2022509156 | A | 20 January 2022 |
| | | | | TW | 202039558 | A | 01 November 2020 |
| | | | | MX | 2021005823 | A | 15 July 2021 |
| CN | 115066440 | A | 16 September 2022 | ZA | 202210624 | B | 29 November 2023 |
| | | | | KR | 20220148209 | A | 04 November 2022 |
| | | | | US | 2024076395 | A1 | 07 March 2024 |
| | | | | AU | 2021225920 | A1 | 15 September 2022 |
| | | | | BR | 112022016491 | A2 | 11 October 2022 |
| | | | | EP | 4112642 | A1 | 04 January 2023 |
| | | | | JP | 2023516945 | A | 21 April 2023 |
| | | | | CA | 3169939 | A1 | 02 September 2021 |
| | | | | WO | 2021170067 | A1 | 02 September 2021 |
| US | 2022112296 | A1 | 14 April 2022 | AU | 2019347408 | A1 | 15 April 2021 |
| | | | | KR | 20210068061 | A | 08 June 2021 |

Form PCT/ISA/210 (patent family annex) (July 2022)

**INTERNATIONAL SEARCH REPORT**
Information on patent family members

| International application No. |
|---|
| **PCT/CN2024/079551** |

| Patent document cited in search report | Publication date (day/month/year) | Patent family member(s) | | | Publication date (day/month/year) |
|---|---|---|---|---|---|
| | | SG | 11202102882 | YA | 29 April 2021 |
| | | EP | 3856789 | A1 | 04 August 2021 |
| | | EP | 3856789 | A4 | 17 August 2022 |
| | | JP | 2022501325 | A | 06 January 2022 |
| | | MX | 2021003609 | A | 28 May 2021 |
| | | BR | 112021005472 | A2 | 15 June 2021 |
| | | WO | 2020067399 | A1 | 02 April 2020 |
| | | CA | 3113594 | A1 | 02 April 2020 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 202310186509
- WO 9404678 A
- US 9266938 B2
- WO 2020108611 A1

**Non-patent literature cited in the description**

- Antibodies: A Laboratory Manual. Cold Spring Harbor Press
- *J. biol. chem.*, 1968, vol. 243, 3558
- **LEDBETTER et al.** *J. Immunol.*, 1987, vol. 138, 788-785
- **STAMENKOVIC et al.** *EMBO J.*, 1989, vol. 8, 1403
- **HOLLIGER** ; **HUDSON**. *Nature Biotech*, 2005, vol. 23 (9), 1126-1136
- **ADAIR** ; **LAWSON**. *Drug Design Reviews-Online*, 2005, vol. 2 (3), 209-217
- **VERMA et al.** *Journal of Immunological Methods*, 1998, vol. 216, 165-181
- **JOHNSON** ; **WU**. *Nucleic Acids Res.*, 2000, vol. 28, 214-8
- **CHOTHIA et al.** *J. Mol. Biol.*, 1986, vol. 196, 901-17
- **CHOTHIA et al.** *Nature*, 1989, vol. 342, 877-83
- AbMTM, A Computer Program for Modeling Variable Regions of Antibodies. **MARTIN et al.** Proc Natl Acad Sci (USA). Oxford Molecular, Ltd, 1989, vol. 86, 9268-9272
- Ab Initio Protein Structure Prediction Using a Combined Hierarchical Approach. **SAMUDRALA et al.** PROTEINS, Structure. Function and Genetics Suppl., 1999, vol. 3, 194-198
- **MACCALLUM et al.** *J. Mol. Biol.*, 1996, vol. 5, 732-45
- **MAKABE et al.** *Journal of Biological Chemistry*, 2008, vol. 283, 1156-1166
- **HAMERS-CASTERMAN C** ; **ATARHOUCH T** ; **MUYLDERMANS S** ; **ROBINSON G** ; **HAMERS C** ; **SONGA EB** ; **BENDAHMAN N** ; **HAMERS R.** Naturally occurring antibodies devoid of light chains. *Nature*, 1993, vol. 363, 446-448
- **R. VAN DER LINDEN et al.** *Journal of Immunological Methods*, 2000, vol. 240, 185-195
- **LI et al.** *J Biol Chem.*, 2012, vol. 287, 13713-13721
- **DEFFA et al.** *African Journal of Biotechnology*, 17 June 2009, vol. 8 (12), 2645-2652
- **KABAT, E. A et al.** Sequences of Proteins of Immunological Interest. 1991
- **CACECI et al.** *Byte*, 1984, vol. 9, 340-362
- **WONG** ; **LOHMAN**. *Proc. Natl. Acad. Sci. USA*, 1993, vol. 90, 5428-5432
- Remington's Pharmaceutical Sciences. Mack Publishing Co., 1990
- **R REMINGTON**. The Science and Practice of Pharmacy. Mack Publishing, 2000